# EUROPEAN PATENT APPLICATION

(11) **EP 4 613 762 A1**
(43) Date of publication of application: **10.09.2025**
(21) Application number: 23884004.5
(22) Date of filing: 24.02.2023
(51) Int. Cl.: C07K 14/725, C07K 19/00, C12N 15/12, A61K 38/17, A61K 39/00, A61P 35/00

(54) **KRAS_G12V MUTANT ANTIGEN-SPECIFIC TCR AND REDIRECTED CD4 T CELL CO-EXPRESSING SAME AND CD8**

(30) Priority: 04.11.2022 CN 202211379445
(71) Applicant: Neowise Biotechnology Co., Ltd., Jiangsu (CN)
(72) Inventor: PENG, Songming, Suzhou, Jiangsu 215123 (CN); AN, Duo, Suzhou, Jiangsu 215123 (CN); ZHONG, Shan, Suzhou, Jiangsu 215123 (CN)
(74) Representative: Vossius & Partner Patentanwälte Rechtsanwälte mbB
(86) International application number: PCT/CN2023/078124
(87) International publication number: WO 2024/093056

(57) **Abstract**

The present invention relates to a T cell receptor (TCR) specifically binding to KRAS_G12V mutant antigen, a fusion protein or a conjugate comprising the TCR, a nucleic acid encoding the TCR, and an engineered cell comprising the same, and a method for preparing the engineered cell. The present invention further relates to co-expressing an exogenous CD8 molecule and the TCR gene in T cells to enhance the function of T cells. The present invention provides use of the TCR and the genetically engineered cell in the detection, prevention and/or treatment of cancers related to the KRAS_G12V mutant antigen.

## Description

### TECHNICAL FIELD

The present invention generally relates to the field of immunology. Specifically, the present invention relates to a T cell receptor (hereinafter also abbreviated as TCR) that specifically binds to KRAS_G12V mutant antigen, a genetically engineered cell expressing the TCR, and a method for preparing the genetically engineered cell. The present invention further relates to co-expressing an exogenous CD8 molecule and the TCR in T cells to enhance the function of T cells. The present invention provides use of the TCR and the genetically engineered cell in the detection, prevention and/or treatment of cancers related to the KRAS_G12V mutant antigen.

### BACKGROUND ART

RAS gene is the first discovered human oncogene, which encodes a RAS protein that is central to many important cell signaling networks. RAS gene is the most frequently mutated oncogene in human cancers. RAS protein activation caused by mutations in RAS gene has been identified in approximately 1/5 of all human tumors.

KRAS gene (Kirsten rat sarcoma viral oncogene homolog) encodes KRAS protein, a small GTPase that belongs to RAS superprotein family.

In cells, KRAS protein shifts between inactivated and activated states, in which KRAS protein is inactivated when it binds to guanosine diphosphate (GDP), while KRAS protein is activated and can activate downstream signaling pathways when it binds to guanosine triphosphate (GTP). KRAS protein is inactivated in most cells.

Of the mutations in KRAS gene, 97% mutations are amino acid residue mutations at position 12 or 13. One of the major mutations is G12V. Structural studies have shown that G12V mutation occurring in KRAS destroys GAP activity and keeps the KRAS protein bind to GTP, locking the KRAS protein in a tyrosine kinase activity state, and causing continuous activations of downstream signaling pathways (e.g., PI3K signaling pathway, MAPK signaling pathway, PI3K and Ral-GEFs signaling pathway, etc.). Switching on these downstream signaling pathways stimulates cell proliferation, migration, and ultimately contributes to tumorigenesis.

In human cancers, KRAS gene is one of the most well-known oncogenes in oncology, and was once considered as an "undruggable" target. Mutation in KRAS gene is found in nearly 90% of pancreatic cancers, 30-40% of colon cancers, 17% of endometrial cancers, 15-20% of lung cancers (including lobular lung cancers), as well as in bile duct cancers, cervical cancers, and bladder cancers.

In recent years, covalent inhibitors against KRAS mutants have been developed, which target KRAS mutants through an allosteric site, to "lock" the activity of KRAS mutants by reducing their affinity for GTP. For example, Amgen's sotorasib (AMG510), which is used to treat non-small cell lung cancer (NSCLC) patients with KRAS_G12C mutation, is a KRAS_G12C inhibitor; Mirati Therapeutics' MRTX1257 is also a KRAS_G12C inhibitor, and is currently in the preclinical development stage.

Currently no relevant therapeutic drug is used for other KRAS mutations, e.g., the KRAS_G12V mutation. There is a need in the art to develop immune cells (e.g., TCR-T cells) specific for the KRAS_G12V mutant antigen, to effectively detect, prevent, and treat cancers related to the KRAS_G12V mutant antigen.

### SUMMARY OF THE INVENTION

The present inventors, through their intensive research, have obtained a T-cell receptor (TCR) capable of binding specifically to the KRAS_G12V mutant antigen, and have prepared lymphocytes recombinantly expressing the TCR, thereby the detection of the presence of cancers related to the KRAS_G12V mutant antigen in mammals can be performed by the TCR's specific binding to the KRAS_G12V mutant antigen; and/or killing of cancer cells expressing the KRAS_G12V mutant antigen can be achieved via an immune response *in vivo* against the target cells expressing the KRAS_G12V mutant antigen, thereby meeting the aforementioned needs.

Accordingly, in a first aspect, the present invention provides an isolated or purified T cell receptor (TCR) which binds specifically to KRAS_G12V mutant antigen, preferably the TCR comprises an α chain and a β chain, wherein each of the α and β chains comprises three complementarity determining regions (CDRs), and wherein the amino acid sequence of CDR3 of the α chain is selected from SEQ ID NOs: 3, 6, 9, 12, 15, 18, 21, 24, 27, 30, 33, 36, 39, 42, 45, 48, and a variant having one or two amino acid residue changes from the sequence, and the amino acid sequence of CDR3 of the β chain is selected from SEQ ID NOs: 179, 182, 185, 188, 191, 194, 197, 200, 203, 206, 209, 212, 215, 218, 221, 224, and a variant having one or two amino acid residue changes from the sequence.

In one embodiment, the amino acid sequence of CDR3 of the α chain and the amino acid sequence of CDR3 of the β chain in TCR of the present invention are:
(i) the α chain CDR3 amino acid sequence shown in SEQ ID NO: 3 or a variant having 1 or 2 amino acid residue changes from the sequence; and the β chain CDR3 amino acid sequence shown in SEQ ID NO: 179 or a variant having 1 or 2 amino acid residue changes from the sequence;
(ii) the α chain CDR3 amino acid sequence shown in SEQ ID NO: 6 or a variant having 1 or 2 amino acid residue changes from the sequence; and the β chain CDR3 amino acid sequence shown in SEQ ID NO: 182 or a variant having 1 or 2 amino acid residue changes from the sequence;
(iii) the α chain CDR3 amino acid sequence shown in SEQ ID NO: 9 or a variant having 1 or 2 amino acid residue changes from the sequence; and the β chain CDR3 amino acid sequence shown in SEQ ID NO: 185 or a variant having 1 or 2 amino acid residue changes from the sequence;
(iv) the α chain CDR3 amino acid sequence shown in SEQ ID NO: 12 or a variant having 1 or 2 amino acid residue changes from the sequence; and the β chain CDR3 amino acid sequence shown in SEQ ID NO: 188 or a variant having 1 or 2 amino acid residue changes from the sequence;
(v) the α chain CDR3 amino acid sequence shown in SEQ ID NO: 15 or a variant having 1 or 2 amino acid residue changes from the sequence; and the β chain CDR3 amino acid sequence shown in SEQ ID NO: 191 or a variant having 1 or 2 amino acid residue changes from the sequence;
(vi) the α chain CDR3 amino acid sequence shown in SEQ ID NO: 18 or a variant having 1 or 2 amino acid residue changes from the sequence; and the β chain CDR3 amino acid sequence shown in SEQ ID NO: 194 or a variant having 1 or 2 amino acid residue changes from the sequence;
(vii) the α chain CDR3 amino acid sequence shown in SEQ ID NO: 21 or a variant having 1 or 2 amino acid residue changes from the sequence; and the β chain CDR3 amino acid sequence shown in SEQ ID NO: 197 or a variant having 1 or 2 amino acid residue changes from the sequence;
(viii) the α chain CDR3 amino acid sequence shown in SEQ ID NO: 24 or a variant having 1 or 2 amino acid residue changes from the sequence; and the β chain CDR3 amino acid sequence shown in SEQ ID NO: 200 or a variant having 1 or 2 amino acid residue changes from the sequence;
(ix) the α chain CDR3 amino acid sequence shown in SEQ ID NO: 27 or a variant having 1 or 2 amino acid residue changes from the sequence; and the β chain CDR3 amino acid sequence shown in SEQ ID NO: 203 or a variant having 1 or 2 amino acid residue changes from the sequence;
(x) the α chain CDR3 amino acid sequence shown in SEQ ID NO: 30 or a variant having 1 or 2 amino acid residue changes from the sequence; and the β chain CDR3 amino acid sequence shown in SEQ ID NO: 206 or a variant having 1 or 2 amino acid residue changes from the sequence;
(xi) the α chain CDR3 amino acid sequence shown in SEQ ID NO: 33 or a variant having 1 or 2 amino acid residue changes from the sequence; and the β chain CDR3 amino acid sequence shown in SEQ ID NO: 209 or a variant having 1 or 2 amino acid residue changes from the sequence;
(xii) the α chain CDR3 amino acid sequence shown in SEQ ID NO: 36 or a variant having 1 or 2 amino acid residue changes from the sequence; and the β chain CDR3 amino acid sequence shown in SEQ ID NO: 212 or a variant having 1 or 2 amino acid residue changes from the sequence;
(xiii) the α chain CDR3 amino acid sequence shown in SEQ ID NO: 39 or a variant having 1 or 2 amino acid residue changes from the sequence; and the β chain CDR3 amino acid sequence shown in SEQ ID NO: 215 or a variant having 1 or 2 amino acid residue changes from the sequence;
(xiv) the α chain CDR3 amino acid sequence shown in SEQ ID NO: 42 or a variant having 1 or 2 amino acid residue changes from the sequence; and the β chain CDR3 amino acid sequence shown in SEQ ID NO: 218 or a variant having 1 or 2 amino acid residue changes from the sequence;
(xv) the α chain CDR3 amino acid sequence shown in SEQ ID NO: 45 or a variant having 1 or 2 amino acid residue changes from the sequence; and the β chain CDR3 amino acid sequence shown in SEQ ID NO: 221 or a variant having 1 or 2 amino acid residue changes from the sequence; or
(xvi) the α chain CDR3 amino acid sequence shown in SEQ ID NO: 48 or a variant having 1 or 2 amino acid residue changes from the sequence; and the β chain CDR3 amino acid sequence shown in SEQ ID NO: 224 or a variant having 1 or 2 amino acid residue changes from the sequence.

In one embodiment, the amino acid sequences of the three complementarity determining regions (CDRs) comprised in the α chain and the amino acid sequences of the three CDRs comprised in the β chain of the TCR of the present invention are:
(i) the α chain CDR1, CDR2, CDR3 amino acid sequences shown in SEQ ID NOs: 1, 2, 3 or variants having 1 or 2 amino acid residue changes from the sequences respectively; and the β chain CDR1, CDR2, CDR3 amino acid sequences shown in SEQ ID NOs: 177, 178, 179 or variants having 1 or 2 amino acid residue changes from the sequences respectively;
(ii) the α chain CDR1, CDR2, CDR3 amino acid sequences shown in SEQ ID NOs: 4, 5, 6 or variants having 1 or 2 amino acid residue changes from the sequences respectively; and the β chain CDR1, CDR2, CDR3 amino acid sequences shown in SEQ ID NOs: 180, 181, 182 or variants having 1 or 2 amino acid residue changes from the sequences respectively;
(iii) the α chain CDR1, CDR2, CDR3 amino acid sequences shown in SEQ ID NOs: 7, 8, 9 or variants having 1 or 2 amino acid residue changes from the sequences respectively; and the β chain CDR1, CDR2, CDR3 amino acid sequences shown in SEQ ID NOs: 183, 184, 185 or variants having 1 or 2 amino acid residue changes from the sequences respectively;
(iv) the α chain CDR1, CDR2, CDR3 amino acid sequences shown in SEQ ID NOs: 10, 11, 12 or variants having 1 or 2 amino acid residue changes from the sequences respectively; and the β chain CDR1, CDR2, CDR3 amino acid sequences shown in SEQ ID NOs: 186, 187, 188 or variants having 1 or 2 amino acid residue changes from the sequences respectively;
(v) the α chain CDR1, CDR2, CDR3 amino acid sequence shown in SEQ ID NOs: 13, 14, 15 or variants having 1 or 2 amino acid residue changes from the sequences respectively; and the β chain CDR1, CDR2, CDR3 amino acid sequence shown in SEQ ID NOs: 189, 190, 191 or variants having 1 or 2 amino acid residue changes from the sequences respectively;
(vi) the α chain CDR1, CDR2, CDR3 amino acid sequences shown in SEQ ID NOs: 16, 17, 18 or variants having 1 or 2 amino acid residue changes from the sequences respectively; and the β chain CDR1, CDR2, CDR3 amino acid sequences shown in SEQ ID NOs: 192, 193, 194 or variants having 1 or 2 amino acid residue changes from the sequences respectively;
(vii) the α chain CDR1, CDR2, CDR3 amino acid sequences shown in SEQ ID NOs: 19, 20, 21 or variants having 1 or 2 amino acid residue changes from the sequences respectively; and the β chain CDR1, CDR2, CDR3 amino acid sequences shown in SEQ ID NOs: 195, 196, 197 or variants having 1 or 2 amino acid residue changes from the sequences respectively;
(viii) the α chain CDR1, CDR2, CDR3 amino acid sequences shown in SEQ ID NOs: 22, 23, 24 or variants having 1 or 2 amino acid residue changes from the sequences respectively; and the β chain CDR1, CDR2, CDR3 amino acid sequences shown in SEQ ID NOs: 198, 199, 200 or variants having 1 or 2 amino acid residue changes from the sequences respectively;
(ix) the α chain CDR1, CDR2, CDR3 amino acid sequences shown in SEQ ID NOs: 25, 26, 27 or variants having 1 or 2 amino acid residue changes from the sequences respectively; and the β chain CDR1, CDR2, CDR3 amino acid sequences shown in SEQ ID NOs: 201, 202, 203 or variants having 1 or 2 amino acid residue changes from the sequences respectively;
(x) the α chain CDR1, CDR2, CDR3 amino acid sequences shown in SEQ ID NOs: 28, 29, 30 or variants having 1 or 2 amino acid residue changes from the sequences respectively; and the β chain CDR1, CDR2, CDR3 amino acid sequences shown in SEQ ID NOs: 204, 205, 206 or variants having 1 or 2 amino acid residue changes from the sequences respectively;
(xi) the α chain CDR1, CDR2, CDR3 amino acid sequences shown in SEQ ID NOs: 31, 32, 33 or variants having 1 or 2 amino acid residue changes from the sequences respectively; and the β chain CDR1, CDR2, CDR3 amino acid sequences shown in SEQ ID NOs: 207, 208, 209 or variants having 1 or 2 amino acid residue changes from the sequences respectively;
(xii) the α chain CDR1, CDR2, CDR3 amino acid sequences shown in SEQ ID NOs: 34, 35, 36 or variants having 1 or 2 amino acid residue changes from the sequences respectively; and the β chain CDR1, CDR2, CDR3 amino acid sequences shown in SEQ ID NOs: 210, 211, 212 or variants having 1 or 2 amino acid residue changes from the sequences respectively;
(xiii) the α chain CDR1, CDR2, CDR3 amino acid sequences shown in SEQ ID NOs: 37, 38, 39 or variants having 1 or 2 amino acid residue changes from the sequences respectively; and the β chain CDR1, CDR2, CDR3 amino acid sequences shown in SEQ ID NOs: 213, 214, 215 or variants having 1 or 2 amino acid residue changes from the sequences respectively;
(xiv) the α chain CDR1, CDR2, CDR3 amino acid sequences shown in SEQ ID NOs: 40, 41, 42 or variants having 1 or 2 amino acid residue changes from the sequences respectively; and the β chain CDR1, CDR2, CDR3 amino acid sequences shown in SEQ ID NOs: 216, 217, 218 or variants having 1 or 2 amino acid residue changes from the sequences respectively;
(xv) the α chain CDR1, CDR2, CDR3 amino acid sequence shown in SEQ ID NOs: 43, 44, 45 or variants having 1 or 2 amino acid residue changes from the sequences respectively; and the β chain CDR1, CDR2, CDR3 amino acid sequence shown in SEQ ID NOs: 219, 220, 221 or variants having 1 or 2 amino acid residue changes from the sequences respectively; or
(xvi) the α chain CDR1, CDR2, CDR3 amino acid sequences shown in SEQ ID NOs: 46, 47, 48 or variants having 1 or 2 amino acid residue changes from the sequences respectively; and the β chain CDR1, CDR2, CDR3 amino acid sequences shown in SEQ ID NOs: 222, 223, 224 or variants having 1 or 2 amino acid residue changes from the sequences respectively.

In some embodiments, the TCR of the present invention comprises an α chain sequence shown in SEQ ID NO: 145, 147, 149, 151, 153, 155, 157, 159, 161, 163, 165, 167, 169, 171, 173, or 175, or a sequence having at least 90%, 91%, 92%, 93%, 94%, 95%, 96%, 97%, 98%, or 99% identity thereto; and a β chain sequence shown in SEQ ID NO: 349, 351, 353, 355, 357, 359, 361, 363, 365, 367, 369, 371, 373, 375, 377, or 379, or a sequence having at least 90%, 91%, 92%, 93%, 94%, 95%, 96%, 97%, 98%, or 99% identity thereto.

In some embodiments, the present invention provides a T-cell receptor fusion protein or a T-cell receptor conjugate, comprising the TCR as described in the first aspect of the present invention and other biologically active molecules, wherein the other biologically active molecules are e.g., antibodies, cytokines, cytotoxic agents, enzymes, radioactive substances, detectable labels, and wherein the TCR and the other biologically active molecules are linked to each other with or without a linker.

The present invention also provides a nucleic acid encoding the TCR α chain and/or β chain of the present invention.

In addition, the present invention provides a vector, preferably a plasmid, a shuttle plasmid, a phagemid, a cosmid, an expression vector, a retroviral vector, an adenoviral vector and/or a vector for homology directed repair (HDR), which comprises one or more nucleic acids as described above.

In a second aspect, the present invention provides an engineered cell transformed with the above vector and expressing the TCR described in the first aspect of the present invention.

In some embodiments, the present invention provides a method of preparing TCR-T cells expressing the exogenous TCR of the present invention through a targeting strategy without using a viral vector.

In some embodiments, the present invention provides a method of editing the genome of a human cell, comprising inserting a nucleic acid sequence comprising from N-terminal to C-terminal:
(i) a sequence encoding a first cleavable linker polypeptide;
(ii) a sequence encoding the β chain of the TCR described in the first aspect of the present invention;
(iii) a sequence encoding a second cleavable linker polypeptide;
(iv) a sequence encoding the α chain variable region of the TCR described in the first aspect of the present invention,

into a target region of exon 1 of an endogenous T-cell receptor (TCR) α chain constant region gene in a human cell;
wherein the first cleavable linker polypeptide and the second cleavable linker polypeptide are the same or different viral 2A peptides.

The exogenous TCR expressing cells prepared by said method have a high binding affinity to VVVGAVGVGK-HLA-A*11:01 complex and/or VVGAVGVGK-HLA-A*11:01 as well as a potent in vitro killing effect on SW620 cells (overexpressing HLA-A*11:01, and KRAS G12V+).

In some embodiments, the method of preparing cells expressing an exogenous TCR is implemented by knocking out the endogenous TCR and knocking in the exogenous TCR using CRISPR/Cas9 technology and homologous recombination technology.

In a third aspect, the present invention provides a method and an engineered cell with improved cell therapy efficacy.

In some embodiments, the present invention co-expresses exogenous TCR and CD8aa molecule in T cells. In some embodiments, the present invention co-expresses exogenous TCR and CD8ab molecule in T cells.

By co-expressing the CD8aa molecule and/or the CD8ab molecule with the TCR in CD8+ and CD4+ T cells, beneficial effects are produced on the functions of CD8+ and CD4+ T cells. In particular, by co-expressing the MHC class I TCR and the CD8 molecule in a CD4+ T cell, the CD4+ T cell is reprogrammed into a multifunctional hybrid T cell with both cytotoxic effect function and natural helper function.

In a fourth aspect, the present invention provides uses of the TCR described in the first aspect, the engineered cells obtained from the second aspect and the third aspect for the detection, prevention and/or treatment of cancers related to the KRAS_G12V mutant antigen.

### BRIEF DESCRIPTION OF THE DRAWINGS

The preferred embodiments of the present invention described in detail below will be better understood when read together with the attached drawings. For purposes of illustrating the present invention, the drawings show presently preferred embodiments. However, it should be understood that the present invention is not limited to the precise arrangement and means of the embodiments shown in the drawings.
Figure 1A is a diagram showing the targeting strategy for knocking an exogenous TCR into the TRAC locus using gRNA002.
Figure 1B is a diagram showing the targeting strategy for knocking out the TRBC1 and TRBC2 loci using gRNA004.
Figure 2 is a schematic diagram showing the results of the TCR gene editing efficiency detected by flow cytometry. The flow cytometry data are analyzed as a distribution diagram of cells in four quadrants (Q1, Q2, Q3, and Q4), wherein Q2 represents a cell population with completed endogenous TCR knock-out (KO) and exogenous TCR knock-in (KI) and expressing nwTCR; Q3 represents the wild-type T cells without gene editing; and Q4 represents KO cells with completed endogenous TCR knock-out.

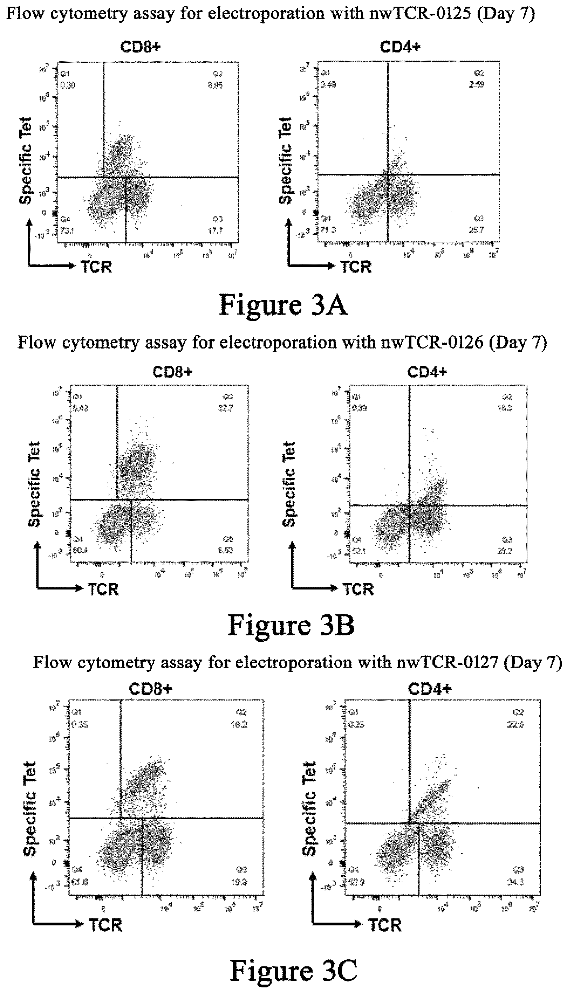

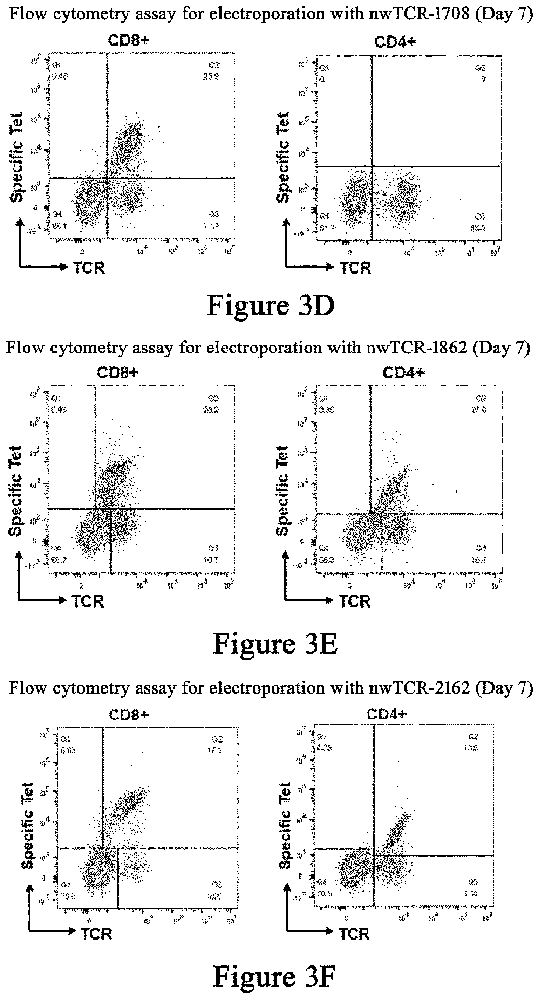

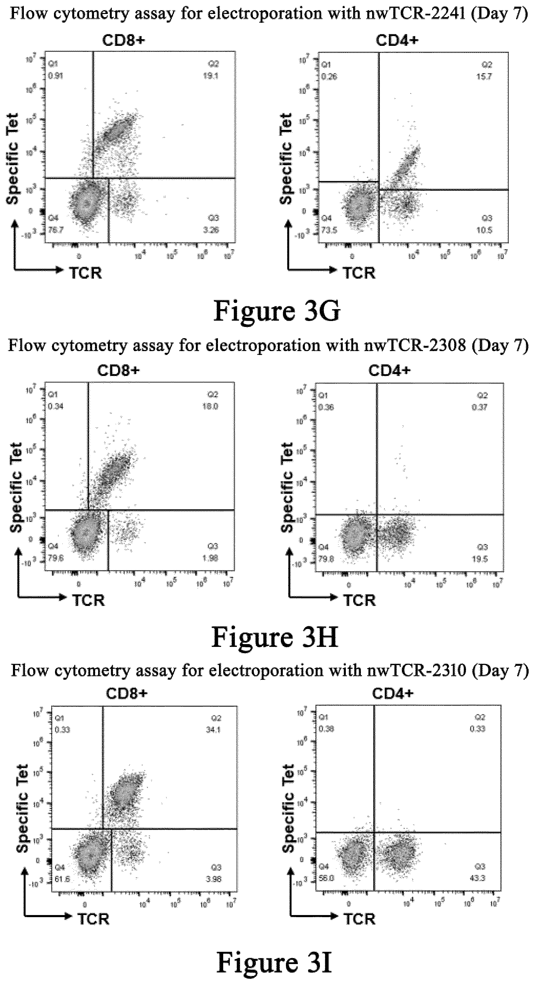

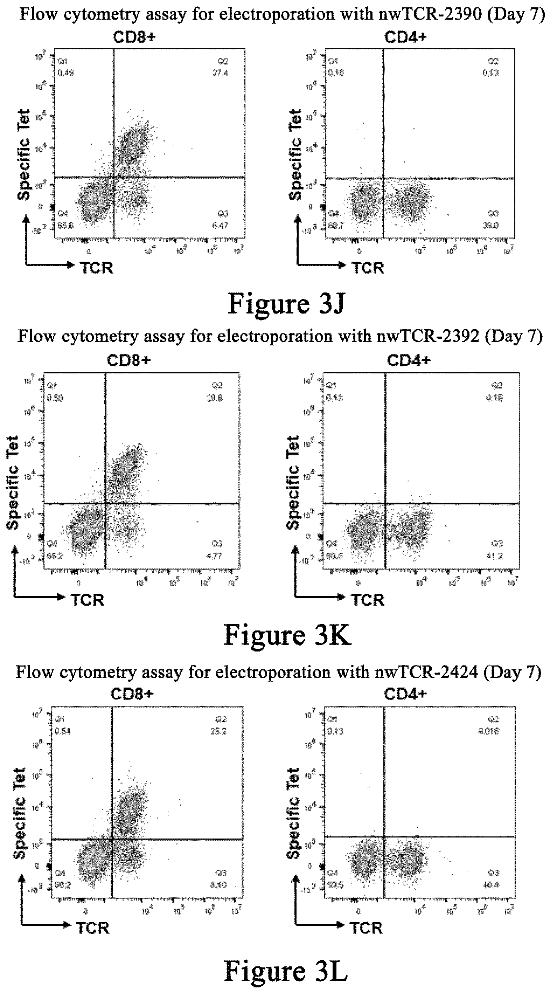

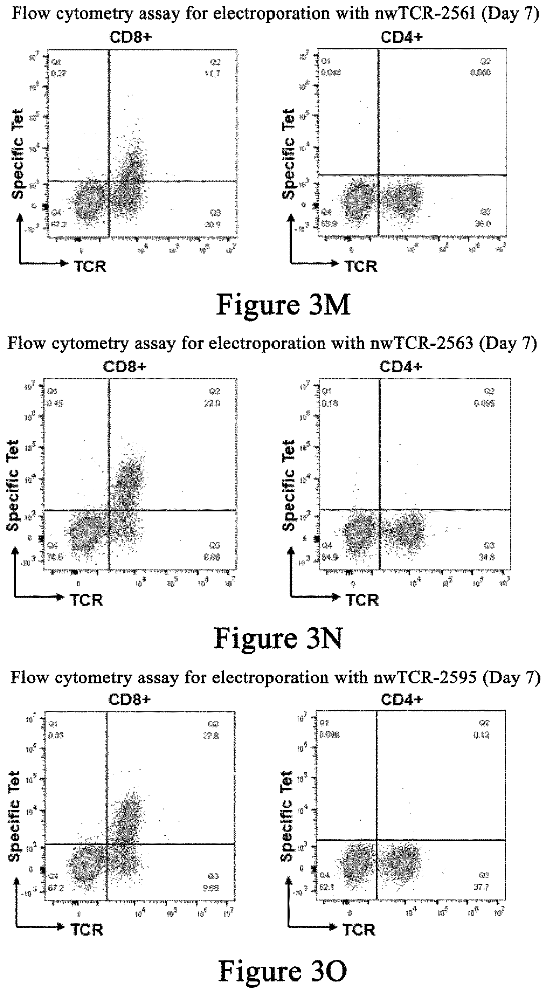

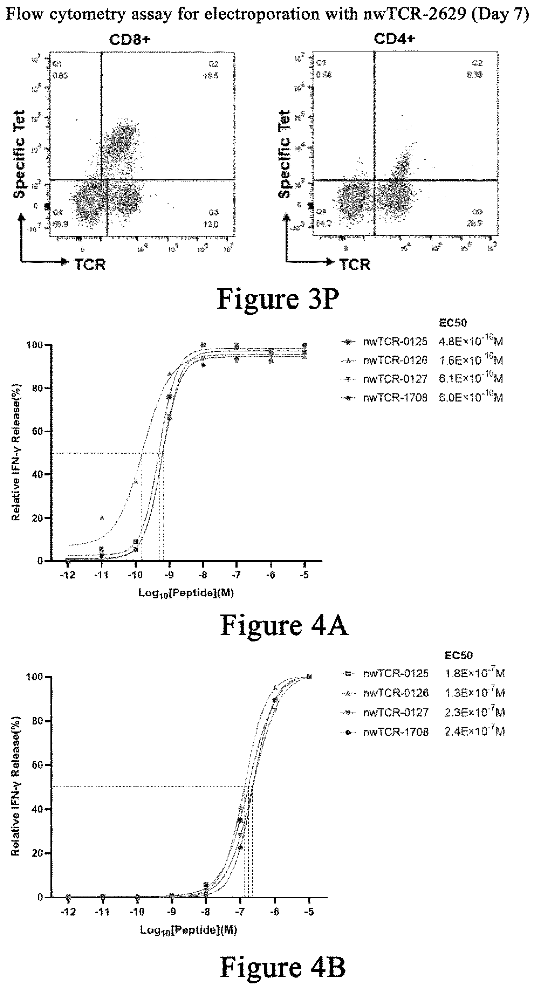

Figure 3A-Figure 3P exemplify flow cytometry results from electroporationly transfecting CD4+ T cells, CD8+ T cells with different nwTCRs and staining with pMHC tetramers. Among them, for electroporationly transfected CD4+ T cells, CD8+ T cells with nwTCR-0125, nwTCR-0126, nwTCR-0127, nwTCR-1708, nwTCR-1862, nwTCR-2162, nwTCR-2241, nwTCR-2308, or nwTCR-2563, exemplified staining results are from staining with a labeled VVVGAVGVGK-HLA-A*11:01 tetramer; and for electroporationly transfected CD4+ T cells, CD8+ T cells with nwTCR-2310, nwTCR-2390, nwTCR-2392, nwTCR-2424, nwTCR-2561, nwTCR-2595, or nwTCR-2629, the exemplified staining results are from staining with a labeled VVGAVGVGK-HLA-A*11:01 tetramer.
Figure 4A-Figure 4H show the experiment results of the binding affinity assay of T cells expressing each nwTCR for the short peptide shown in VVVGAVGVGK (SEQ ID NO: 381), or VVGAVGVGK (SEQ ID NO: 382) and presented by HLA-A*11:01, and EC50 values, respectively, wherein Figure 4A, Figure 4C, Figure 4D, and Figure 4G show the binding affinities for the short peptide shown in VVVGAVGVGK (SEQ ID NO: 381), and Figure 4B, Figure 4E, Figure 4F, and Figure 4H show the binding affinities for the short peptide shown in VVGAVGVGK (SEQ ID NO: 382).
Figure 5A-Figure 5E show *in vitro* killing effect of T cells expressing respective nwTCRs on SW620 cell line (overexpressing HLA-A11:01, and KRAS G12V+), a colorectal cancer (CRC) cell line, as a target cell. "Blank" in the Figure indicates that only target cells, without adding T cells expressing any nwTCR.
Figure 6 exemplifies the fluorescence imaging results from the killing of SW620 cells (overexpressing HLA-A*11:01, and KRAS G12V+) by T cells expressing nwTCR-2404. "Blank" in the Figure indicates that only target cells, without adding T cells expressing nwTCR-2404.
Figure 7 exemplifies real-time analysis data from the killing of SW620 cells (overexpressing HLA-A*11:01, and KRAS G12V+) by T cells expressing nwTCR-2404. The results shows that the gene-edited T cells have specific killing effect on SW620 cells (overexpressing HLA-A*11:01, and KRAS G12V+). "Blank" in the Figure indicates that only target cells, without adding T cells expressing any nwTCR.
Figure 8A is a diagram showing the targeting strategy of nwTCR-CD8a.
Figure 8B is a diagram showing the targeting strategy of nwTCR-CD8ab.
Figure 9A shows flow cytometry results from electroporationly transfecting CD4+ T cells, CD8+ T cells with nwTCR-1708 and staining with pMHC tetramer.
Figure 9B shows flow cytometry results from electroporationly transfecting CD4+ T cells, CD8+ T cells with nwTCR-1708-CD8a and staining with pMHC tetramer.
Figure 9C shows flow cytometry results from electroporationly transfecting CD4+ T cells, CD8+ T cells with nwTCR-1708-CD8ab and staining with pMHC tetramer.

### DETAILED DESCRIPTION

Before describing the present invention in detail, it should be understood that the present invention is not limited by the particular methods and experimental conditions in the specification, as the methods and conditions may be subject to change. Furthermore, the terms used herein are only for the purpose of illustrating particular embodiments and not intended to be limiting.

### I. Definitions

Unless otherwise defined, all technical and scientific terms used herein have the same meaning as commonly understood by those of ordinary skill in the art. For purpose of the present invention, the terms are defined below.

The term "about", when used in connection with a numerical value, is intended to cover a numerical value within a range having a lower limit that is 10% less than the specified numerical value and an upper limit that is 10% greater than the specified numerical value.

The term "and/or", when used in connection with two or more options, is to be understood as meaning any one of the options or any two or more of the options.

As used herein, the terms "comprise" or "include" are intended to include the elements, integers or steps described, but not to exclude any other elements, integers or steps. When used herein, the terms "comprise" or "include" also cover the situation consisting of the described elements, integers or steps, unless otherwise indicated. For example, when referring to an antibody variable region "comprising" a specific sequence, it is also intended to cover an antibody variable region consisting of the specific sequence.

"RAS protein family" belongs to a large family of small GTPase. RAS protein may be constitutively activated by a single amino acid mutation. A mutant RAS protein product is involved in signal transduction during the early stages of tumor formation in many human cancers. A variety of human cancers (e.g., lung cancer (e.g., lung adenocarcinoma), ovarian cancer (e.g., epithelial ovarian cancer), pancreatic cancer, prostate cancer, endometrial cancer, and colorectal cancer) express a mutant RAS protein.

"Kirsten rat sarcoma viral oncogene homolog (also abbreviated as KRAS protein)" is an important member of the RAS protein family. KRAS is regulated by the upstream epidermal growth factor receptor (EGFR) family, in which EGFR signaling activates SOS protein, thereby regulates KRAS activation. The transition between inactivation and activation states of an intracellular KRAS protein is determined by a molecule the intracellular KRAS binds to. Guanine nucleotide exchange factor GEF catalyzes the binding of KARS to GTP, thus activates KRAS, while GTPase-activating protein GAP promotes the hydrolysis of KRAS-bound GTP to GDP, leading to KRAS inactivation. Activated KRAS regulates its downstream signaling pathways, such as MAPK and PI3K, which are related to cell proliferation and cell migration functions, etc. Mutations in KRAS cause it to continue binding GTP and maintain its activated state, which leads to the continuous activation of the downstream signaling pathways and thus promote tumorigenesis.

The term "antigen" is any molecule that can be specifically detected by an organism's immune system.

"KRAS_G12V mutant antigen" means a KRAS protein with a G12V mutation, which can be specifically detected by an organism's immune system. "G12V" or "G12V mutation" are used interchangeably to refer to the replacement of the glycine at position 12 of the KRAS protein with a valine.

T cell receptor (TCR) is a protein on the surface of T cells, responsible for the specific recognition of an antigenic peptide bound to MHC (major histocompatibility complex). When the TCR binds to the antigenic peptide and MHC, T lymphocytes are activated through signal transduction, and enter the subsequent immune response process. There are four TCR genes in human genome, in which two TCR genes encode light chains of TCRs, with TRA gene encodes TCRα, and TRG gene encodes TCRγ; and two TCR genes encode heavy chains of TCRs, with TRB gene encodes TCRβ, and TRD gene encodes TCRδ. A heavy chain of a TCR and a light chain of the TCR form a heterodimer that makes up a complete TCR. Two types of TCRs exist in humans: TCRα/β and TCRγ/δ, wherein 95% of T cells express TCRα/β, called αβ T cells, and 5% of T cells express TCRγ/δ, called γ/δ T cells. This proportion changes during individual development and in disease states (e.g., leukemia), and varies among species.

A mature heavy chain TCR gene consists of four-part gene fragments (VDJC), which are a variable region (V), a diversity region (D), a junction region (J), and a constant region (C), while a light chain TCR gene lacks the D region (VJC). Each heavy chain and light chain of a TCR has three complementarity determining regions (CDRs) that play a major role in antigen recognition, with CDR1 and CDR2 being relatively conserved and responsible for MHC recognition; and CDR3 is the major CDR responsible for antigen recognition.

TCR gene is the most complex gene in human genome, which also has the highest degree of variation. Human peripheral blood comprises roughly 2x10¹⁶-10¹⁸ T cells expressing different TCRs. This complexity stems mainly from 3 factors: (i) compositional diversity: the VDJC/VJC structure of a mature TCR is generated by complex rearrangements. There are 65-100 V gene fragments, 2 D gene fragments and 13 J gene fragments in genome, and TCR recombination requires the selection of one from each of these three fragments, which confers a high degree of diversity to the TCR; (ii) flexibility of junction: during rearrangement, there are often random insertions or deletions of non-templated nucleotides in the V-D and D-J join regions, further increasing the diversity of CDR3 region; (iii) somatic mutation: the mutation frequency of the D region of T cells is about 1000 times higher than normal .

As known in the art, "polynucleotide" or "nucleic acid" as used interchangeably herein refers to a nucleotide chain of any length and includes DNA and RNA. A nucleotide may be a deoxyribonucleotide, a ribonucleotide, a modified nucleotide or a base and/or an analog thereof, or any substrate capable of being incorporated into the chain by a DNA- or RNA-polymerase.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In a preferred embodiment, for comparison purpose, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needleman and Wunsch algorithm ((1970) J. *Mol. Biol.,* 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna, CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

The term "antigen presenting cells" or "APCs" refers to cells of the immune system, such as helper cells (e.g., B-cells, dendritic cells, etc.), which present foreign antigens complexed with major histocompatibility complexes (MHCs) on the surface thereof. T cells can recognize these complexes using T-cell receptors (TCRs). APCs process an antigen and present the antigen to T cells.

The term "guide RNA (gRNA)" refers to an RNA specific to a target DNA, which can form a complex with a Cas protein and bring the Cas protein to the target DNA, whereby the Cas protein introduces a double-stranded break at the site of the target DNA. In the present invention, a guide RNA may consist of two RNAs, i.e. a CRISPR RNA (crRNA) and a trans-activating crRNA (tracrRNA), or a guide RNA may be a single guide RNA (sgRNA) produced by fusing the necessary portions of the crRNA and the tracrRNA.

Ribonucleoprotein (RNP) is a complex with gene editing function, which is formed by complexing the Cas9 protein and the gRNA.

CRISPR/Cas9 gene editing system mainly comprises two components: a Cas9 protein, which acts like a "wrench", and a CRISPR guide RNA, which acts like a "screw nail". The guide RNA is responsible for localizing the target site, and recruiting and activating the Cas9 protein, and the Cas9 protein is responsible for cutting the target DNA.

The term "recombinant", when used with respect to, for example, a cell, a nucleic acid, a protein or a vector, indicates that the cell, the nucleic acid, the protein or the vector has been modified by the introduction of a heterologous nucleic acid or protein, or by altering a natural nucleic acid or protein.

The term "target site" refers to any segment of DNA sequence in a target genome that is to be modified or repaired. DNA sequence in the vicinity of the target site allows for the integration of an exogenous sequence at the target site, and the integration includes, but is not limited to, gene knock-in (KI). In specific embodiments, the target DNA sequence is a double-stranded DNA sequence including, but not limited to, a DNA sequence in the chromosomal genome of a cell, a DNA sequence outside of the chromosomal genome of a cell (e.g., mitochondrial genome), a plasmid, a virus, and the like.

In the present invention, the term "site-directed recombination" means the integration of an exogenous sequence into a specific target site is in a non-random manner, including integration into 5' upstream of, 3' downstream of a specific target site, or between target sites.

In the present invention, the term "exogenous DNA sequence" means a DNA sequence that is desired to be site-directed recombinantly to a target site. The exogenous DNA sequence may be a sequence that does not occur at the target site or is an altered sequence.

The term "donor DNA" or "donor nucleic acid sequence" refers to a polynucleotide comprising a polynucleotide sequence of interest to be expressed that is inserted at a target site in the target genome. In some embodiments, the donor DNA further comprises a sequence that is homologous to the genomic sequence (also referred to as a "homology arm"). "Homology" means similar DNA sequences. The homology arm is sufficient for homologous recombination with the homologous genomic sequence. For example, the homology arm may comprise at least 50-3500 or more bases in length.

The term "homology directed repair (HDR)" refers to homologous recombination-based repair, which can be used to specifically insert a donor DNA template (coding for a sequence of interest) into a target genomic site with high efficiency, and is a repair pathway initiated by cellular DNA double-strand damage. HDR would occur when a DNA fragment homologous to the damaged DNA is present in the nucleus of the cell. HDR vector may refer to a vector for CRISPR/Cas9 and for electroporation transfection via homologous recombination technology. HDR efficiency may refer to the efficiency of gene knock-in using CRISPR/Cas9 and electroporation transfection via homologous recombination technology.

A synonymous mutation is a neutral mutation in which the genetic code is degenerate, i.e., there is mostly more than one codon that determines an amino acid, and the substitution of the third nucleotide in a triplet codon often does not change the amino acid composition. Although a third nucleotide in a triplet codon is mutated, the coded amino acid is not changed, and this mutation is a synonymous mutation.

As used herein, "vector" denotes a construct that is capable of delivering one or more genes or sequences of interest into a host cell, and preferably expressing the genes or sequences in the host cell. Examples of vectors include, but are not limited to, viral vectors, plasmids, cosmids, or phage vectors. The vector may comprise a nucleic acid sequence, such as a replication initiation region, that allows the gene or sequence of interest to replicate in the host cell. The vector may also comprise one or more selectable marker genes and other genetic elements known to those skilled in the art. The vector is preferably an expression vector comprising a nucleic acid according to the present invention, and the nucleic acid is operatively linked to a sequence that allows the expression of the nucleic acid.

The term "operatively linked to" refers to a functional ligation between a regulatory sequence for a nucleic acid expression and a nucleic acid sequence encoding a protein of interest, in order to perform an overall function. Recombinant techniques well known in the art can be used to prepare an operative ligation in a recombinant vector, and a site-specific DNA cleavage and ligation can be performed using enzymes well known in the art.

In the present invention, the term "engineered cells" refers to cells into which an exogenous nucleic acid has been introduced, including progeny of these cells. Engineered cells include "transfected cells", which include transfected primary cells as well as progeny derived therefrom, regardless of the number of passages. The progeny may not be identical in nucleic acid content to the parental cell, but may comprise mutations. Included herein are mutant progeny that have the same function or biological activity as cells screened or selected from initially transfected cells.

As used herein, "subject", "individual" refers to an animal, preferably a mammal, more preferably a human, in need of remission and/or treatment of a cancer related to the KRAS_G12V mutant antigen. Mammals also include, but are not limited to, farm animals, race animals, pets, primates, horses, dogs, cats, mice, and rats.

Adoptive Cell Transfer Therapy (ACT) refers to isolation of immunologically active cells from a subject or a patient in vivo, activation and expansion, gene editing, and other operations in vitro, then reinfusion of the cells into the patient's body, to effect the killing of target cells.

### II. T-cell receptor (TCR) of the present invention and nucleic acid encoding the TCR

The wild-type human KRAS protein is 188 amino acid residues long, with a molecular weight of about 21.6 KD, and at position 12 is glycine. Among the gene mutations in KRAS, 83% are mutations of amino acid residue at position 12, and one of the most important mutations is the mutation of glycine at position 12 to valine (also abbreviated as G12V herein).

The present invention provides an isolated or purified TCR having antigenic specificity for KRAS peptide with G12V mutation presented by a human leukocyte antigen (HLA) class I molecule. The KRAS peptide with G12V mutation presented by a human leukocyte antigen (HLA) class I molecule has any length suitable for binding to any HLA class I molecule.

In some embodiments, the KRAS peptide with G12V mutation has a length of from about 9 to about 10 amino acid residues, comprising any contiguous amino acid residues from about 9 to about 10 amino acid residues in the KRAS protein with G12V mutation. In some embodiments, the TCR of the present invention has antigenic specificity for the KRAS peptide with G12V mutation, and the mutated KRAS peptide has a length of about 9 amino acid residues or about 10 amino acid residues. Examples of KRAS peptide having G12V mutation and recognized by the TCR of the present invention are a short peptide from amino acid position 7 to 16 of KRAS shown in VVVGAVGVGK (SEQ ID NO:381)(also abbreviated as "KRAS_G12V_7-16 peptide" herein); and a short peptide from amino acid position 8 to 16 of KRAS shown in VVGAVGVGK (SEQ ID NO:382) (also abbreviated as "KRAS_G12V_8-16 peptide" herein).

T cell receptor (TCR) is a molecule present on the surface of T cells that is responsible for recognizing the antigenic peptide-MHC complex (i.e., pMHC). The specific binding of TCR to the antigenic peptide-MHC complex triggers the activation of the T cell through a series of biochemical events mediated by associated enzymes, co-receptors, and auxiliary molecules. In 95% of T cells, the TCR heterodimer consists of α and β chains, whereas in 5% of T cells, the TCR heterodimer consists of γ and δ chains.

Each chain of the TCR is a member of the immunoglobulin superfamily and has an N-terminal immunoglobulin (Ig) variable (V) domain, an Ig constant (C) domain, a transmembrane region (i.e., transmembrane domain), and a short cytoplasmic tail at C-terminus. In the variable domains of the TCR α and β chains, each variable domain has three highly variable regions or complementarity determining regions (CDRs), wherein CDR3 in each variable domain is the major CDR responsible for recognizing a processed antigen. It is believed that CDR2 recognizes MHC molecule.

The constant domain of the TCR consists of a short connecting sequence, in which cysteine residues form a disulfide bond that creates a linkage between TCR α and β chains.

During T cell maturation, the TCR and CD3 form a TCR/CD3 complex. The process of TCR/CD3 complex formation usually proceeds in the following order; firstly, the three peptide chains, CD3γ, δ, and ε, become a stable complex core through the formation of two heterodimers, γ-ε and δ-ε, to which TCRαβ (or TCRγδ) binds, subsequently, ζ-ζ or ζ-η dimer binds to TCRαβ (or TCRγδ)/CD3γεδε complex, and finally transferred to T cell surface. Signal is transduced from TCR to intracellular via TCR/CD3 complex.

Signaling from the TCR/CD3 complex is enhanced by the simultaneous binding of MHC to a specific co-receptor. In helper T cells, the co-receptor is a CD4 molecule, and the CD4 molecule is specific for MHC class II, while in cytotoxic T cells, the co-receptor is a CD8 molecule, and the CD8 molecule is specific for MHC class I.

As used herein, the term "T cell receptor" has a conventional meaning in the art and is used to denote a molecule that recognizes a peptide presented by an MHC molecule. The TCR molecule is a heterodimer, with two chains α and β (or optionally γ and δ).

The TCRs of the present invention provide specific affinity recognition of KRAS_G12V mutant antigen. The KRAS_G12V mutant antigen is degraded intracellularly by proteasome into short peptides from 8 to 10 amino acids in length, e.g., KRAS_G12V_7-16 peptide as shown in SEQ ID NO: 381 and/or KRAS_G12V_8-16 peptide as shown in SEQ ID NO: 382. These short peptides are presented on cell surface by MHC class I as peptide/MHC complex (pMHC). Some pMHCs have been verified to be associated with various cancers, and may be potential targets for TCR therapy.

The present invention provides isolated or purified T cell receptor (TCR) α chain and/or β chain. The TCR of the present invention may be a hybrid TCR comprising sequences derived from more than one species. For example, given that a murine TCR can be expressed more efficiently in human T cells than a human TCR, the TCR of the present invention may comprise a human variable region and a murine constant region.

In one embodiment, the TCR of the present invention comprises an α chain and a β chain, wherein each of the α and β chains comprises three complementarity determining regions (CDRs), and wherein the amino acid sequence of CDR3 of the α chain is selected from SEQ ID NOs: 3, 6, 9, 12, 15, 18, 21, 24, 27, 30, 33, 36, 39, 42, 45, 48, and a variant having one or two amino acid residue changes from the sequence, and the amino acid sequence of CDR3 of the β chain is selected from SEQ ID NOs: 179, 182, 185, 188, 191, 194, 197, 200, 203, 206, 209, 212, 215, 218, 221, 224, and a variant having one or two amino acid residue changes from the sequence.

In one embodiment, the TCR of the present invention comprises an α chain and a β chain, wherein the amino acid sequences of the three complementarity determining regions (CDRs) comprised in the α chain and the amino acid sequences of the three CDRs comprised in the β chain of the TCR of the present invention are:
(i) the α chain CDR3 amino acid sequence shown in SEQ ID NO: 3 or a variant having 1 or 2 amino acid residue changes from the sequence; and the β chain CDR3 amino acid sequence shown in SEQ ID NO: 179 or a variant having 1 or 2 amino acid residue changes from the sequence;
(ii) the α chain CDR3 amino acid sequence shown in SEQ ID NO: 6 or a variant having 1 or 2 amino acid residue changes from the sequence; and the β chain CDR3 amino acid sequence shown in SEQ ID NO: 182 or a variant having 1 or 2 amino acid residue changes from the sequence;
(iii) the α chain CDR3 amino acid sequence shown in SEQ ID NO: 9 or a variant having 1 or 2 amino acid residue changes from the sequence; and the β chain CDR3 amino acid sequence shown in SEQ ID NO: 185 or a variant having 1 or 2 amino acid residue changes from the sequence;
(iv) the α chain CDR3 amino acid sequence shown in SEQ ID NO: 12 or a variant having 1 or 2 amino acid residue changes from the sequence; and the β chain CDR3 amino acid sequence shown in SEQ ID NO: 188 or a variant having 1 or 2 amino acid residue changes from the sequence;
(v) the α chain CDR3 amino acid sequence shown in SEQ ID NO: 15 or a variant having 1 or 2 amino acid residue changes from the sequence; and the β chain CDR3 amino acid sequence shown in SEQ ID NO: 191 or a variant having 1 or 2 amino acid residue changes from the sequence;
(vi) the α chain CDR3 amino acid sequence shown in SEQ ID NO: 18 or a variant having 1 or 2 amino acid residue changes from the sequence; and the β chain CDR3 amino acid sequence shown in SEQ ID NO: 194 or a variant having 1 or 2 amino acid residue changes from the sequence;
(vii) the α chain CDR3 amino acid sequence shown in SEQ ID NO: 21 or a variant having 1 or 2 amino acid residue changes from the sequence; and the β chain CDR3 amino acid sequence shown in SEQ ID NO: 197 or a variant having 1 or 2 amino acid residue changes from the sequence;
(viii) the α chain CDR3 amino acid sequence shown in SEQ ID NO: 24 or a variant having 1 or 2 amino acid residue changes from the sequence; and the β chain CDR3 amino acid sequence shown in SEQ ID NO: 200 or a variant having 1 or 2 amino acid residue changes from the sequence;
(ix) the α chain CDR3 amino acid sequence shown in SEQ ID NO: 27 or a variant having 1 or 2 amino acid residue changes from the sequence; and the β chain CDR3 amino acid sequence shown in SEQ ID NO: 203 or a variant having 1 or 2 amino acid residue changes from the sequence;
(x) the α chain CDR3 amino acid sequence shown in SEQ ID NO: 30 or a variant having 1 or 2 amino acid residue changes from the sequence; and the β chain CDR3 amino acid sequence shown in SEQ ID NO: 206 or a variant having 1 or 2 amino acid residue changes from the sequence;
(xi) the α chain CDR3 amino acid sequence shown in SEQ ID NO: 33 or a variant having 1 or 2 amino acid residue changes from the sequence; and the β chain CDR3 amino acid sequence shown in SEQ ID NO: 209 or a variant having 1 or 2 amino acid residue changes from the sequence;
(xii) the α chain CDR3 amino acid sequence shown in SEQ ID NO: 36 or a variant having 1 or 2 amino acid residue changes from the sequence; and the β chain CDR3 amino acid sequence shown in SEQ ID NO: 212 or a variant having 1 or 2 amino acid residue changes from the sequence;
(xiii) the α chain CDR3 amino acid sequence shown in SEQ ID NO: 39 or a variant having 1 or 2 amino acid residue changes from the sequence; and the β chain CDR3 amino acid sequence shown in SEQ ID NO: 215 or a variant having 1 or 2 amino acid residue changes from the sequence;
(xiv) the α chain CDR3 amino acid sequence shown in SEQ ID NO: 42 or a variant having 1 or 2 amino acid residue changes from the sequence; and the β chain CDR3 amino acid sequence shown in SEQ ID NO: 218 or a variant having 1 or 2 amino acid residue changes from the sequence;
(xv) the α chain CDR3 amino acid sequence shown in SEQ ID NO: 45 or a variant having 1 or 2 amino acid residue changes from the sequence; and the β chain CDR3 amino acid sequence shown in SEQ ID NO: 221 or a variant having 1 or 2 amino acid residue changes from the sequence; or
(xvi) the α chain CDR3 amino acid sequence shown in SEQ ID NO: 48 or a variant having 1 or 2 amino acid residue changes from the sequence; and the β chain CDR3 amino acid sequence shown in SEQ ID NO: 224 or a variant having 1 or 2 amino acid residue changes from the sequence.

In one embodiment, the TCR of the present invention comprises an α chain sequence shown in SEQ ID NO: 145, 147, 149, 151, 153, 155, 157, 159, 161, 163, 165, 167, 169, 171, 173, or 175, or a sequence having at least 90%, 91%, 92%, 93%, 94%, 95%, 96%, 97%, 98%, or 99% identity thereto; and a β chain sequence shown in SEQ ID NO: 349, 351, 353, 355, 357, 359, 361, 363, 365, 367, 369, 371, 373, 375, 377, or 379, or a sequence having at least 90%, 91%, 92%, 93%, 94%, 95%, 96%, 97%, 98%, or 99% identity thereto. Preferably, the constant region of the TCR of the present invention is a mouse constant region.

In some embodiments, the amino acid residue changes in a TCR variant of the present invention are substitutions, additions, or deletions of amino acid residues in the α chain sequence shown in any one of SEQ ID NOs: 145, 147, 149, 151, 153, 155, 157, 159, 161, 163, 165, 167, 169, 171, 173, or 175, in the β chain sequence shown in any one of SEQ ID NOs: 349, 351, 353, 355, 357, 359, 361, 363, 365, 367, 369, 371, 373, 375, 377, or 379, provided that the TCR variant still retains or improves the ability to bind to the KRAS_G12V mutant antigen epitope peptide-MHC complex. In one embodiment, the substitution is a conservative substitution. Examples of the conservative substitution are given in Table A below.

**Table A**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids can be grouped according to common side chain properties:
(1) Hydrophobic: Norleucine, Met, Ala, Val, Leu; Ile;
(2) Neutral hydrophilic: Cys, Ser, Thr, Asn; Gln;
(3) Acidic: Asp, Glu;
(4) Basic: His, Lys, Arg;
(5) Residues affecting chain orientation: Gly, Pro;
(6) Aromatic: Trp, Tyr, Phe.

A non-conservative substitution would exchange a member of one of these classifications for a member of the other classifications.

In some embodiments, the TCR of the present invention can recognize and bind to the epitope peptide of mutant KRAS protein presented by HLA class I molecule, triggering an immune response.

In some embodiments, the HLA class I molecule is any HLA-A molecule, e.g., the HLA class I molecule is an HLA-A11 molecule. The HLA-A11 molecule may be any HLA-A11 molecule. Examples of HLA-A11 molecule include, but are not limited to, HLA-A*11:01, HLA-A*11:02, HLA-A*11:03, or HLA-A*11:04. Preferably, the HLA class I molecule is an HLA-A*11:01 molecule. The HLA-A*11:01 molecule is the most common HLA-A molecule in Asians.

The present invention also relates to a nucleic acid encoding a TCR of the present invention or a portion thereof, and the portion of the TCR is, for example, one or more CDRs; one or more variable regions; an α chain; or a β chain, and the like. The nucleic acid may be double stranded or single stranded, and may be RNA or DNA. The nucleic acid sequence may be codon-optimized to achieve high expression in mammalian producer cells. Codon usages for mammalian cells and a variety of other organisms are well known in the art. Codon optimization may also include removing mRNA unstable motifs and cryptic splice sites.

The TCR of the present invention may be modified by a variety of methods (e.g., gene fusion, chemical conjugation, etc.) such that the TCR is linked to other bioactive molecules. The TCR that can be linked to other bioactive molecules can be a TCR heterodimer or a soluble form thereof, more preferably a soluble, single chain TCR. The other bioactive molecules can be a variety of biologically active effectors, e.g., antibodies, cytokines, cytotoxic agents, enzymes, radioactive substances, detectable markers, etc. The TCR and other bioactive molecules have or do not have a linker therebetween.

In some embodiments, a TCR fusion protein is a fusion of a TCR with an antibody, and the antibody comprises an intact antibody (e.g., IgG, IgM, or IgA class) or a fragment thereof (e.g., Fv, Fab, Fab', Fab'-SH, F(ab')₂, a diabody, a single chain antibody (e.g., scFv), a single domain antibody); and a multi-specific antibody (e.g., a bispecific antibody).

In some embodiments, a TCR fusion protein is a fusion of a TCR with a cytokine, and the cytokine is, for example, an interleukin (e.g., IL-2), a chemokine (e.g., MIP-1β), a growth factor (e.g., GCSF).

In some embodiments, a TCR conjugate is a TCR covalently attached to a cytotoxic agent, such as adriamycin.

In some embodiments, a TCR conjugate is a TCR covalently attached to a radioactive material, such as I¹²¹.

In some embodiments, a TCR conjugate is a TCR covalently attached to a detectable marker, such as a fluorescent marker.

The T cell receptor fusion protein or T cell receptor conjugate of the present invention can be used for a variety of applications, including in vivo detection of cells and/or imaging of cells or tissues, as well as therapeutic uses, such as in vivo or in vitro killing of target cells or target tissues expressing the KRAS_G12V mutant antigen and binding the TCR specifically.

### III. Vector comprising the nucleic acid encoding the TCR of the present invention

The present invention also relates to a vector comprising the nucleic acid encoding the TCR of the present invention. In one embodiment, the vector is pUC57-Simple vector (purchased from GenScript Biotech Corporation). In yet another embodiment, pUC57-HA vector is used, which is an optimized vector based on the pUC57-Simple vector, retaining only Ori and Amp sequences of the pUC57-Simple vector, and then replacing Amp sequence with Kana sequence, and adding left and right homology arm (HA) sequences (around 800 bp) corresponding to the TRAC locus.

The vector transfers the nucleic acid encoding the TCR of the present invention into a cell, such as a T cell, an NK cell, a stem cell, e.g., a pluripotent stem cell, an induced pluripotent stem cell (iPSC), such that the engineered cell expresses the KRAS_G12V mutant antigen-specific TCR.

The KRAS_G12V mutant antigen-specific TCR refers to such a TCR that specifically binds to and immunologically recognizes G12V mutant KRAS with high affinity. For example, after co-culturing about 1×10⁴ to about 1×10⁵ T cells expressing the TCR with antigen-presenting cells such as T2 cells or K562 cells pulsed by G12V mutant KRAS and overexpressing HLA class I molecules, the secretion of IFN-γ is induced with an EC50 of the TCR of about 1×10⁻⁷ M or less (e.g., 1×10⁻⁸ M or less, 1×10⁻⁹ M or less, 1×10⁻¹⁰ M or less), and the TCR is considered to be antigenically specific for the G12V mutant KRAS. HLA class I molecule may be any of the HLA class I molecules described herein (e.g., HLA-A*11:01 molecule).

Preferably, the vector enables sustained high-level expression of the introduced exogenous TCR in the engineered cells (e.g., the engineered T cells), and the introduced exogenous TCR may successfully compete with an endogenous TCR for a limited pool of CD3 molecules. Alternatively, increasing the supply of CD3 molecules may also increase the exogenous TCR expression in the genetically modified cells. Thus, the vector optionally comprises CD3-γ, CD3-δ, CD3-ε and/or CD3-ζ genes. In one embodiment, the vector comprises CD3-ζ gene. Further, one or more separate vectors encoding CD3 gene(s) may be provided with an exogenous TCR encoding vector for co-transfer into the cells.

The form of the vector is not limited to a homology directed repair (HDR) vector, but can also be a viral vector. The viral vector may be a lentiviral vector, an adenoviral vector, an adeno-associated virus (AAV) vector, a herpesvirus vector, a retroviral vector, a baculoviral vector, for genome editing in cells.

Genome editing technology refers to a technology that involves insertions, deletions or substitutions of nucleic acids in a cellular genome DNA. Genetically modified T cells using the gene editing technology for human primary T cells have demonstrated excellent efficacy in clinical trials of a variety of adoptive immunotherapeutic drugs. Among them, Chimeric Antigen Receptors (CARs) or T Cell Receptors (TCRs) are often used to modify human primary T cells to effect the recognition of a specific target epitope. These engineered T cells can specifically kill specific target cells.

Common TCR gene editing means can be generally categorized into two types according to gene integration manner, one type is the random integration of a gene, including Lenti Virus (LV) system, Adeno-Associated Virus (AAV) system, Transposon system, etc., and the other type is the precise gene editing approach, including Zinc Finger Nucleases (ZFN), Transcription Activator-like Effector Nucleases (TALEN), and Clustered Regularly Interspaced Short Palindromic Repeats (CRISPR) technology, etc. Among them, CRISPR technology has the advantages of being easy to manipulate and having stronger expandability since it recognizes and edits DNA through gRNA guidance, and performs site-directed insertion of a large gene fragment in a homologous recombination manner.

### IV. Preparation of engineered cells

A viral vector can be used to introduce a desired TCR into cells. However, the viral vector-based method of introducing exogenous TCRα/β genes into cells without knocking out the endogenous TCR of the cells may result in a mismatch of the exogenous TCR α chain and β chain. Even though the issue of the mismatch of the exogenous TCR α chain and β chain can be reduced by modifying disulfide bond or by changing to a murine constant region, the viral vector is inserted randomly into the cellular genome, and this still brings about a potential risk of disrupting other genes.

A non-viral vector may also be used to introduce a desired TCR into cells to precisely integrate the exogenous TCRα/β genes into a specific genomic locus of the cell. In some embodiments, a gene editing method not based on a viral vector can knock out the endogenous T cell receptor α chain and β chain of human-derived T cells by CRISPR/Cas9 technology and homologous recombination technology, and knock in nucleotides encoding desired exogenous T cell receptor α chain and β chain at an exon of the TRAC gene. As a result, the endogenous TCR expression is disrupted and the endogenous TCR promoter is employed to express the desired exogenous TCR α chain and β chain.

In one embodiment, nucleotides encoding the desired exogenous T cell receptor α chain and β chain are knocked into exon 1 of the endogenous TRAC gene, so no TRAC gene is required to be inserted into the exogenous knock-in fragment, thereby reducing the length of the fragment for the gene knock-in, and decreasing the difficulty of the gene knock-in. Compared to the technique of expressing TCR using a viral vector, the technique of expressing TCR via a non-viral vector approach can serve as a rapid, straightforward, and cost-effective way for introducing exogenous TCRα/β genes into cells.

### IV.1 Selection of knock-out site

TCR is a dimer consisting of TCR α chain and TCR β chain. TCR α chain gene is formed by the rearrangement of TRAV, TRAJ, and TRAC genes, wherein TRAV and TRAJ genes comprise multiple sequences respectively, and there are differences among these multiple sequences. Only one of these multiple sequences respectively can be randomly selected during the rearrangement, to be expressed. If TRAV and TRAJ genes are selected as knock-out sites, then it is difficult to avoid the generation of any random TCR α chain gene. Given there is only one TRAC gene, and knocking out the TRAC gene can bring about knocking out any random TCR α chain gene, TRAC is suitable as a knock-out site. TCR β chain gene is formed by the rearrangement of TRBV, TRBJ, TRBD, and TRBC genes, wherein TRBV and TRBJ genes comprise multiple sequences respectively, and there are differences among these multiple sequences, thus TRBV and TRBJ genes are not suitable as knock-out sites. TRBC gene comprises both TRBC1 and TRBC2 comprising a partially identical sequence, and the common sequence can be selected as a knock-out site. Any random TCR β gene is knocked out by knocking out the common sequence.

In some embodiments, one or more of an endogenous TRAC gene, an endogenous TRBC1 gene, and/or a TRBC2 gene are knocked out. In some embodiments, the endogenous TRAC gene and the endogenous TRBC1 and TRBC2 genes are knocked out simultaneously, which results in a higher endogenous TCR knockout efficiency and reduces the mismatch risk between chains of an exogenous TCR and an endogenous TCR derived from endogenous TCR expression.

Nuclease-based genome editing tools can be utilized to target and disrupt endogenous TRAC gene and TRBC gene through non-homologous end joining (NHEJ) induced double-strand breaks and DNA repair. These tools include meganucleases, zinc finger nucleases (ZFNs), transcription activator-like effector nucleases (TALENs), megaTAL nucleases, and CRISPR/CRISPR-associated protein 9 (CRISPR/Cas9).

### IV.2 Selection of knock-in site

Since an endogenous TRAC gene is unique and required for all TCRα expression, an endogenous TRAC site is selected as an exogenous TCRα/β gene knock-in site, whereby an endogenous TCR is eliminated, meanwhile an endogenous TCR promoter of the human-derived T cells can be employed to express an exogenous TCRα/β gene of the present invention (also referred to as the "nwTCR" gene) without additionally adding TRAC gene, thereby reducing the size of a knock-in fragment, which is conducive to improving the efficiency of gene editing.

In some embodiments, an expression construct of nwTCR is cloned into a targeting vector (e.g., pUC57-S vector), and nwTCR is knocked into the constant region of the TCR α chain site-directedly via designed homology arms, with the expression of nwTCR regulated by the transcriptional regulatory sequence of the locus. Since the regulation level by the endogenous promoter at the knock-in site is better than that of other sites, it ensures the continuous and stable expression of the nwTCR gene.

### IV.3 Engineered cells

The present invention provides an engineered cell expressing an exogenous TCR.

In some embodiments, the engineered cells expressing a TCR are prepared from cells derived from blood, bone marrow, lymph or lymphoid organs, e.g., lymphocytes or stem cells, wherein the lymphocytes include, but are not limited to, T-cells, NK-cells, and the stem cells are e.g., pluripotent stem cells, induced pluripotent stem cells (iPSCs).

The cells are typically primary cells, e.g., cells isolated directly from a subject and/or isolated from a subject and frozen. The cells may be allogeneic cells and/or autologous cells.

In some embodiments, RNPs and plasmids are employed to electroporationly transfect CD3/CD28-activated primary cells (e.g., sorted CD4+ T cells and CD8+ T cells) via CRISPR/Cas9 and homologous recombination technology, thereby preparing engineered TCR cells.

In some embodiments, sgRNA is designed to target an endogenous TRAC gene, and sgRNA is designed to target endogenous TRBC1 gene and TRBC2 gene.

Cas9 protein guided by sgRNA binds to a specific site in a target genome, then cleaves the specific site. For double-strand break generated in an endogenous TRAC gene due to the use of RNP, a homologous recombination may occur in the presence of a donor DNA with homology arms, thus enabling site-directed insertion of the nwTCR gene of interest.

In a specific embodiment, the specific site in the target genome to which Cas9 protein guided by sgRNA binds is located at exon 1 of TRAC gene, and the Cas9 protein cleaves the specific site. The sgRNA recognition sequence and PAM sequence targeted efficiently by the designed and validated sequence comprises a nucleotide sequence shown in TCAGGGTTCTGGATATCTGT-GGG (i.e., sgRNA recognition sequence shown in SEQ ID NO: 383 - PAM sequence shown in SEQ ID NO: 385, wherein "-" is used to separate the CRISPR/Cas9 recognition site and the PAM sequence).

In a specific embodiment, the specific site in the target genome to which Cas9 protein guided by sgRNA binds is located at exon 1 of TRBC1 and TRBC2 genes, and the Cas9 protein cleaves the specific sites. The sgRNA recognition sequence and PAM sequence targeted efficiently by the designed and validated sequence comprises a nucleotide sequence shown in CTGCCTGAGCAGCCGCCTGA-GGG (i.e., sgRNA recognition sequence shown in SEQ ID NO: 384 - PAM sequence shown in SEQ ID NO: 385, wherein "-" is used to separate the CRISPR/Cas9 recognition site and the PAM sequence).

CRISPR/Cas system may comprise Cas component in the form of a Cas protein or in the form of a nucleic acid encoding a Cas protein.

In the present invention, the Cas protein may be any Cas protein, provided that it has endonuclease activity or nickase activity when complexed with a guide RNA.

Preferably, the Cas protein is a Cas9 protein or a variant thereof or a functional fragment thereof.

The Cas protein may be, but is not limited to, a protein isolated from organisms such as Streptococcus sp., preferably Streptococcus pyogens, or a recombinant protein.

In one embodiment, the Cas protein comprises Cas9 derived from Streptococcus pyogens, such as Cas9 having an amino acid sequence shown in SEQ ID NO: 405.

In another embodiment, the Cas protein comprises an amino acid sequence having at least 50% homology to the amino acid sequence shown in SEQ ID NO: 405, preferably having at least 60, 70, 80, 90, 95, 97, 98, or 99% homology to the amino acid sequence shown in SEQ ID NO: 405, but not limited thereto.

In the present invention, a Cas protein coding nucleic acid may be in a vector, such as in a plasmid comprising a Cas coding sequence under the control of a promoter such as CMV promotor or CAG promotor. When the Cas protein is Cas9, the Cas9 coding sequence may be derived from Streptococcus sp., preferably from Streptococcus pyogens. For example, the Cas9 coding nucleic acid may comprise a nucleotide sequence encoding SEQ ID NO: 405. In addition, the Cas9 coding nucleic acid may comprise a nucleotide sequence having at least 50% homology to the nucleotide sequence encoding SEQ ID NO: 405, preferably having at least 60, 70, 80, 90, 95, 97, 98, or 99% homology to the nucleotide sequence encoding SEQ ID NO: 405, but is not limited thereto.

In one embodiment, a donor DNA comprises, in sequence, a 5' homology arm, a sequence encoding a cleavable linker polypeptide, an exogenous TCRα/β gene or a functional fragment thereof, and a 3' homology arm. After the sequence encoding the cleavable linker polypeptide is expressed, the cleavable linker polypeptide is cleaved. In some embodiments, the cleavable linker polypeptide sequence comprises a 2A ribosome skipping element such as T2A, E2A, P2A, and F2A.

In one embodiment, the donor DNA is comprised in a targeting vector. Without specifically restricting the basic targeting vector used as a backbone, as long as it has a prokaryotic replication origin for the vector proliferation in bacteria and a selectable label.

In a preferred embodiment, in order to increase the expression of the exogenous TCRα/β gene or fragments thereof, in the targeting vector a sequence encoding a cleavable linker polypeptide and a signal peptide sequence are linked at the respective N-terminal end of the exogenous TCRα chain gene and of the exogenous TCRβ chain gene.

In a specific embodiment, a targeting vector for knocking in an nwTCR gene sequence comprises the following operatively linked structure: 5'-2A ribosome skipping element-SP-TCRβ-2A ribosome skipping element-SP-TRAV-TRAJ-3', wherein:
SP is a signal peptide coding sequence.

A targeting vector for knocking in an nwTCR gene sequence, an RNP complex, and cells are mixed, then a delivery step of the nwTCR gene sequence to the cells is performed. In some embodiments, the delivery step is selected from: electroporation, transfection, deformation of the cell membrane by physical means, lipid nanoparticles (LNPs), virus-like particles (VLPs), and sonication. In some embodiments, the delivery step comprises electroporation.

In some embodiments, the engineered cells are primary cells.

In some embodiments, the engineered cells are isolated cells, wherein the isolated cells are isolated from a subject.

In some embodiments, the engineered cells are ex vivo cultured cells. In some embodiments, the ex vivo cultured cells comprise stimulated cells. In some embodiments, the stimulated cells comprise cytokine-stimulated T cells. Optionally, the cytokine-stimulated T cells comprise CD3-stimulated T cells, CD28-stimulated T cells, or CD3 and CD28 co-stimulated T cells. In some embodiments, the cytokine-stimulated T cells are cultured in the presence of IL7, IL15, or a combination thereof. In some embodiments, the cytokine-stimulated T cells are cultured in the presence of IL2.

In some embodiments, the engineered cells are stem cells, e.g., hematopoietic stem cells (HSCs). Transfer of an nwTCR gene to HSCs does not result in expression of TCR on the cell surface because stem cells do not express CD3 molecules. However, when stem cells differentiate into lymphoid precursor cells that migrate to thymus, the initiation of CD3 expression would result in expression of the introduced nwTCR on the surface of the thymocytes. The advantage of the approach is that mature T cells, once generated, express only the introduced nwTCR, and little or no expression of endogenous TCR chain, because the expression of the introduced nwTCR chain inhibits the rearrangement of endogenous TCR gene fragments to form functional TCR α and β genes. An additional benefit of the approach is that TCR genetically modified stem cells are a continuous source of mature T cells having desired antigen specificity. Thus, nwTCR genetically modified stem cells generate T cells expressing the TCR of the present invention upon differentiation.

### V. Methods for detecting, preventing, or treating cancers related to KRAS_G12V mutant antigen

The present invention provides a method for preventing or treating a cancer associated with KRAS_G12V mutant antigen, comprising administering to a subject in need thereof an engineered cell of the present invention, a TCR nucleic acid, a vector, or a pharmaceutical composition of the present invention. In some embodiments, the method comprises administering a polynucleotide encoding the TCR. In some embodiments, the method comprises administering a vector comprising a polynucleotide encoding the TCR. In some embodiments, the method comprises administering an effective amount of the engineered cells of the present invention.

In some embodiments, an engineered cell of the present invention, a TCR nucleic acid, a vector, or a pharmaceutical composition of the present invention is used to prevent or treat cancers related to KRAS_G12V mutant antigen. Without being restricted by any theory, it is believed that the TCR of the present invention is capable of specifically binding to KRAS_G12V mutant antigen and, thereby, mediating an immune response against a target cell expressing the KRAS_G12V mutant antigen.

The treatment or prevention may include treatment or prevention of one or more symptoms of the cancer being treated or prevented, including promoting tumor regression, delaying the onset of the cancer or its symptoms, and preventing or delaying recurrence of the cancer or its symptoms.

The present invention also provides a method for detecting the presence of a cancer in a mammal. The method comprises (i) contacting a sample comprising one or more cells from a mammal with any one of a TCR of the present invention, a population of cells expressing a TCR of the present invention, or a pharmaceutical composition comprising a population of cells expressing a TCR of the present invention described herein, to form a complex; and (ii) detecting the complex, wherein the detected complex is indicative of the presence of the cancer in the mammal. The contact may be implemented in vitro or in vivo in the mammal. In one embodiment, the contact is implemented in vitro. The complex can be detected in a variety of ways known in the art. In some embodiments, a TCR of the present invention or a population of cells expressing a TCR of the present invention is labeled with a detectable marker, for example, a radioisotope, a fluorophore (e.g., fluorescein isothiocyanate (FITC), phycoerythrin (PE)), an enzyme (e.g., alkaline phosphatase, horseradish peroxidase), and an elemental particle (e.g., gold particle).

The present invention further provides a method for inducing anti-tumor immunity, comprising administering to a subject an effective amount of engineered cells of the present invention, wherein the tumor is a KRAS__G12V mutant antigen-associated tumor.

The present invention provides a method for inducing an immune response in a subject, comprising administering an effective amount of engineered cells of the present invention. In some embodiments, the immune response is a T cell-mediated immune response. In some embodiments, the T cell-mediated immune response is directed against one or more target cells. In some embodiments, the engineered immune cell comprises a TCR of the present invention. In some embodiments, the target cell is a KRAS_G12V mutant antigen-associated cancer cell.

In some embodiments, donor T cells for T cell therapy are obtained from a patient (e.g., for autologous T cell therapy). In other embodiments, donor stem cells to be differentiated into T cells for T cell therapy are obtained from a subject who is not a patient.

The T cells may be administered in a therapeutically effective amount. For example, the therapeutically effective amount of T cells may be at least about 10⁴ cells, at least about 10⁵ cells, at least about 10⁶ cells, at least about 10⁷ cells, at least about 10⁸ cells, at least about 10⁹ cells, or at least about 10¹⁰ cells per kg body weight.

The cancer mentioned in the various methods of the present invention may be any cancer including, but not limited to: acute lymphoblastic cancer, acute myeloid leukemia, chronic lymphoblastic leukemia, chronic myeloid cancer, Hodgkin's lymphoma, non-Hodgkin's lymphoma, brain cancer, glioma, nasopharyngeal cancer, ocular carcinoma, oral cavity carcinoma, neck carcinoma, esophageal carcinoma, liver cancer, intrahepatic cholangiocarcinoma, gallbladder carcinoma, lung carcinoma, bone cancer, breast cancer, gastrointestinal tumor, colon cancer, small intestine cancer, colorectal cancer, rectal cancer, gastric cancer, skin cancer, melanoma, multiple myeloma, cervical cancer, endometrial cancer, uterine cancer, ovarian cancer, ureteral cancer, bladder cancer, penile cancer, testicular cancer, pancreatic cancer, prostate cancer, kidney cancer, soft tissue cancer, and thyroid cancer. Preferably, the cancer is lung cancer, pancreatic cancer, colorectal cancer, endometrial cancer, ovarian cancer or prostate cancer.

### VI. Methods for improving cell therapy and engineered cells

The present invention further provides methods for improving cell therapy and engineered cells thereof.

Naturally occurring CD8+ cells express CD8 molecules. A CD8 molecule is a type I transmembrane glycoprotein expressed on the cell surface in the form of a homodimer of two CD8a chains (also abbreviated herein as "CD8aa") and/or in the form of a heterodimer of one CD8a chain and one CD8b chain (also abbreviated herein as "CD8ab").

In some embodiments, the T cells of the present invention co-express an exogenous TCR and a CD8aa molecule. Using a non-viral gene editing method based on CRISPR/Cas9 technology, CD8+ T cells/CD4+ T cells are gene edited by a nucleic acid encoding an nwTCR of the present invention and a nucleic acid encoding CD8a chain, demonstrating that co-expression of the exogenous nwTCR and CD8aa molecule in the CD8+ T cells/CD4+ T cells can enhance the binding of the TCR-T cells to pMHC molecule. When the exogenous nwTCR and CD8aa molecule are co-expressed in CD8+ T cells, it is expected to improve TCR-specific cytotoxicity (including its continuous killing ability) and in vivo anti-tumor function of CD8+ T cells due to the increased number of CD8aa molecules in CD8+ T cells available for utilization by the exogenous nwTCR. When the exogenous nwTCR and CD8aa molecule are co-expressed in CD4+ T cells, accompanied by the expression of endogenous CD4 molecules, the CD4+ T cells exhibit a hybrid phenotype. It is expected to recognize an antigen with an affinity similar to that of natural CD8+ T cells and kill target cells, demonstrating a function of cytotoxic effect; while preserving the natural helper function of CD4+ T cells.

Further in some embodiments, the present invention co-expresses an exogenous TCR and a CD8ab molecule in T cells. Using a non-viral gene editing method based on CRISPR/Cas9 technology, CD8+ T cells/CD4+ T cells are gene edited by a nucleic acid encoding an nwTCR of the present invention, a nucleic acid encoding CD8a chain, and a nucleic acid encoding CD8b chain, demonstrating that co-expression of the exogenous nwTCR and CD8ab molecule in the CD8+ T cells/CD4+ T cells can enhance the binding of the TCR-T cells to pMHC molecule. When the exogenous nwTCR and CD8ab molecule are co-expressed in CD8+ T cells, it is expected to improve TCR-specific cytotoxicity (including its continuous killing ability) and in vivo anti-tumor function of CD8+ T cells due to the increased number of CD8ab molecules in CD8+ T cells available for utilization by the exogenous nwTCR. When the exogenous nwTCR and CD8ab molecule are co-expressed in CD4+ T cells, accompanied by the expression of endogenous CD4 molecules, the CD4+ T cells exhibit a hybrid phenotype. It is expected to recognize an antigen with an affinity similar to that of natural CD8+ T cells and kill target cells, demonstrating a function of cytotoxic effect; meanwhile to preserve the natural helper function of CD4+ T cells.

Thus, co-expression of CD8aa molecules and/or CD8ab molecules with TCR genes in CD8+ and CD4+ T cells has beneficial effects on the functions of CD8+ and CD4+ T cells. CD4+ T cells can be reprogrammed using MHC class I TCRs and CD8 molecules into multifunctional hybrid T cells exhibiting both a cytotoxic effect function and a natural helper function.

The following Examples are described to aid in the understanding of the present invention. It is not intended and should not be construed in any way that Examples are a limitation on the scope of protection of the present invention.

### EXAMPLES

The present invention as generally described herein will be more readily understood by reference to following Examples, which are provided by way of illustration and are not intended to limit the scope of the present invention. These Examples are not intended to indicate that the following experiments are all the experiments performed or the only ones performed.

### Example 1. Generation and cloning of T cells and TCRs recognizing KRAS_G12V mutant antigen

The nearby sequences at position 12 of KRAS with amino acid residue at position 12 mutated from G to V were chemically synthesized, i.e., a short peptide from amino acid position 7 to 16 of KRAS shown in VVVGAVGVGK(SEQ ID NO:381)(also abbreviated as "KRAS_G12V_7-16 peptide" herein); and a short peptide from amino acid position 8 to 16 of KRAS shown in VVGAVGVGK(SEQ ID NO:382) (also abbreviated as "KRAS_G12V_8-16 peptide" herein).

Dendritic cells (DC cells) derived from a cancer patient with HLA-A*11:01 genotype and expressing KRAS_G12V were subject to *in vitro* pulse stimulation using KRAS_G12V_7-16 peptide or KRAS_G12V_8-16 peptide suspended in DMSO, respectively, and were co-cultured for 10 days with CD8+ T cells sorted from peripheral blood of the patient. Negative control was stimulation of DC cells from the patient using DMSO in vitro pulse stimulation and co-culture with CD8+ T cells sorted from peripheral blood of the patient for 10 days.

Reactive T cells releasing IFN-γ and expressing CD137 were then sorted by detecting the release of cytokine IFN-γ in culture supernatants and the expression of CD137 on CD8+ T cells. Flow cytometry-based staining was used to assess the binding of the sorted reactive T cells releasing IFN-γ and expressing CD137 to a tetramer of a peptide-MHC (HLA-A*11:01) (VVVGAVGVGK-HLA-A*11:01 and VVGAVGVGK-HLA-A*11:01); and a tetramer comprising an unrelated peptide was used as a negative control. The flow cytometry staining of T cells using the two kinds of tetramers (VVVGAVGVGK-HLA-A*11:01 tetramer or VVGAVGVGK-HLA-A*11:01 tetramer, respectively) increased the confidence of T cell specificity.

Sixteen specific T cell clones with the desired high affinity were screened out. The antigen-specific T-cell receptors (TCRs) on these 16 T-cell clones that specifically bind to the KRAS_G12V_7-16 epitope peptide or the KRAS_G12V_8-16 epitope peptide were named as nwTCR-0125, nwTCR-0126, nwTCR-0127, nwTCR-1708, nwTCR-1862, nwTCR-2162, nwTCR-2241, nwTCR-2308, nwTCR-2310, nwTCR-2390, nwTCR-2392, nwTCR-2424, nwTCR-2561, nwTCR-2563, nwTCR-2595, and nwTCR-2629, and were sequenced using high-throughput paired-TCR sequencing. Amino acid sequences of the paired TCR α and β chains on these 16 T cell clones were determined on a single-cell basis.

Since several nucleotides can be translated into the same amino acid, and the frequency of codon usage is different in different organisms, codon optimizations of nucleotide sequences encoding the amino acid sequences of TCR α chain and β chain were carried out with the aim of increasing the expression of the TCR when expressed in eukaryotic cells. After codon optimization, nucleotide sequences of the 16 TCRs specifically recognizing KRAS_GI12V_7-16 epitope peptide or KRAS_G12V_8-16 epitope peptide were obtained.

Table 1A and Table 1B showed respectively the amino acid sequence information and nucleotide sequence information generated by sequencing of the α chain and β chain of the 16 TCRs expressed by clonal T cell lines.

**Table 1A. Amino acid and nucleotide sequences of the α chains in KRAS_G12V mutant antigen-specific TCRs**

| Name | Amino acid sequence | Nucleotide sequence |
|---|---|---|
| nwTCR-0125 complete TRA (TCR α chain) | SEQ ID NO: 145 | SEQ ID NO: 146 |
| nwTCR-0126 complete TRA (TCR α chain) | SEQ ID NO: 147 | SEQ ID NO: 148 |
| nwTCR-0127 complete TRA (TCR α chain) | SEQ ID NO: 149 | SEQ ID NO: 150 |
| nwTCR-1708 complete TRA (TCR α chain) | SEQ ID NO: 151 | SEQ ID NO: 152 |
| nwTCR-1862 complete TRA (TCR α chain) | SEQ ID NO: 153 | SEQ ID NO: 154 |
| nwTCR-2162 complete TRA (TCR α chain) | SEQ ID NO: 155 | SEQ ID NO: 156 |
| nwTCR-2241 complete TRA (TCR α chain) | SEQ ID NO: 157 | SEQ ID NO: 158 |
| nwTCR-2308 complete TRA (TCR α chain) | SEQ ID NO: 159 | SEQ ID NO: 160 |
| nwTCR-2310 complete TRA (TCR α chain) | SEQ ID NO: 161 | SEQ ID NO: 162 |
| nwTCR-2390 complete TRA (TCR α chain) | SEQ ID NO: 163 | SEQ ID NO: 164 |
| nwTCR-2392 complete TRA (TCR α chain) | SEQ ID NO: 165 | SEQ ID NO: 166 |
| nwTCR-2424 complete TRA (TCR α chain) | SEQ ID NO: 167 | SEQ ID NO: 168 |
| nwTCR-2561 complete TRA (TCR α chain) | SEQ ID NO: 169 | SEQ ID NO: 170 |
| nwTCR-2563 complete TRA (TCR α chain) | SEQ ID NO: 171 | SEQ ID NO: 172 |
| nwTCR-2595 complete TRA (TCR α chain) | SEQ ID NO: 173 | SEQ ID NO: 174 |
| nwTCR-2629 complete TRA (TCR α chain) | SEQ ID NO: 175 | SEQ ID NO: 176 |

**Table 1B. Amino acid and nucleotide sequences of the β chains in KRAS_G12V mutant antigen-specific TCRs**

| Name | Amino acid sequence | Nucleotide sequence |
|---|---|---|
| nwTCR-0125 complete TRB (TCR β chain) | SEQ ID NO: 349 | SEQ ID NO: 350 |
| nwTCR-0126 complete TRB (TCR β chain) | SEQ ID NO: 351 | SEQ ID NO: 352 |
| nwTCR-0127 complete TRB (TCR β chain) | SEQ ID NO: 353 | SEQ ID NO: 354 |
| nwTCR-1708 complete TRB (TCR β chain) | SEQ ID NO: 355 | SEQ ID NO: 356 |
| nwTCR-1862 complete TRB (TCR β chain) | SEQ ID NO: 357 | SEQ ID NO: 358 |
| nwTCR-2162 complete TRB (TCR β chain) | SEQ ID NO: 359 | SEQ ID NO: 360 |
| nwTCR-2241 complete TRB (TCR β chain) | SEQ ID NO: 361 | SEQ ID NO: 362 |
| nwTCR-2308 complete TRB (TCR β chain) | SEQ ID NO: 363 | SEQ ID NO: 364 |
| nwTCR-2310 complete TRB (TCR β chain) | SEQ ID NO: 365 | SEQ ID NO: 366 |
| nwTCR-2390 complete TRB (TCR β chain) | SEQ ID NO: 367 | SEQ ID NO: 368 |
| nwTCR-2392 complete TRB (TCR β chain) | SEQ ID NO: 369 | SEQ ID NO: 370 |
| nwTCR-2424 complete TRB (TCR β chain) | SEQ ID NO: 371 | SEQ ID NO: 372 |
| nwTCR-2561 complete TRB (TCR β chain) | SEQ ID NO: 373 | SEQ ID NO: 374 |
| nwTCR-2563 complete TRB (TCR β chain) | SEQ ID NO: 375 | SEQ ID NO: 376 |
| nwTCR-2595 complete TRB (TCR β chain) | SEQ ID NO: 377 | SEQ ID NO: 378 |
| nwTCR-2629 complete TRB (TCR β chain) | SEQ ID NO: 379 | SEQ ID NO: 380 |

### Example 2. Preparation of KRAS_G12V mutant antigen-specific TCR-T cells from T cells

This Example described knocking out TCR gene in primary T cells by CRISPR/Cas9 technology and knocking in KRAS_G12V mutant antigen-specific TCR gene by homologous recombination technology, thereby KRAS_G12V mutant antigen-specific TCR-T cells were prepared and characterized.

### 2.1 Sorting and activation of T cells

A mixture of CD4 T-cells and CD8 T-cells (also referred to as "CD4/CD8 T-cells" herein) was enriched and sorted from peripheral blood mononuclear cells (PBMC, purchased from: Shanghai Saily Biotechnology Co., Ltd., Donor: S2001095). The enriched and sorted CD4/CD8 T cells were aliquoted and frozen (5x10⁶ cells/cryotube) for future use.

A cryotube was thawed as needed, and the sorted T cells were activated by adding a 1:100-fold dilution of T cell activator Miltenyi T cell TransACT (Miltenyi catalog No. 130-111-160) to T cell culture media (e.g. RPMI 1640, FBS, L-glutamine, non-essential amino acids, sodium pyruvate, HEPES buffer, 2-mercaptoethanol, and optionally IL2), and cultured for approximately 48 hours (2 days), then used for electroporation transfection.

### 2.2 Targeting strategy and preparation of targeting vector

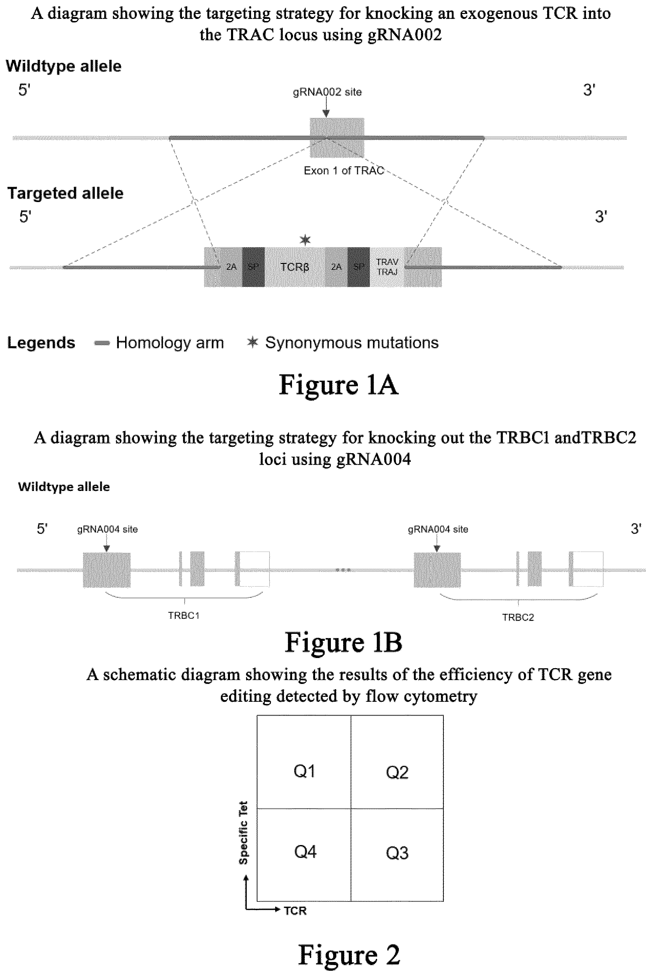

gRNA002 and gRNA004 (see Table 2) were used as gRNAs, wherein gRNA002's hit site was at exon 1 of endogenous TRAC gene (Figure 1A); gRNA004's hit sites were at exon 1 of endogenous TRBC1 gene and at exon 1 of endogenous TRBC2 gene (Figure 1B). Cas9 enzyme was purchased from GenScript Biotech Corporation, catalog No. Z03469.

**Table 2. sgRNAs corresponding to TRAC and TRBC genes**

| sgRNA name | sgRNA recognition site | PAM sequence | Target gene |
|---|---|---|---|
| gRNA002 | TCAGGGTTCTGGATATCTGT (SEQ ID NO: 383) | GGG (SEQ ID NO: 385) | TRAC gene |
| gRNA004 | CTGCCTGAGCAGCCGCCTGA (SEQ ID NO: 384) | GGG (SEQ ID NO: 385) | TRBC1 gene, and TRBC2 gene |

The targeting vector (also referred to as HDR vector) used a backbone of pUC57-HA vector, which was optimized on the basis of pUC57-Simple vector. It retained only the Ori and Amp sequences of the pUC57-Simple vector, then replaced the Amp sequence with Kana sequence, and inserted left and right homology arm (HA) sequences (around 800 bp) of TRAC locus. The gene sequence to be knocked-in (KI) could be constructed between the left HA and the right HA. KI sequence construct structure of an nwTCR comprised: 2A or 2A variant-SP-TCRβ-2A or 2A variant-SP-TRAV-TRAJ, wherein 2A represented a ribosome skipping element; SP represented a signal peptide; and four synonymous mutation bases were introduced into the TRBC gene in the targeting vector, which were mutation of TRBC S77 coding nucleotide from AGC to TCC and mutation of S78 coding nucleotide from AGC to TCC, respectively. With respect to nwTCR-0126, the sequence of the KI sequence construct was shown in SEQ ID NO: 386, wherein: HA represented a homology arm (5'HA was shown in SEQ ID NO: 387, 3'HA was shown in SEQ ID NO: 391; 2A or 2A variant was shown in SEQ ID NO: 388 or SEQ ID NO: 390, respectively; SP represented a signal peptide sequence shown in SEQ ID NO: 389; TCR β represented a nucleotide sequence of nwTCR-0126 complete TRB (TCR β chain) (SEQ ID NO: 352), and the TRBC S77 coding nucleotide was mutated from AGC to TCC and the S78 coding nucleotide was mutated from AGC to TCC, thereby introducing four synonymous mutation bases to the TRBC gene; TRAV represented a nucleotide sequence of nwTCR-0126 TRAV gene (SEQ ID NO: 54); TRAJ represented a nucleotide sequence of nwTCR-0126 TRAJ gene (SEQ ID NO:56). Similarly, KI sequence constructs of nwTCR-0125, nwTCR-0127, nwTCR-1708, nwTCR-1862, nwTCR-2162, nwTCR-2241, nwTCR-2308, nwTCR-2310, nwTCR-2390, nwTCR-2392, nwTCR-2424, nwTCR-2561, nwTCR-2563, nwTCR-2595, and nwTCR-2629 were prepared.

### 2.3 Transfection by electroporation (Day 2)

RNP was prepared by mixing the sgRNAs of Example 2.2 with Cas9 enzyme thoroughly and incubating for 10 min at room temperature.

The targeting vector comprising the KI TCR sequence prepared in Example 2.2 was thoroughly mixed with the incubated RNP, and the indicated concentration of T cells prepared in Example 2.1 (about 1.25E6 T-cells/electroporation tube), in order to knock-out (KO) endogenous TCR and knock-in (KI) exogenous TCR.

The above mixture was loaded on an electroporation transfection apparatus (Celetrix; Catalog No. CTX-1500A LE) for cell electroporation transfection, which was performed at 480-560 V for 20 ms.

After the electroporation transfection, the electroporation-transfected T cells were left to stand for 15 min, then taken out from the electroporation tube, and transferred into pre-warmed media (ImmunoCult^{™}-XF T Cell Expansion Medium, Stemcell Inc., Catalog No. 10981). After culturing the cells for 5 days, flow cytometry characterization was performed on Day 7.

### 2.4 Flow cytometry analysis of nwTCR expression (Day 7)

The cell suspension obtained from Example 2.3 was thoroughly mixed, cell counting was performed, and appropriate amounts of cells were collected for staining of two labeled peptide-MHC(HLA-A*11:01) tetramers, which were VVVGAVGVGK-HLA-A*11:01 tetramer and VVGAVGVGK-HLA-A*11:01 tetramer, respectively, abbreviated as pMHCs.

Each stain comprising any of the two labeled peptide-MHC(HLA-A*11:01) tetramers, and LIVE/DEAD^{™} Fixable Near-IR, purchased from Invitrogen, Catalog No. L10119; CD4-FITC, purchased from BioLegend, Catalog No. 357406; CD8-PerCP-cy5.5, purchased from BioLegend, Catalog No. 344710; anti-human TCR α/β-BV510 antibody, purchased from BioLegend, Catalog No. 306734, were prepared in advance.

Collected cells were stained with the two labeled peptide-MHC(HLA-A*11:01) tetramer stains, washed, and characterized by flow cytometry.

Figure 3A-Figure 3P exemplified flow cytometry results from CD4+ T cells, CD8+ T cells electroporationly transfected with different nwTCRs and stained with pMHC tetramers, wherein for nwTCR-0125, nwTCR-0126, nwTCR-0127, nwTCR-1708, nwTCR-1862, nwTCR-2162, nwTCR-2241, nwTCR-2308, and nwTCR-2563, the exemplified staining results were from staining with the labeled VVVGAVGVGK-HLA-A*11:01 tetramer; and for nwTCR-2310, nwTCR-2390, nwTCR-2392, nwTCR-2424, nwTCR-2561, nwTCR-2595, and nwTCR-2629, the exemplified staining results were from staining with the labeled VVGAVGVGK-HLA-A*11:01 tetramer. As shown, on Day7 cells were classified into three populations:
1) Wild-type T cells without gene editing (Q3);
2) KO cells with completed endogenous TCR knock-out (Q4);
3) Cell population with completed KO and KI, and expressing nwTCR (Q2).

The results of the TCR gene editing efficiency detected by flow cytometry were shown schematically in Figure 2.

As seen in Figure 3A-Figure 3P, CD8+ T cells having each knocked-in nwTCR respectively could bind to the peptide-MHC complex (pMHC) tetramer; while CD4+ T cells having each knocked-in nwTCR respectively were significantly different in the binding to the peptide-MHC complex (pMHC) tetramer. This is because, as shown in Example 1, each nwTCR in the present invention was obtained by screening CD8+ T cells. Thus, when each nwTCR was knocked into CD8+ T cells respectively, CD8+ T cells expressing each nwTCR could bind specifically to the peptide-MHC complex (pMHC) tetramers; whereas, when each nwTCR was knocked into CD4+ T cells respectively, there were following situations with respect to CD4+ T cells expressing each nwTCR: in general, when the affinity of the nwTCR for MHC was sufficiently strong, the TCR could bind MHC molecules without the assistance of CD8 molecules, e.g., it was deemed that nwTCR-0127 had a stronger binding ability to MHC molecules than that of nwTCR-1708. Thus, they differed in the staining of CD4+ T cells with a pMHC tetramer. Hence, after editing each nwTCR into CD4+ T cells respectively and expressing, nwTCRs with strong affinity could still bind to MHC class I antigen, while nwTCRs with poor affinity had weak ability to bind to MHC class I antigen.

### Example 3. In vitro functional studies of KRAS_G12V mutant antigen-specific TCR-T cells

The KRAS_G12V mutant antigen-specific TCR-T cells of Example 2 were selectively activated with TransACT activator (Miltenyi, Catalog No. 130-111-160) on Day7. Culturing of the TCR-T cells was continued until Day14. In vitro functional studies were performed on respective TCR-T cells on Day14.

### 3.1 Affinity detection of respective TCR-T cells for peptide

The affinity assay of TCR-T cells was performed as follows. Antigen-presenting cells (HLA-A*11:01 overexpressing T2 cells or K562 cells) were collected, and counted. The cells were resuspended by adding an appropriate amount of medium (e.g., RPMI-1640 medium, purchased from Gibco, Catalog No. 22400089; FBS, purchased from Gibco, Catalog No. 10099141C) to a cell density of 1E6 cells/mL. 1 mL of cell suspension was added to each well of a 24-well plate, respectively. The polypeptide solutions to be detected (the polypeptides were KRAS_G12V_7-16 peptide shown in SEQ ID NO: 381 and/or KRAS_G12V_8-16 peptide shown in SEQ ID NO:382) were subjected to gradient dilutions to 10⁻¹⁰-10⁻³ M, and 10 µl of the diluted polypeptide solutions were added into the corresponding well of the 24-well plate, respectively. The cells were incubated in an incubator (37°C, 5% CO₂) for 2 h. After incubation, the antigen-presenting cells were collected and washed, and 100 µl of the antigen-presenting cells at 1E6/mL were added to corresponding wells of 96-well plates. The nwTCR-T cells to be detected were collected, added an appropriate amount of T cell culture medium (purchased from STEMCELL, Catalog No. 10981) to a cell density of 1E6/mL, and 100 µl of the cell suspension was added to the corresponding wells of 96-well plate. Various nwTCR-T cells were co-cultured with the antigen-presenting cells (37°C, 5% CO₂) for 16h respectively. The cell supernatants were collected to detect the concentration of IFN-γ by ELISA kit (purchased from Biolegend, Catalog No. 430104). The binding affinity of T cells expressing each nwTCR for the short peptide shown in SEQ ID NO: 381 or SEQ ID NO: 382 and presented by HLA-A*11:01 was assayed by detecting the level of IFN-γ release.

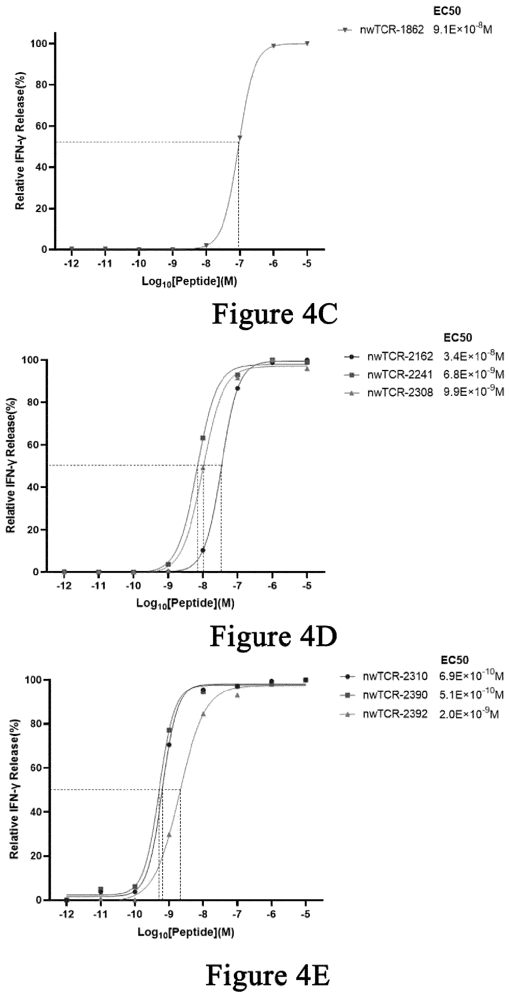

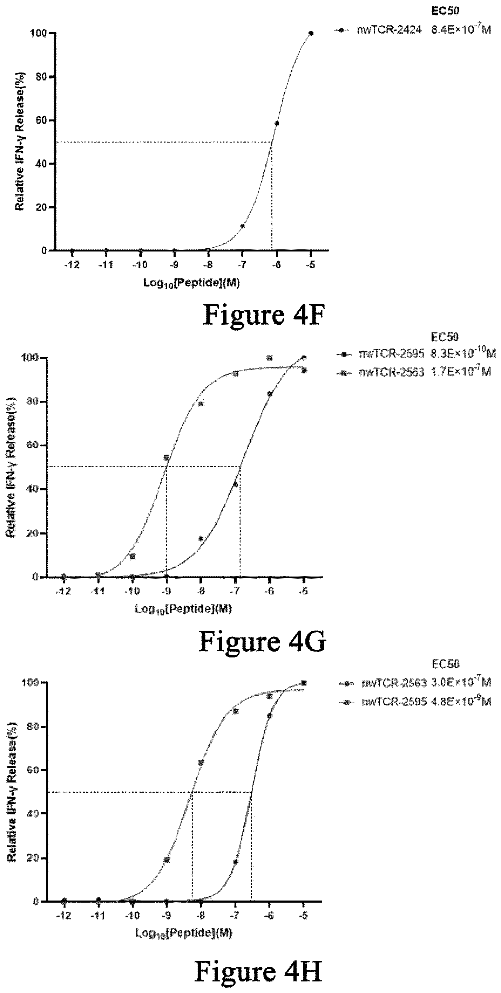

Figure 4A-Figure 4H showed the experiment results of the binding affinity assay of T cells expressing each nwTCR for the short peptide shown in VVVGAVGVGK(SEQ ID NO: 381) or VVGAVGVGK(SEQ ID NO: 382) and presented by HLA-A*11:01, and EC50 values, respectively, wherein Figure 4A, Figure 4C, Figure 4D, and Figure 4G showed the binding affinities for the short peptide shown in VVVGAVGVGK(SEQ ID NO: 381), and Figure 4B, Figure 4E, Figure 4F, and Figure 4H showed the binding affinities for the short peptide shown in VVGAVGVGK(SEQ ID NO: 382).

The results in Figure 4A-Figure 4H suggested that after co-incubation of T2 cells presenting the peptide-MHC complex with T cells expressing each nwTCR, T cells expressing each nwTCR were detected to bind specifically to the peptide-MHC complex, resulting in IFN-γ release. In addition, considering that both 9-mer and 10-mer short peptides were likely to be presented by MHCs when MHCs present polypeptides, the results in Figure 4A-Figure 4F of the experiments performed using 9-mer and 10-mer short peptides indicated that T cells expressing each nwTCR could specifically bind to the pMHC complex presenting 9-mer or 10-mer short peptide, respectively.

### 3.2 Killing of target cells by respective TCR-T cells

The killing assay of respective TCR-T cells on target cells was performed as follows. Target cells (i.e, SW620 cell line (overexpressing HLA-A*11:01, and KRAS G12V+) (purchased from Nanjing Cobioer Biosciences Co., Ltd.) were collected, counted, and resuspended using target cell medium (RPMI-1640 medium, purchased from Gibco, Catalog No. 22400089, and FBS, purchased from Gibco, Catalog No. 10099141C) to a cell density of 1E6 cells/mL. E-plate (obtained from Agilent, Catalog No. 300600890) was prepared by adding 100 µL of well-mixed target cell suspension to corresponding wells, then placed into RTCA real-time cell analyzer (purchased from Agilent, Model: xCELLigence RTCA DP), and detected overnight.

Various TCR-T cells to be detected were collected, counted, and resuspend with an appropriate amount of T-cell culture medium (purchased from STEMCELL, Catalog No. 10981). The E-plate inoculated with target cells as described above was taken out, and T-cell suspension was added. The E-Plate was placed back into the RTCA analyzer for detection. A 72-hour cell index was obtained. Each independent experiment was performed in triplicate. The change ratio of the cell index was evaluated by automatically calculating the slope of the intervals using RTCA software. To demonstrate the effect of the treatment, the cell indices were normalized to an equal value at normalized time points.

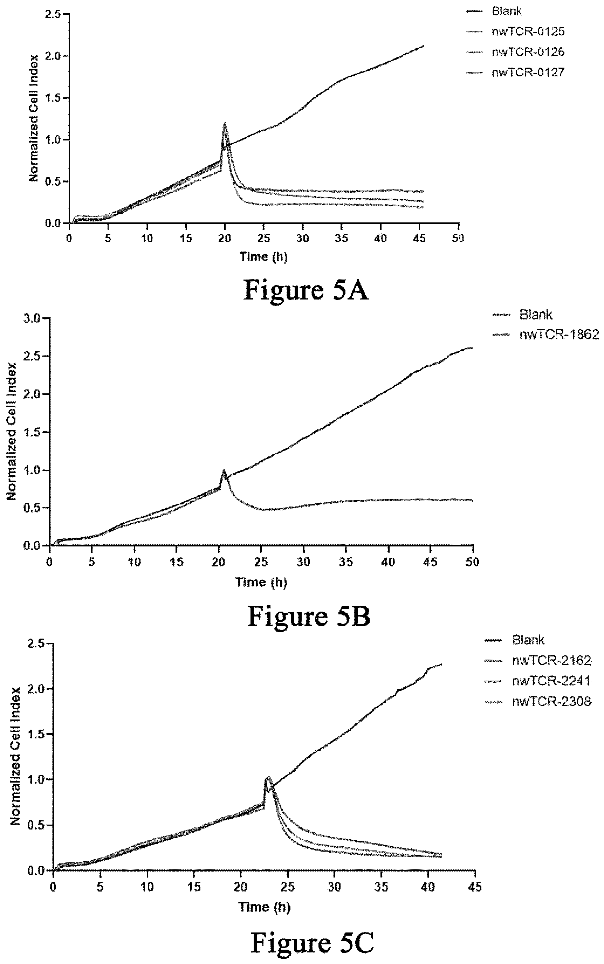

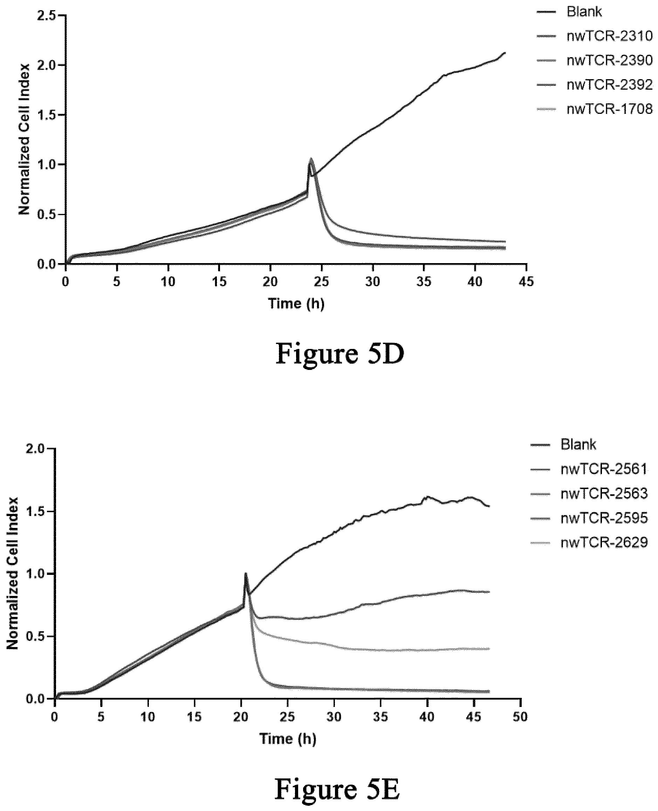

The in vitro killing results of respective TCR-T cells on SW620 cells (overexpressing HLA-A*11:01, and KRAS G12V+) were shown in Figure 5A-Figure 5E, demonstrating that respective TCR-T cells showed significant in vitro killing effect on SW620 cell line (overexpressing HLA-A*11:01, and KRAS G12V+) as target cells.

The nwTCR-T cells to be detected were collected, added an appropriate amount of T cell culture medium (purchased from STEMCELL, Catalog No. 10981) to a cell density of 1E6/mL. 100 µL of the nwTCR-T cell suspension to be detected was added to the wells of a 96-well plate, and mixed with SW620 (overexpressing HLA-A*11:01, and KRAS G12V+) in the wells of the 96-well plate. To this cell mixture, 1 µL of ethidium bromide (1 mg/mL) solution was added, and after mixing well, the cell culture plate was placed in a real-time fluorescence imaging system (BioTek Lionheart) for a cell killing characterization experiment. The target cells specifically recognized by the T cells would be stained by ethidium bromide and exhibit red fluorescent signals after entering an apoptotic state.

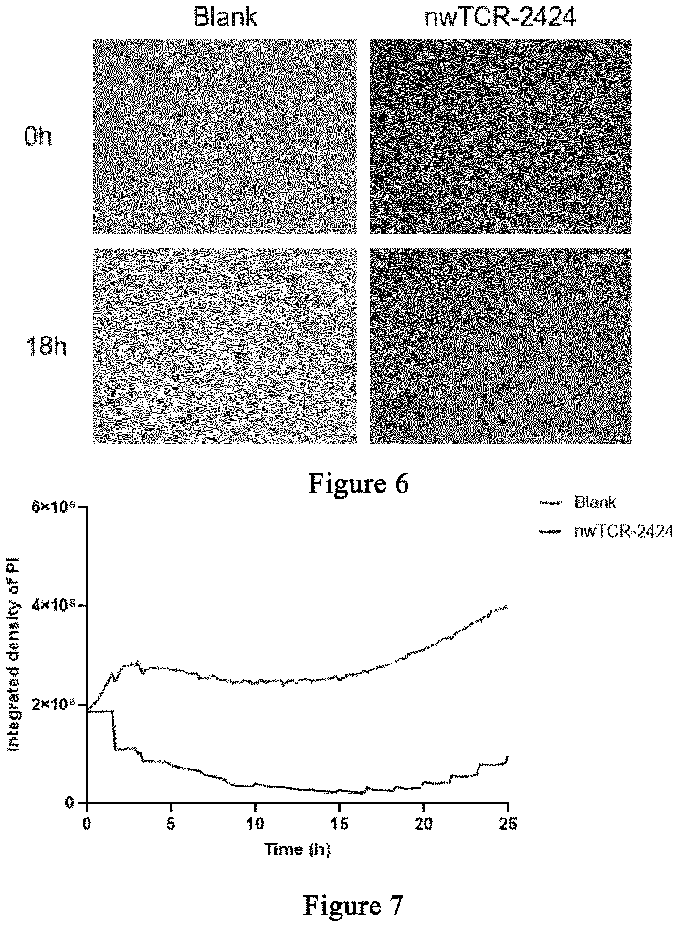

The results indicated that T cells expressing respective nwTCR enabled SW620 cells (overexpressing HLA-A*11:01, and KRAS G12V+) to be stained by ethidium bromide. Figure 6 exemplified the results of T cells expressing nwTCR-2424 at the beginning (0h) of co-incubation with SW620 cells, and at 18 hours of co-incubation (18h) with SW620 cells. The red fluorescent signal at 18 hours of co-incubation (18h) indicated the specific killing of SW620 cells by the T cells expressing nwTCR-2424.

Real-time data analysis of the cell killing indicated that the T cells expressing respective nwTCR had specific killing of SW620 cells (overexpressing HLA-A*11:01, and KRAS G12V+). Figure 7 exemplified the specific killing of SW620 cells by T cells expressing nwTCR-2424.

### Example 4. Co-expression of exogenous TCRs with CD8 molecules

This Example described the redirection of CD4+ T cells by co-expressing exogenous TCRs and CD8 molecules on CD4+ T cell membrane surface using CRISPR/Cas9 and homologous recombination technologies. Moreover, the gene editing method could enhance the binding of TCR-T cells to pMHC molecules.

### 4.1 Sorting and activation of T cells

T cells could be obtained commercially (e.g., frozen human peripheral blood CD4+ CD45RA+ T cells, Stem Cell Technology, Catalog No. 70029), or T cells were prepared from leukocyte apheresis samples (Day 0).

With respect to T cell preparation from leukocyte apheresis samples, CD4/CD8 T cells were enriched and sorted from the leukocyte apheresis samples. The enriched and sorted CD4/CD8 T cells were aliquoted and frozen (5x10⁶ cells/cryotube) for future use.

### 4.2 Targeting strategy and preparation of targeting vector

The targeting strategy and the targeting vector for TCR were the same as in Example 2.2 above. when the TCR sequences were from nwTCR-1708, the KI amino acid sequence of nwTCR-1708 was: 2A or 2A variant-SP-TCRβ-2A or 2A variant-SP-TRAV-TRAJ (SEQ ID NO: 392), and the KI nucleotide sequence of nwTCR-1708 was: 2A or 2A variant-SP-TCRβ-2A or 2A variant-SP-TRAV-TRAJ (SEQ ID NO: 393). As seen in Figure 3D above, CD8+ T cells expressing nwTCR-1708 could bind to the pMHC molecule having the amino acid sequence from position 7 to 16 of KRAS G12V (KRAS_G12V_7-16 peptide), but CD4+ T cells expressing nwTCR-1708 could not bind to the pMHC molecule presenting KRAS_G12V_7-16 peptide. In this Example, the selection of the CD4+ T cells expressing the TCR after gene editing but not binding to the pMHC tetramer was intended to show that, even for such an nwTCR, the binding of CD4 T cells after gene editing of the nwTCR to the pMHC molecule could be enhanced by introducing CD8 molecules into the CD4 T cells. Hence, the particular nwTCR used in this Example could be replaced with any of other nwTCRs in the present invention, and the enhanced binding of CD4 T cells after gene editing of the other nwTCRs to pMHC molecules could be achieved.

In order to enable CD4+ T cells to bind to pMHC molecules having the amino acid sequence from position 7 to 16 of KRAS G12V (KRAS_G12V_7-16 peptide), the following constructs were further constructed, and inserted into pUC57-HA targeting vector (also referred to as HDR vector):
The KI amino acid sequence of nwTCR-1708-CD8a was: 2A or 2A variant-CD8a-2A or 2A variant-SP-TCRβ-2A or 2A variant-SP-TRAV-TRAJ (SEQ ID NO: 394).

The KI nucleotide sequence of nwTCR-1708-CD8a was: 2A or 2A variant-CD8a-2A or 2A variant-SP-TCRβ-2A or 2A variant-SP-TRAV-TRAJ (SEQ ID NO: 395).

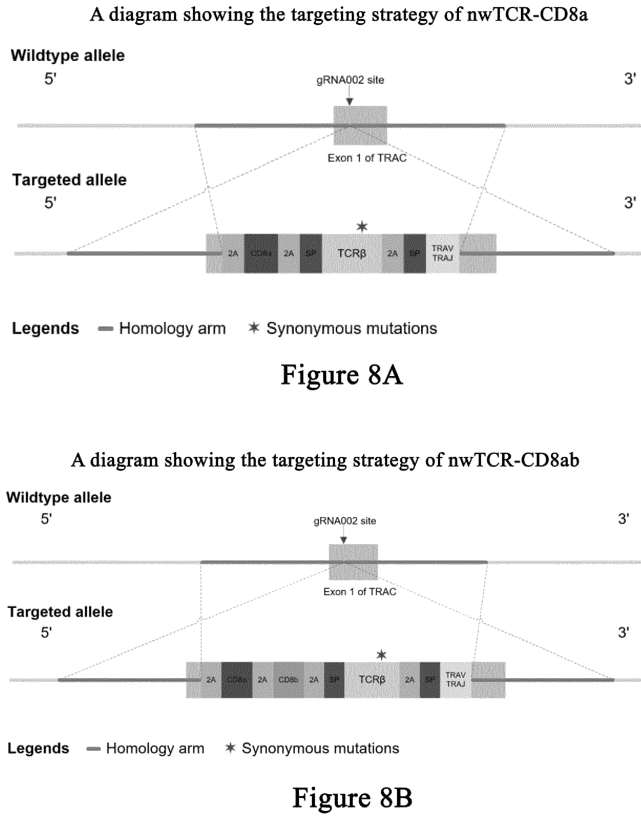

The targeting strategy diagram for nwTCR-CD8a was shown in Figure 8A.

The KI amino acid sequence of nwTCR-1708-CD8ab was: 2A or 2A variant-CD8a-2A or 2A variant-CD8b-2A or 2A variant-SP-TCRβ-2A or 2A variant-SP-TRAV-TRAJ (SEQ ID NO: 398).

The KI nucleotide sequence of nwTCR-1708-CD8ab was: 2A or 2A variant-CD8a-2A or 2A variant-CD8b-2A or 2A variant-SP-TCRβ-2A or 2A variant-SP-TRAV-TRAJ (SEQ ID NO: 399).

The targeting strategy diagram for nwTCR-CD8ab was shown in Figure 8B.

### 4.3 Transfection by electroporation (Day 2)

The sgRNAs designed and synthesized in Example 2.2 were mixed thoroughly with Cas9 enzyme and incubated for 10 min at room temperature to prepare RNP.

Three targeting vectors, i.e., targeting vectors for knocking in nwTCR-1708, nwTCR-1708-CD8a, or nwTCR-1708-CD8ab, prepared in Example 4.2, were respectively mixed thoroughly with the incubated RNP, and the indicated concentration of T cells (1.25E6/electroporation tube) prepared in Example 4.1, in order to knock-out (KO) endogenous TCR and knock-in (KI) exogenous TCR, exogenous TCR and CD8a, or exogenous TCR and CD8ab.

The above mixtures were loaded on an electroporation transfection apparatus (Celetrix; Catalog No. CTX-1500A LE) for cell electroporation transfection, which was performed at 480-560 V for 20 ms.

After the electroporation transfection, the electroporation-transfected cells were left to stand for 15 min, then taken out from the electroporation tube, and transferred into pre-warmed media (ImmunoCult^{™}-XF T Cell Expansion Medium, Stemcell Inc., Catalog No. 10981). After culturing the cells for 5 days, flow cytometry characterization was performed on Day 7.

### 4.4 Flow cytometry analysis of nwTCR expression (Day 7)

The cell suspension obtained from Example 4.3 was thoroughly mixed, cell counting was performed, and appropriate amounts of cells were collected for staining of the labeled peptide-MHC(HLA-A*11:01) tetramer (VVVGAVGVGK-HLA-A11:01).

A stain solution comprising the specific antigen in the labeled peptide-MHC(HLA-A*11:01) tetramer, and LIVE/DEAD^{™} Fixable Near-IR, purchased from Invitrogen, Catalog No. L10119; CD4-FITC, purchased from BioLegend, Catalog No. 357406; CD8-PerCP-cy5.5, purchased from BioLegend, Catalog No. 344710; anti-human TCR α/β-BV510 antibody, purchased from BioLegend, Catalog No. 306734, were prepared in advance.

Collected cells were stained with the labeled peptide-MHC(HLA-A*11:01) tetramer stain solution, washed, and characterized by flow cytometry. The results were shown below. In the flow cytogram, on Day7 cells were classified into three populations:
1) Wild-type T cells without gene editing (Q3);
2) KO cells with completed endogenous TCR knock-out (Q4);
3) Cell population with completed KO and KI, and expressing nwTCR (Q2).

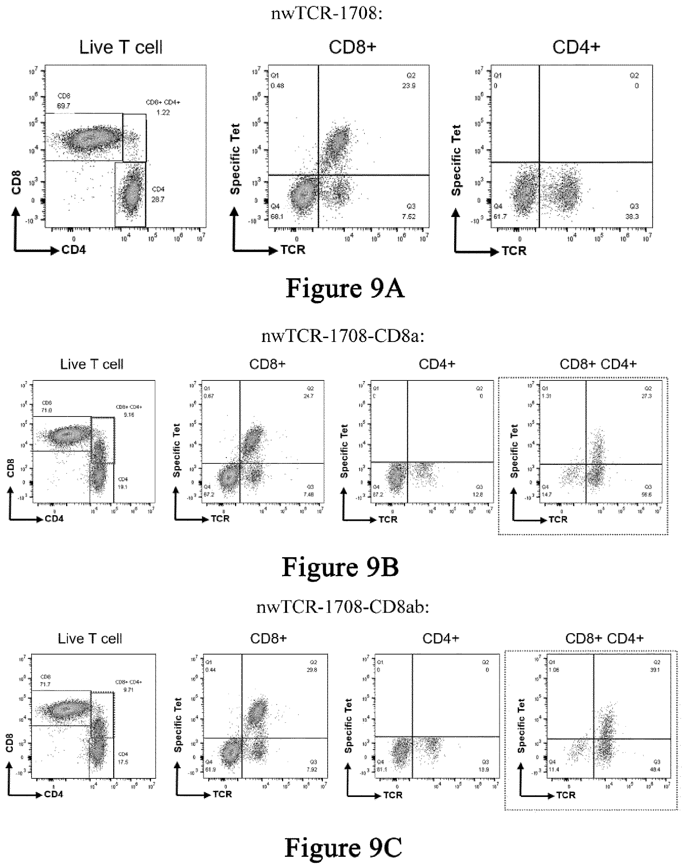

CD8+ T cells edited by nwTCR-1708 could bind the specific antigen MHC tetramer, whereas CD4+ T cells edited by nwTCR-1708 were unable to bind the specific antigen MHC tetramer due to the lack of help from CD8 molecules (Figure 9A).

When CD8a molecule or CD8ab molecule was co-edited with nwTCR-1708 into primary T cells (the primary T cells comprise CD4+ T cells and CD8+ T cells), it was seen as characterized by flow cytometry, that the proportion of CD8+ CD4+ T cells was increased, which cells were the CD4+ T cells expressing exogenous CD8 molecules, and under the help from exogenous CD8 molecules could specifically bind the labeled peptide-MHC(HLA-A11:01) tetramer (VVVGAVGVGK-HLA-A11:01) (Figure 9B and Figure 9C).

At the same time, thanks to the ability of the method to redirect CD4+ T cells, the overall gene editing efficiency for T cells was increased (Table 3), in which the gene editing efficiency (GE%) was calculated as follows: percentage of cells expressing only CD8+ in live T-cell population × percentage of cells expressing only CD8+ in the tetramer staining + percentage of cells expressing only CD4+ in live T-cell population × percentage of cells expressing only CD4+ in the tetramer staining + percentage of cells expressing CD8+ and CD4+ in live T-cell population × percentage of cells expressing CD8+ and CD4+ in the tetramer staining.

**Table 3. Gene editing efficiency of respective cell population in T cells co-expressing CD8 molecules.**

| Samples | Live T-cell population | | | Tetramer staining | | | Calculated gene editing efficiency (GE%) |
|---|---|---|---|---|---|---|---|
| | Express ing only CD8+ | Express ing only CD4+ | Expressing CD8+ and CD4+ | Express ing only CD8+ | Expres sing only CD4+ | Expressing CD8+, and CD4+ | |
| nwTCR-1708 | 69.70% | 28.70% | 1.22% | 23.90% | 0.00% | 0.00% | 16.70% |
| nwTCR-1708-CD8a | 71.00% | 19.10% | 9.16% | 24.70% | 0.00% | 27.30% | 20.00% |
| nwTCR-1708-CD8ab | 71.70% | 17.50% | 9.71% | 29.80% | 0.00% | 39.10% | 25.20% |

The above describes exemplary Examples of the present invention, and it should be understood by those skilled in the art that these disclosures are merely exemplary and that various other substitutions, adaptations, and modifications may be made within the scope of the present invention. Accordingly, the present invention is not limited to the specific embodiments exemplified herein.

### EXEMPLARY SEOUENCES

| SEQ ID NO: | Description | Amino acid sequence/nucleotide sequence |
|---|---|---|
| 1 | nwTCR-0125 α chain CDR1 | TRDTTYY |
| 2 | nwTCR-0125 α chain CDR2 | RNSFDEQN |
| 3 | nwTCR-0125 α chain CDR3 | ARDQTGANNLF |
| 4 | nwTCR-0126 α chain CDR1 | TRDTTYY |
| 5 | nwTCR-0126 α chain CDR2 | RNSFDEQN |
| 6 | nwTCR-0126 α chain CDR3 | ALSDPRYSGGGADGLT |
| 7 | nwTCR-0127 α chain CDR1 | NSMFDY |
| 8 | nwTCR-0127 α chain CDR2 | ISSIKDK |
| 9 | nwTCR-0127 α chain CDR3 | AGYQGGYKLS |
| 10 | nwTCR-1708 α chain CDR1 | DRGSQS |
| 11 | nwTCR-1708 α chain CDR2 | IYSNGD |
| 12 | nwTCR-1708 α chain CDR3 | AVNMPGNMLT |
| 13 | nwTCR-1862 α chain CDR1 | DSSSTY |
| 14 | nwTCR-1862 α chain CDR2 | IFSNMDM |
| 15 | nwTCR-1862 α chain CDR3 | AERGTDSWGKLQ |
| 16 | nwTCR-2162 α chain CDR1 | TSINN |
| 17 | nwTCR-2162 α chain CDR2 | IRSNERE |
| 18 | nwTCR-2162 α chain CDR3 | ATDDYGGATNKLI |
| 19 | nwTCR-2241 α chain CDR1 | TTSDR |
| 20 | nwTCR-2241 α chain CDR2 | LLSNGAV |
| 21 | nwTCR-2241 α chain CDR3 | AVDKAAGNKLT |
| 22 | nwTCR-2308 α chain CDR1 | VSNAYN |
| 23 | nwTCR-2308 α chain CDR2 | GSKP |
| 24 | nwTCR-2308 α chain CDR3 | AGSNDYKLS |
| 25 | nwTCR-2310 α chain CDR1 | TSINN |
| 26 | nwTCR-2310 α chain CDR2 | IRSNERE |
| 27 | nwTCR-2310 α chain CDR3 | ATDGGGGADGLT |
| 28 | nwTCR-2390 α chain CDR1 | TSINN |
| 29 | nwTCR-2390 α chain CDR2 | IRSNERE |
| 30 | nwTCR-2390 α chain CDR3 | ATDSGGGADGLT |
| 31 | nwTCR-2392 α chain CDR1 | NSMFDY |
| 32 | nwTCR-2392 α chain CDR2 | ISSIKDK |
| 33 | nwTCR-2392 α chain CDR3 | AASYAGSARQLT |
| 34 | nwTCR-2424 α chain CDR1 | DRGSQS |
| 35 | nwTCR-2424 α chain CDR2 | IYSNGD |
| 36 | nwTCR-2424 α chain CDR3 | ARKKTSYDKVI |
| 37 | nwTCR-2561 α chain CDR1 | ATGYPS |
| 38 | nwTCR-2561 α chain CDR2 | ATKADDK |
| 39 | nwTCR-2561 α chain CDR3 | ALPAGGGADGLT |
| 40 | nwTCR-2563 α chain CDR1 | TSINN |
| 41 | nwTCR-2563 α chain CDR2 | IRSNERE |
| 42 | nwTCR-2563 α chain CDR3 | ATDRTGGYNKLI |
| 43 | nwTCR-2595 α chain CDR1 | SSNFYA |
| 44 | nwTCR-2595 α chain CDR2 | MTLNGDE |
| 45 | nwTCR-2595 α chain CDR3 | AFNNQGGKLI |
| 46 | nwTCR-2629 α chain CDR1 | SSYSPS |
| 47 | nwTCR-2629 α chain CDR2 | YTSAATLV |
| 48 | nwTCR-2629 α chain CDR3 | VVSVWTDKLI |
| 49 | nwTCR-0125 amino acid sequence encoded by TRAV gene | AQKVTQAQTEISVVEKEDVTLDCVYETRDTTYYLFWYKQPPSGELVFLIRRNSFDEQNEI SGRYSWNFQKSTSSFNFTITASQVVDSAVYFC |
| 50 | nwTCR-0125 nucleotide sequence of TRAV gene | GCTCAGAAGGTAACTCAAGCGCAGACTGAAATTTCTGTGGTGGAGAAGGAGGATGT GACCTTGGACTGTGTGTATGAAACCCGTGATACTACTTATTACTTATTCTGGTACAA GCAACCACCAAGTGGAGAATTGGTTTTCCTTATTCGTCGGAACTCTTTTGATGAGCA AAATGAAATAAGTGGTCGGTATTCTTGGAACTTCCAGAAATCCACCAGTTCCTTCAA CTTCACCATCACAGCCTCACAAGTCGTGGACTCAGCAGTATACTTCTGT |
| 51 | nwTCR-0125 amino acid sequence encoded by TRAJ gene | QTGANNLFFGTGTRLTVIP |
| 52 | nwTCR-0125 nucleotide sequence of TRAJ gene | CAAACTGGGGCAAACAACCTCTTCTITGGGACTGGAACGAGACTCACCGTTATTCCC |
| 53 | nwTCR-0126 amino acid sequence encoded by TRAV gene | AQKVTQAQTEISVVEKEDVTLDCVYETRDTTYYLFWYKQPPSGELVFLIRRNSFDEQNEI SGRYSWNFQKSTSSFNFTITASQVVDSAVYFCALS |
| 54 | nwTCR-0126 nucleotide sequence of TRAV gene | GCTCAGAAGGTAACTCAAGCGCAGACTGAAATTTCTGTGGTGGAGAAGGAGGATGT GACCTTGGACTGTGTGTATGAAACCCGTGATACTACTTATTACTTATTCTGGTACAA GCAACCACCAAGTGGAGAATTGGTTTTCCTTATTCGTCGGAACTCTTTTGATGAGCA AAATGAAATAAGTGGTCGGTATTCTTGGAACTTCCAGAAATCCACCAGTTCCTTCAA |
| | | CTTCACCATCACAGCCTCACAAGTCGTGGACTCAGCAGTATACTTCTGTGCTCTGAG T |
| 55 | nwTCR-0126 amino acid sequence encoded by TRAJ gene | YSGGGADGLTFGKGTHLIIQP |
| 56 | nwTCR-0126 nucleotide sequence of TRAJ gene | TATTCAGGAGGAGGTGCTGACGGACTCACCTTTGGCAAAGGGACTCATCTAATCATC CAGCCC |
| 57 | nwTCR-0127 amino acid sequence encoded by TRAV gene | DQQVKQNSPSLSVQEGRISILNCDYTNSMFDYFLWYKKYPAEGPTFLISISSIKDKNEDG RFTVFLNKSAKHLSLHIVPSQPGDSAVYFCA |
| 58 | nwTCR-0127 nucleotide sequence of TRAV gene | GACCAGCAAGTTAAGCAAAATTCACCATCCCTGAGCGTCCAGGAAGGAAGAATTTC TATTCTGAACTGTGACTATACTAACAGCATGTTTGATTATTTCCTATGGTACAAAAA ATACCCTGCTGAAGGTCCTACATTCCTGATATCTATAAGTTCCATTAAGGATAAAAA TGAAGATGGAAGATTCACTGTCTTCTTAAACAAAAGTGCCAAGCACCTCTCTCTGCA CATTGTGCCCTCCCAGCCTGGAGACTCTGCAGTGTACTTCTGTGCA |
| 59 | nwTCR-0127 amino acid sequence encoded by TRAJ gene | YKLSFGAGTTVTVRA |
| 60 | nwTCR-0127 nucleotide sequence of TRAJ gene | TACAAGCTCAGCTTTGGAGCCGGAACCACAGTAACTGTAAGAGCA |
| 61 | nwTCR-1708 amino acid sequence encoded by TRAV gene | QKEVEQNSGPLSVPEGAIASLNCTYSDRGSQSFFWYRQYSGKSPELIMFIYSNGDKEDGR FTAQLNKASQYVSLLIRDSQPSDSATYLCAVN |
| 62 | nwTCR-1708 nucleotide sequence of TRAV gene | CAGAAGGAGGTGGAGCAGAATTCTGGACCCCTCAGTGTTCCAGAGGGAGCCATTGC CTCTCTCAACTGCACTTACAGTGACCGAGGTTCCCAGTCCTTCTTCTGGTACAGACA ATATTCTGGGAAAAGCCCTGAGTTGATAATGTTCATATACTCCAATGGTGACAAAGA AGATGGAAGGTTTACAGCACAGCTCAATAAAGCCAGCCAGTATGTTTCTCTGCTCAT CAGAGACTCCCAGCCCAGTGATTCAGCCACCTACCTCTGTGCCGTGAAC |
| 63 | nwTCR-1708 amino acid sequence encoded by TRAJ gene | GNMLTFGGGTRLMVKP |
| 64 | nwTCR-1708 nucleotide sequence of TRAJ gene | GGCAACATGCTCACCTTTGGAGGGGGAACAAGGTTAATGGTCAAACCC |
| 65 | nwTCR-1862 amino acid sequence encoded by TRAV gene | GEDVEQSLFLSVREGDSSVINCTYTDSSSTYLYWYKQEPGAGLQLLTYIFSNMDMKQDQ RLTVLLNKKDKHLSLRIADTQTGDSAIYFCAE |
| 66 | nwTCR-1862 nucleotide sequence of TRAV gene | GGAGAGGATGTGGAGCAGAGTCTTTTCCTGAGTGTCCGAGAGGGAGACAGCTCCGT TATAAACTGCACTTACACAGACAGCTCCTCCACCTACTTATACTGGTATAAGCAAGA ACCTGGAGCAGGTCTCCAGTTGCTGACGTATATTTTTTCAAATATGGACATGAAACA AGACCAAAGACTCACTGTTCTATTGAATAAAAAGGATAAACATCTGTCTCTGCGCAT TGCAGACACCCAGACTGGGGACTCAGCTATCTACTTCTGTGCAGAG |
| 67 | nwTCR-1862 amino acid sequence encoded by TRAJ gene | TDSWGKLQFGAGTQVVVTP |
| 68 | nwTCR-1862 nucleotide sequence of TRAJ gene | ACTGACAGCTGGGGGAAATTGCAGTTTGGAGCAGGGACCCAGGTTGTGGTCACCCC A |
| 69 | nwTCR-2162 amino acid sequence encoded by TRAV gene | SQQGEEDPQALSIQEGENATMNCSYKTSINNLQWYRQNSGRGLVHLILIRSNEREKHSG RLRVTLDTSKKSSSLLITASRAADTASYFCATD |
| 70 | nwTCR-2162 nucleotide sequence of TRAV gene | AGTCAACAGGGAGAAGAGGATCCTCAGGCCTTGAGCATCCAGGAGGGTGAAAATGC |
| | | CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGAC |
| 71 | nwTCR-2162 amino acid sequence encoded by TRAJ gene | YGGATNKLIFGTGTLLAVQP |
| 72 | nwTCR-2162 nucleotide sequence of TRAJ gene | TATGGTGGTGCTACAAACAAGCTCATCTTTGGAACTGGCACTCTGCTTGCTGTCCAG CCA |
| 73 | nwTCR-2241 amino acid sequence encoded by TRAV gene | ELKVEQNPLFLSMQEGKNYTIYCNYSTTSDRLYWYRQDPGKSLESLFVLLSNGAVKQE GRLMASLDTKARLSTLHITAAVHDLSATYFCAVD |
| 74 | nwTCR-2241 nucleotide sequence of TRAV gene | GAGCTGAAAGTGGAACAAAACCCTCTGTTCCTGAGCATGCAGGAGGGAAAAAACTA TACCATCTACTGCAATTATTCAACCACTTCAGACAGACTGTATTGGTACAGGCAGGA TCCTGGGAAAAGTCTGGAATCTCTGTTTGTGTTGCTATCAAATGGAGCAGTGAAGCA GGAGGGACGATTAATGGCCTCACTTGATACCAAAGCCCGTCTCAGCACCCTCCACAT CACAGCTGCCGTGCATGACCTCTCTGCCACCTACTTCTGTGCCGTGGAC |
| 75 | nwTCR-2241 amino acid sequence encoded by TRAJ gene | AAGNKLTFGGGTRVLVKP |
| 76 | nwTCR-2241 nucleotide sequence of TRAJ gene | GCTGCAGGCAACAAGCTE1ACTTTTGGAGGAGGAACCAGGGTGCTAGTTAAACCA |
| 77 | nwTCR-2308 amino acid sequence encoded by TRAV gene | KDQVFQPSTVASSEGAVVEIFCNHSVSNAYNFFWYLHFPGCAPRLLVKGSKPSQQGRYN MTYERFSSSLLILQVREADAAVYYCA |
| 78 | nwTCR-2308 nucleotide sequence of TRAV gene | AAGGACCAAGTGTTTCAGCCTTCCACAGTGGCATCTTCAGAGGGAGCTGTGGTGGA AATCTTCTGTAATCACTCTGTGTCCAATGCTTACAACTTCTTCTGGTACCTTCACTTC CCGGGATGTGCACCAAGACTCCTTGTTAAAGGCTCAAAGCCTTCTCAGCAGGGACG ATACAACATGACCTATGAACGGTTCTCTTCATCGCTGCTCATCCTCCAGGTGCGGGA GGCAGATGCTGCTGTTTACTACTGTGCT |
| 79 | nwTCR-2308 amino acid sequence encoded by TRAJ gene | SNDYKLSFGAGTTVTVRA |
| 80 | nwTCR-2308 nucleotide sequence of TRAJ gene | TCTAACGACTACAAGCTCAGCTTTGGAGCCGGAACCACAGTAACTGTAAGAGCA |
| 81 | nwTCR-2310 amino acid sequence encoded by TRAV gene | SQQGEEDPQALSIQEGENATMNCSYKTSINNLQWYRQNSGRGLVHLILIRSNEREKHSG RLRVTLDTSKKSSSLLITASRAADTASYFCATD |
| 82 | nwTCR-2310 nucleotide sequence of TRAV gene | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGAC |
| 83 | nwTCR-2310 amino acid sequence encoded by TRAJ gene | GGGADGLTFGKGTHLIIQP |
| 84 | nwTCR-2310 nucleotide sequence of TRAJ gene | GGAGGAGGTGCTGACGGACTCACCTTTGGCAAAGGGACTCATCTAATCATCCAGCC C |
| 85 | nwTCR-2390 amino acid sequence encoded by TRAV gene | SQQGEEDPQALSIQEGENATMNCSYKTSINNLQWYRQNSGRGLVHLILIRSNEREKHSG RLRVTLDTSKKSSSLLITASRAADTASYFCAT |
| 86 | nwTCR-2390 nucleotide sequence of TRAV gene | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACG |
| 87 | nwTCR-2390 amino acid sequence encoded by TRAJ gene | SGGGADGLTFGKGTHLIIQP |
| 88 | nwTCR-2390 nucleotide sequence of TRAJ gene | TCAGGAGGAGGTGCTGACGGACTCACCTTTGGCAAAGGGACTCATCTAATCATCCA GCCC |
| 89 | nwTCR-2392 amino acid sequence encoded by TRAV gene | DQQVKQNSPSLSVQEGRISILNCDYTNSMFDYFLWYKKYPAEGPTFLISISSIKDKNEDG RFTVFLNKSAKHLSLHIVPSQPGDSAVYFCAAS |
| 90 | nwTCR-2392 nucleotide sequence of TRAV gene | GACCAGCAAGTTAAGCAAAATTCACCATCCCTGAGCGTCCAGGAAGGAAGAATTTC TATTCTGAACTGTGACTATACTAACAGCATGTTTGATTATTTCCTATGGTACAAAAA ATACCCTGCTGAAGGTCCTACATTCCTGATATCTATAAGTTCCATTAAGGATAAAAA TGAAGATGGAAGATTCACTGTCTTCTTAAACAAAAGTGCCAAGCACCTCTCTCTGCA CATTGTGCCCTCCCAGCCTGGAGACTCTGCAGTGTACTTCTGTGCAGCAAGC |
| 91 | nwTCR-2392 amino acid sequence encoded by TRAJ gene | GSARQLTFGSGTQLTVLP |
| 92 | nwTCR-2392 nucleotide sequence of TRAJ gene | GGTTCTGCAAGGCAACTGACCTTTGGATCTGGGACACAATTGACTGTTTTACCT |
| 93 | nwTCR-2424 amino acid sequence encoded by TRAV gene | QKEVEQNSGPLSVPEGAIASLNCTYSDRGSQSFFWYRQYSGKSPELIMFIYSNGDKEDGR FTAQLNKASQYVSLLIRDSQPSDSATYLCA |
| 94 | nwTCR-2424 nucleotide sequence of TRAV gene | CAGAAGGAGGTGGAGCAGAATTCTGGACCCCTCAGTGTTCCAGAGGGAGCCATTGC CTCTCTCAACTGCACTTACAGTGACCGAGGTTCCCAGTCCTTCTTCTGGTACAGACA ATATTCTGGGAAAAGCCCTGAGTTGATAATGTTCATATACTCCAATGGTGACAAAGA AGATGGAAGGTTTACAGCACAGCTCAATAAAGCCAGCCAGTATGTTTCTCTGCTCAT CAGAGACTCCCAGCCCAGTGATTCAGCCACCTACCTCTGTGCC |
| 95 | nwTCR-2424 amino acid sequence encoded by TRAJ gene | KTSYDKVIFGPGTSLSVIP |
| 96 | nwTCR-2424 nucleotide sequence of TRAJ gene | AAAACCTCCTACGACAAGGTGATATTTGGGCCAGGGACAAGCTTATCAGTCATTCCA |
| 97 | nwTCR-2561 amino acid sequence encoded by TRAV gene | GNSVTQMEGPVTLSEEAFLTINCTYTATGYPSLFWYVQYPGEGLQLLLKATKADDKGS NKGFEATYRKETTSFHLEKGSVQVSDSAVYFCAL |
| 98 | nwTCR-2561 nucleotide sequence of TRAV gene | GGAAATTCAGTGACCCAGATGGAAGGGCCAGTGACTCTCTCAGAAGAGGCCTTCCT GACTATAAACTGCACGTACACAGCCACAGGATACCCTTCCCTTTTCTGGTATGTCCA ATATCCTGGAGAAGGTCTACAGCTCCTCCTGAAAGCCACGAAGGCTGATGACAAGG GAAGCAACAAAGGTTTTGAAGCCACATACCGTAAAGAAACCACTTCTTTCCACTTGG AGAAAGGCTCAGTTCAAGTGTCAGACTCAGCGGTGTACTTCTGTGCTCTG |
| 99 | nwTCR-2561 amino acid sequence encoded by TRAJ gene | GGGADGLTFGKGTHLIIQP |
| 100 | nwTCR-2561 nucleotide sequence of TRAJ gene | GGAGGAGGTGCTGACGGACTCACCTTTGGCAAAGGGACTCATCTAATCATCCAGCC C |
| 101 | nwTCR-2563 amino acid sequence encoded by TRAV gene | SQQGEEDPQALSIQEGENATMNCSYKTSINNLQWYRQNSGRGLVHLILIRSNEREKHSG |
| | | RLRVTLDTSKKSSSLLITASRAADTASYFCATD |
| 102 | nwTCR-2563 nucleotide sequence of TRAV gene | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGAC |
| 103 | nwTCR-2563 amino acid sequence encoded by TRAJ gene | GGYNKLIFGAGTRLA VHP |
| 104 | nwTCR-2563 nucleotide sequence of TRAJ gene | GGTGGCTACAATAAGCTGATTTTTGGAGCAGGGACCAGGCTGGCTGTACACCCA |
| 105 | nwTCR-2595 amino acid sequence encoded by TRAV gene | ILNVEQSPQSLHVQEGDSTNFTCSFPSSNFYALHWYRWETAKSPEALFVMTLNGDEKKK GRISATLNTKEGYSYLYIKGSQPEDSATYLCAF |
| 106 | nwTCR-2595 nucleotide sequence of TRAV gene | ATACTGAACGTGGAACAAAGTCCTCAGTCACTGCATGTTCAGGAGGGAGACAGCAC CAATTTCACCTGCAGCTTCCCTTCCAGCAATTTTTATGCCTTACACTGGTACAGATGG GAAACTGCAAAAAGCCCCGAGGCCTTGTTTGTAATGACTTTAAATGGGGATGAAAA GAAGAAAGGACGAATAAGTGCCACTCTTAATACCAAGGAGGGTTACAGCTATTTGT ACATCAAAGGATCCCAGCCTGAAGACTCAGCCACATACCTCTGTGCCTTT |
| 107 | nwTCR-2595 amino acid sequence encoded by TRAJ gene | NQGGKLIFGQGTELSVKP |
| 108 | nwTCR-2595 nucleotide sequence of TRAJ gene | AACCAGGGAGGAAAGCTTATCTTCGGACAGGGAACGGAGTTATCTGTGAAACCC |
| 109 | nwTCR-2629 amino acid sequence encoded by TRAV gene | AQSVTQLDSHVSVSEGTPVLLRCNYSSSYSPSLFWYVQHPNKGLQLLLKYTSAATLVKG INGFEAEFKKSETSFHLTKPSAHMSDAAEYFCVVS |
| 110 | nwTCR-2629 nucleotide sequence of TRAV gene | GCCCAGTCGGTGACCCAGCTTGACAGCCACGTCTCTGTCTCTGAAGGAACCCCGGTG CTGCTGAGGTGCAACTACTCATCTTCTTATTCACCATCTCTCTTCTGGTATGTGCAAC ACCCCAACAAAGGACTCCAGCTTCTCCTGAAGTACACATCAGCGGCCACCCTGGTTA AAGGCATCAACGGTTTTGAGGCTGAATTTAAGAAGAGTGAAACCTCCTTCCACCTGA CGAAACCCTCAGCCCATATGAGCGACGCGGCTGAGTACTTCTGTGTTGTGAGT |
| 111 | nwTCR-2629 amino acid sequence encoded by TRAJ gene | TDKLIFGTGTRLQVFP |
| 112 | nwTCR-2629 nucleotide sequence of TRAJ gene | ACCGACAAGCTCATCTTTGGGACTGGGACCAGATTACAAGTCTTTCCA |
| 113 | nwTCR-0125 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 114 | nwTCR-0125 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 115 | nwTCR-0126 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 116 | nwTCR-0126 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 117 | nwTCR-0127 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 118 | nwTCR-0127 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 119 | nwTCR-1708 amino acid sequence encoded by TRAC gene | HIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 120 | nwTCR-1708 nucleotide sequence of TRAC gene | CATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 121 | nwTCR-1862 amino acid sequence encoded by TRAC gene | DIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 122 | nwTCR-1862 nucleotide sequence of TRAC gene | GATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 123 | nwTCR-2162 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 124 | nwTCR-2162 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 125 | nwTCR-2241 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 126 | nwTCR-2241 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA |
| | | ACCTGTCAGTGATTGGGTTCCGAATCCTCCTCCTGAAAGTGGCCGGGTTTAATCTGC TCATGACGCTGCGGCTGTGGTCCAGC |
| 127 | nwTCR-2308 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 128 | nwTCR-2308 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 129 | nwTCR-2310 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 130 | nwTCR-2310 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 131 | nwTCR-2390 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 132 | nwTCR-2390 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 133 | nwTCR-2392 amino acid sequence encoded by TRAC gene | DIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 134 | nwTCR-2392 nucleotide sequence of TRAC gene | GATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 135 | nwTCR-2424 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 136 | nwTCR-2424 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 137 | nwTCR-2561 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 138 | nwTCR-2561 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 139 | nwTCR-2563 amino acid sequence encoded by TRAC gene | YIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 140 | nwTCR-2563 nucleotide sequence of TRAC gene | TATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 141 | nwTCR-2595 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 142 | nwTCR-2595 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 143 | nwTCR-2629 amino acid sequence encoded by TRAC gene | NIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGF RILLLKVAGFNLLMTLRLWSS |
| 144 | nwTCR-2629 nucleotide sequence of TRAC gene | AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 145 | nwTCR-0125 complete TRA (TCR α chain) amino acid sequence | AQKVTQAQTEISVVEKEDVTLDCVYETRDTTYYLFWYKQPPSGELVFLIRRNSFDEQNEI SGRYSWNFQKSTSSFNFTITASQVVDSAVYFCARDQTGANNLFFGTGTRLTVIPYIQNPD PAVYQLRDSKSSDKSVCLFTDFDSQTNVSQSKDSDVYITDKTVLDMRSMDFKSNSAVA WSNKSDFACANAFNNSIIPEDTFFPSPESSCDVKLVEKSFETDTNLNFQNLSVIGFRILLLK VAGFNLLMTLRLWSS |
| 146 | nwTCR-0125 complete TRA (TCR α chain) nucleotide sequence | GCTCAGAAGGTAACTCAAGCGCAGACTGAAATTTCTGTGGTGGAGAAGGAGGATGT GACCTTGGACTGTGTGTATGAAACCCGTGATACTACTTATTACTTATTCTGGTACAA GCAACCACCAAGTGGAGAATTGGTTTTCCTTATTCGTCGGAACTCTTTTGATGAGCA AAATGAAATAAGTGGTCGGTATTCTTGGAACTTCCAGAAATCCACCAGTTCCTTCAA CTTCACCATCACAGCCTCACAAGTCGTGGACTCAGCAGTATACTTCTGTGCCCGTGA TCAAACTGGGGCAAACAACCTCTTCTTTGGGACTGGAACGAGACTCACCGTTATTCC CTATATCCAGAACCCTGACCCTGCCGTGTACCAGCTGAGAGACTCTAAATCCAGTGA CAAGTCTGTCTGCCTATTCACCGATTTTGATTCTCAAACAAATGTGTCACAAAGTAA GGATTCTGATGTGTATATCACAGACAAAACTGTGCTAGACATGAGGTCTATGGACTT CAAGAGCAACAGTGCTGTGGCCTGGAGCAACAAATCTGACTTTGCATGTGCAAACG CCTTCAACAACAGCATTATTCCAGAAGACACCTTCTTCCCCAGCCCAGAAAGTTCCT GTGATGTCAAGCTGGTCGAGAAAAGCTTTGAAACAGATACGAACCTAAACTTTCAA AACCTGTCAGTGATTGGGTTCCGAATCCTCCTCCTGAAAGTGGCCGGGTTTAATCTG CTCATGACGCTGCGGCTGTGGTCCAGC |
| 147 | nwTCR-0126 complete TRA (TCR α chain) amino acid sequence | AQKVTQAQTEISVVEKEDVTLDCVYETRDTTYYLFWYKQPPSGELVFLIRRNSFDEQNEI SGRYSWNFQKSTSSFNFTITASQVVDSAVYFCALSDPRYSGGGADGLTFGKGTHLIIQPY IQNPDPAVYQLRDSKSSDKSVCLFTDFDSQTNVSQSKDSDVYITDKTVLDMRSMDFKSN |
| | | SAVAWSNKSDFACANAFNNSIIPEDTFFPSPESSCDVKLVEKSFETDTNLNFQNLSVIGFR ILLLKVAGFNLLMTLRLWSS |
| 148 | nwTCR-0126 complete TRA (TCR α chain) nucleotide sequence | GCTCAGAAGGTAACTCAAGCGCAGACTGAAATTTCTGTGGTGGAGAAGGAGGATGT GACCTTGGACTGTGTGTATGAAACCCGTGATACTACTTATTACTTATTCTGGTACAA GCAACCACCAAGTGGAGAATTGGTTTTCCTTATTCGTCGGAACTCTTTTGATGAGCA AAATGAAATAAGTGGTCGGTATTCTTGGAACTTCCAGAAATCCACCAGTTCCTTCAA CTTCACCATCACAGCCTCACAAGTCGTGGACTCAGCAGTATACTTCTGTGCTCTGAG TGACCCCCGTTATTCAGGAGGAGGTGCTGACGGACTCACCTTTGGCAAAGGGACTC ATCTAATCATCCAGCCCTATATCCAGAACCCTGACCCTGCCGTGTACCAGCTGAGAG ACTCTAAATCCAGTGACAAGTCTGTCTGCCTATTCACCGATTTTGATTCTCAAACAA ATGTGTCACAAAGTAAGGATTCTGATGTGTATATCACAGACAAAACTGTGCTAGAC ATGAGGTCTATGGACTTCAAGAGCAACAGTGCTGTGGCCTGGAGCAACAAATCTGA CTTTGCATGTGCAAACGCCTTCAACAACAGCATTATTCCAGAAGACACCTTCTTCCC CAGCCCAGAAAGTTCCTGTGATGTCAAGCTGGTCGAGAAAAGCTTTGAAACAGATA CGAACCTAAACTTTCAAAACCTGTCAGTGATTGGGTTCCGAATCCTCCTCCTGAAAG TGGCCGGGTTTAATCTGCTCATGACGCTGCGGCTGTGGTCCAGC |
| 149 | nwTCR-0127 complete TRA (TCR α chain) amino acid sequence | DQQVKQNSPSLSVQEGRISILNCDYTNSMFDYFLWYKKYPAEGPTFLISISSIKDKNEDG RFTVFLNKSAKHLSLHIVPSQPGDSAVYFCAGYQGGYKLSFGAGTTVTVRANIQNPDPA VYQLRDSKSSDKSVCLFTDFDSQTNVSQSKDSDVYITDKTVLDMRSMDFKSNSAVAWS NKSDFACANAFNNSIIPEDTFFPSPESSCDVKLVEKSFETDTNLNFQNLSVIGFRILLLKVA GFNLLMTLRLWSS |
| 150 | nwTCR-0127 complete TRA (TCR α chain) nucleotide sequence | GACCAGCAAGTTAAGCAAAATTCACCATCCCTGAGCGTCCAGGAAGGAAGAATTTC TATTCTGAACTGTGACTATACTAACAGCATGTTTGATTATTTCCTATGGTACAAAAA ATACCCTGCTGAAGGTCCTACATTCCTGATATCTATAAGTTCCATTAAGGATAAAAA TGAAGATGGAAGATTCACTGTCTTCTTAAACAAAAGTGCCAAGCACCTCTCTCTGCA CATTGTGCCCTCCCAGCCTGGAGACTCTGCAGTGTACTTCTGTGCAGGGTATCAAGG GGGCTACAAGCTCAGCTTTGGAGCCGGAACCACAGTAACTGTAAGAGCAAATATCC AGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCT GATGTGTATATCACAGACAAAACTGTGCTAGACATGAGGTCTATGGACTTCAAGAG CAACAGTGCTGTGGCCTGGAGCAACAAATCTGACTTTGCATGTGCAAACGCCTTCAA CAACAGCATTATTCCAGAAGACACCTTCTTCCCCAGCCCAGAAAGTTCCTGTGATGT CAAGCTGGTCGAGAAAAGCTTTGAAACAGATACGAACCTAAACTTTCAAAACCTGT CAGTGATTGGGTTCCGAATCCTCCTCCTGAAAGTGGCCGGGTTTAATCTGCTCATGA CGCTGCGGCTGTGGTCCAGC |
| 151 | nwTCR-1708 complete TRA (TCR α chain) amino acid sequence | QKEVEQNSGPLSVPEGAIASLNCTYSDRGSQSFFWYRQYSGKSPELIMFIYSNGDKEDGR FTAQLNKASQYVSLLIRDSQPSDSATYLCAVNMPGNMLTFGGGTRLMVKPHIQNPDPA VYQLRDSKSSDKSVCLFTDFDSQTNVSQSKDSDVYITDKTVLDMRSMDFKSNSAVAWS NKSDFACANAFNNSIIPEDTFFPSPESSCDVKLVEKSFETDTNLNFQNLSVIGFRILLLKVA GFNLLMTLRLWSS |
| 152 | nwTCR-1708 complete TRA (TCR α chain) nucleotide sequence | CAGAAGGAGGTGGAGCAGAATTCTGGACCCCTCAGTGTTCCAGAGGGAGCCATTGC CTCTCTCAACTGCACTTACAGTGACCGAGGTTCCCAGTCCTTCTTCTGGTACAGACA ATATTCTGGGAAAAGCCCTGAGTTGATAATGTTCATATACTCCAATGGTGACAAAGA AGATGGAAGGTTTACAGCACAGCTCAATAAAGCCAGCCAGTATGTTTCTCTGCTCAT CAGAGACTCCCAGCCCAGTGATTCAGCCACCTACCTCTGTGCCGTGAACATGCCAGG CAACATGCTCACCTTTGGAGGGGGAACAAGGTTAATGGTCAAACCCCATATCCAGA ACCCTGACCCTGCCGTGTACCAGCTGAGAGACTCTAAATCCAGTGACAAGTCTGTCT GCCTATTCACCGATTTTGATTCTCAAACAAATGTGTCACAAAGTAAGGATTCTGATG TGTATATCACAGACAAAACTGTGCTAGACATGAGGTCTATGGACTTCAAGAGCAAC AGTGCTGTGGCCTGGAGCAACAAATCTGACTTTGCATGTGCAAACGCCTTCAACAAC AGCATTATTCCAGAAGACACCTTCTTCCCCAGCCCAGAAAGTTCCTGTGATGTCAAG CTGGTCGAGAAAAGCTTTGAAACAGATACGAACCTAAACTTTCAAAACCTGTCAGT GATTGGGTTCCGAATCCTCCTCCTGAAAGTGGCCGGGTTTAATCTGCTCATGACGCT GCGGCTGTGGTCCAGC |
| 153 | nwTCR-1862 complete TRA (TCR α chain) amino acid sequence | GEDVEQSLFLSVREGDSSVINCTYTDSSSTYLYWYKQEPGAGLQLLTYIFSNMDMKQDQ RLTVLLNKKDKHLSLRIADTQTGDSAIYFCAERGTDSWGKLQFGAGTQVVVTPDIQNPD PAVYQLRDSKSSDKSVCLFTDFDSQTNVSQSKDSDVYITDKTVLDMRSMDFKSNSAVA WSNKSDFACANAFNNSIIPEDTFFPSPESSCDVKLVEKSFETDTNLNFQNLSVIGFRILLLK VAGFNLLMTLRLWSS |
| 154 | nwTCR-1862 complete TRA (TCR α chain) nucleotide sequence | GGAGAGGATGTGGAGCAGAGTCTTTTCCTGAGTGTCCGAGAGGGAGACAGCTCCGT TATAAACTGCACTTACACAGACAGCTCCTCCACCTACTTATACTGGTATAAGCAAGA ACCTGGAGCAGGTCTCCAGTTGCTGACGTATATTTTTTCAAATATGGACATGAAACA AGACCAAAGACTCACTGTTCTATTGAATAAAAAGGATAAACATCTGTCTCTGCGCAT TGCAGACACCCAGACTGGGGACTCAGCTATCTACTTCTGTGCAGAGAGAGGGACTG ACAGCTGGGGGAAATTGCAGTTTGGAGCAGGGACCCAGGTTGTGGTCACCCCAGAT ATCCAGAACCCTGACCCTGCCGTGTACCAGCTGAGAGACTCTAAATCCAGTGACAA |
| | | GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA GAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGA TGTCAAGCTGGTCGAGAAAAGCTTTGAAACAGATACGAACCTAAACTTTCAAAACC TGTCAGTGATTGGGTTCCGAATCCTCCTCCTGAAAGTGGCCGGGTTTAATCTGCTCA TGACGCTGCGGCTGTGGTCCAGC |
| 155 | nwTCR-2162 complete TRA (TCR α chain) amino acid sequence | SQQGEEDPQALSIQEGENATMNCSYKTSINNLQWYRQNSGRGLVHLILIRSNEREKHSG RLRVTLDTSKKSSSLLITASRAADTASYFCATDDYGGATNKLIFGTGTLLAVQPNIQNPD PAVYQLRDSKSSDKSVCLFTDFDSQTNVSQSKDSDVYITDKTVLDMRSMDFKSNSAVA WSNKSDFACANAFNNSIIPEDTFFPSPESSCDVKLVEKSFETDTNLNFQNLSVIGFRILLLK VAGFNLLMTLRLWSS |
| 156 | nwTCR-2162 complete TRA (TCR α chain) nucleotide sequence | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGACGATTAT GGTGGTGCTACAAACAAGCTCATCTTTGGAACTGGCACTCTGCTTGCTGTCCAGCCA AATATCCAGAACCCTGACCCTGCCGTGTACCAGCTGAGAGACTCTAAATCCAGTGAC AAGTCTGTCTGCCTATTCACCGATTTTGATTCTCAAACAAATGTGTCACAAAGTAAG GATTCTGATGTGTATATCACAGACAAAACTGTGCTAGACATGAGGTCTATGGACTTC AAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTG TGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGC TCATGACGCTGCGGCTGTGGTCCAGC |
| 157 | nwTCR-2241 complete TRA (TCR α chain) amino acid sequence | ELKVEQNPLFLSMQEGKNYTIYCNYSTTSDRLYWYRQDPGKSLESLFVLLSNGAVKQE GRLMASLDTKARLSTLHITAAVHDLSATYFCAVDKAAGNKLTFGGGTRVLVKPNIQNP DPAVYQLRDSKSSDKSVCLFTDFDSQTNVSQSKDSDVYITDKTVLDMRSMDFKSNSAV AWSNKSDFACANAFNNSIIPEDTFFPSPESSCDVKLVEKSFETDTNLNFQNLSVIGFRILLL KVAGFNLLMTLRLWSS |
| 158 | nwTCR-2241 complete TRA (TCR α chain) nucleotide sequence | GAGCTGAAAGTGGAACAAAACCCTCTGTTCCTGAGCATGCAGGAGGGAAAAAACTA TACCATCTACTGCAATTATTCAACCACTTCAGACAGACTGTATTGGTACAGGCAGGA TCCTGGGAAAAGTCTGGAATCTCTGTTTGTGTTGCTATCAAATGGAGCAGTGAAGCA GGAGGGACGATTAATGGCCTCACTTGATACCAAAGCCCGTCTCAGCACCCTCCACAT CACAGCTGCCGTGCATGACCTCTCTGCCACCTACTTCTGTGCCGTGGACAAAGCTGC AGGCAACAAGCTAACTTTTGGAGGAGGAACCAGGGTGCTAGTTAAACCAAATATCC AGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCT GATGTGTATATCACAGACAAAACTGTGCTAGACATGAGGTCTATGGACTTCAAGAG CAACAGTGCTGTGGCCTGGAGCAACAAATCTGACTTTGCATGTGCAAACGCCTTCAA CAACAGCATTATTCCAGAAGACACCTTCTTCCCCAGCCCAGAAAGTTCCTGTGATGT CAAGCTGGTCGAGAAAAGCTTTGAAACAGATACGAACCTAAACTTTCAAAACCTGT CAGTGATTGGGTTCCGAATCCTCCTCCTGAAAGTGGCCGGGTTTAATCTGCTCATGA CGCTGCGGCTGTGGTCCAGC |
| 159 | nwTCR-2308 complete TRA (TCR α chain) amino acid sequence | KDQVFQPSTVASSEGAVVEIFCNHSVSNAYNFFWYLHFPGCAPRLLVKGSKPSQQGRYN MTYERFSSSLLILQVREADAAVYYCAGSNDYKLSFGAGTTVTVRANIQNPDPAVYQLR DSKSSDKSVCLFTDFDSQTNVSQSKDSDVYITDKTVLDMRSMDFKSNSAVAWSNKSDF ACANAFNNSIIPEDTFFPSPESSCDVKLVEKSFETDTNLNFQNLSVIGFRILLLKVAGFNLL MTLRLWSS |
| 160 | nwTCR-2308 complete TRA (TCR α chain) nucleotide sequence | AAGGACCAAGTGTTTCAGCCTTCCACAGTGGCATCTTCAGAGGGAGCTGTGGTGGA AATCTTCTGTAATCACTCTGTGTCCAATGCTTACAACTTCTTCTGGTACCTTCACTTC CCGGGATGTGCACCAAGACTCCTTGTTAAAGGCTCAAAGCCTTCTCAGCAGGGACG ATACAACATGACCTATGAACGGTTCTCTTCATCGCTGCTCATCCTCCAGGTGCGGGA GGCAGATGCTGCTGTTTACTACTGTGCTGGGTCTAACGACTACAAGCTCAGCTTTGG AGCCGGAACCACAGTAACTGTAAGAGCAAATATCCAGAACCCTGACCCTGCCGTGT ACCAGCTGAGAGACTCTAAATCCAGTGACAAGTCTGTCTGCCTATTCACCGATTTTG ATTCTCAAACAAATGTGTCACAAAGTAAGGATTCTGATGTGTATATCACAGACAAA ACTGTGCTAGACATGAGGTCTATGGACTTCAAGAGCAACAGTGCTGTGGCCTGGAG CAACAAATCTGACTTTGCATGTGCAAACGCCTTCAACAACAGCATTATTCCAGAAGA CACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCT CCTCCTGAAAGTGGCCGGGTTTAATCTGCTCATGACGCTGCGGCTGTGGTCCAGC |
| 161 | nwTCR-2310 complete TRA (TCR α chain) amino acid sequence | SQQGEEDPQALSIQEGENATMNCSYKTSINNLQWYRQNSGRGLVHLILIRSNEREKHSG RLRVTLDTSKKSSSLLITASRAADTASYFCATDGGGGADGLTFGKGTHLIIQPYIQNPDP AVYQLRDSKSSDKSVCLFTDFDSQTNVSQSKDSDVYITDKTVLDMRSMDFKSNSAVAW |
| | | SNKSDFACANAFNNSIIPEDTFFPSPESSCDVKLVEKSFETDTNLNFQNLSVIGFRILLLKV AGFNLLMTLRLWSS |
| 162 | nwTCR-2310 complete TRA (TCR α chain) nucleotide sequence | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGACGGCGGA GGAGGTGCTGACGGACTCACCTTTGGCAAAGGGACTCATCTAATCATCCAGCCCTAT ATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA GAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGA TGTCAAGCTGGTCGAGAAAAGCTTTGAAACAGATACGAACCTAAACTTTCAAAACC TGTCAGTGATTGGGTTCCGAATCCTCCTCCTGAAAGTGGCCGGGTTTAATCTGCTCA TGACGCTGCGGCTGTGGTCCAGC |
| 163 | nwTCR-2390 complete TRA (TCR α chain) amino acid sequence | SQQGEEDPQALSIQEGENATMNCSYKTSINNLQWYRQNSGRGLVHLILIRSNEREKHSG RLRVTLDTSKKSSSLLITASRAADTASYFCATDSGGGADGLTFGKGTHLIIQPYIQNPDPA VYQLRDSKSSDKSVCLFTDFDSQTNVSQSKDSDVYITDKTVLDMRSMDFKSNSAVAWS NKSDFACANAFNNSIIPEDTFFPSPESSCDVKLVEKSFETDTNLNFQNLSVIGFRILLLKVA GFNLLMTLRLWSS |
| 164 | nwTCR-2390 complete TRA (TCR α chain) nucleotide sequence | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGATTCAGGA GGAGGTGCTGACGGACTCACCTTTGGCAAAGGGACTCATCTAATCATCCAGCCCTAT ATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA GAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGA TGTCAAGCTGGTCGAGAAAAGCTTTGAAACAGATACGAACCTAAACTTTCAAAACC TGTCAGTGATTGGGTTCCGAATCCTCCTCCTGAAAGTGGCCGGGTTTAATCTGCTCA TGACGCTGCGGCTGTGGTCCAGC |
| 165 | nwTCR-2392 complete TRA (TCR α chain) amino acid sequence | DQQVKQNSPSLSVQEGRISILNCDYTNSMFDYFLWYKKYPAEGPTFLISISSIKDKNEDG RFTVFLNKSAKHLSLHIVPSQPGDSAVYFCAASYAGSARQLTFGSGTQLTVLPDIQNPDP AVYQLRDSKSSDKSVCLFTDFDSQTNVSQSKDSDVYITDKTVLDMRSMDFKSNSAVAW SNKSDFACANAFNNSIIPEDTFFPSPESSCDVKLVEKSFETDTNLNFQNLSVIGFRILLLKV AGFNLLMTLRLWSS |
| 166 | nwTCR-2392 complete TRA (TCR α chain) nucleotide sequence | GACCAGCAAGTTAAGCAAAATTCACCATCCCTGAGCGTCCAGGAAGGAAGAATTTC TATTCTGAACTGTGACTATACTAACAGCATGTTTGATTATTTCCTATGGTACAAAAA ATACCCTGCTGAAGGTCCTACATTCCTGATATCTATAAGTTCCATTAAGGATAAAAA TGAAGATGGAAGATTCACTGTCTTCTTAAACAAAAGTGCCAAGCACCTCTCTCTGCA CATTGTGCCCTCCCAGCCTGGAGACTCTGCAGTGTACTTCTGTGCAGCAAGCTACGC CGGTTCTGCAAGGCAACTGACCTTTGGATCTGGGACACAATTGACTGTTTTACCTGA TATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA GAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGA TGTCAAGCTGGTCGAGAAAAGCTTTGAAACAGATACGAACCTAAACTTTCAAAACC TGTCAGTGATTGGGTTCCGAATCCTCCTCCTGAAAGTGGCCGGGTTTAATCTGCTCA TGACGCTGCGGCTGTGGTCCAGC |
| 167 | nwTCR-2424 complete TRA (TCR α chain) amino acid sequence | QKEVEQNSGPLSVPEGAIASLNCTYSDRGSQSFFWYRQYSGKSPELIMFIYSNGDKEDGR FTAQLNKASQYVSLLIRDSQPSDSATYLCARKKTSYDKVIFGPGTSLSVIPNIQNPDPAVY QLRDSKSSDKSVCLFTDFDSQTNVSQSKDSDVYITDKTVLDMRSMDFKSNSAVAWSNK SDFACANAFNNSIIPEDTFFPSPESSCDVKLVEKSFETDTNLNFQNLSVIGFRILLLKVAGF NLLMTLRLWSS |
| 168 | nwTCR-2424 complete TRA (TCR α chain) nucleotide sequence | CAGAAGGAGGTGGAGCAGAATTCTGGACCCCTCAGTGTTCCAGAGGGAGCCATTGC CTCTCTCAACTGCACTTACAGTGACCGAGGTTCCCAGTCCTTCTTCTGGTACAGACA ATATTCTGGGAAAAGCCCTGAGTTGATAATGTTCATATACTCCAATGGTGACAAAGA AGATGGAAGGTTTACAGCACAGCTCAATAAAGCCAGCCAGTATGTTTCTCTGCTCAT CAGAGACTCCCAGCCCAGTGATTCAGCCACCTACCTCTGTGCCCGCAAGAAAACCTC CTACGACAAGGTGATATTTGGGCCAGGGACAAGCTTATCAGTCATTCCAAATATCCA GAACCCTGACCCTGCCGTGTACCAGCTGAGAGACTCTAAATCCAGTGACAAGTCTGT |
| | | CTGCCTATTCACCGATTTTGATTCTCAAACAAATGTGTCACAAAGTAAGGATTCTGA TGTGTATATCACAGACAAAACTGTGCTAGACATGAGGTCTATGGACTTCAAGAGCA ACAGTGCTGTGGCCTGGAGCAACAAATCTGACTTTGCATGTGCAAACGCCTTCAACA ACAGCATTATTCCAGAAGACACCTTCTTCCCCAGCCCAGAAAGTTCCTGTGATGTCA AGCTGGTCGAGAAAAGCTTTGAAACAGATACGAACCTAAACTTTCAAAACCTGTCA GTGATTGGGTTCCGAATCCTCCTCCTGAAAGTGGCCGGGTTTAATCTGCTCATGACG CTGCGGCTGTGGTCCAGC |
| 169 | nwTCR-2561 complete TRA (TCR α chain) amino acid sequence | GNSVTQMEGPVTLSEEAFLTINCTYTATGYPSLFWYVQYPGEGLQLLLKATKADDKGS NKGFEATYRKETTSFHLEKGSVQVSDSAVYFCALPAGGGADGLTFGKGTHLIIQPYIQNP DPAVYQLRDSKSSDKSVCLFTDFDSQTNVSQSKDSDVYITDKTVLDMRSMDFKSNSAV AWSNKSDFACANAFNNSIIPEDTFFPSPESSCDVKLVEKSFETDTNLNFQNLSVIGFRILLL KVAGFNLLMTLRLWSS |
| 170 | nwTCR-2561 complete TRA (TCR α chain) nucleotide sequence | GGAAATTCAGTGACCCAGATGGAAGGGCCAGTGACTCTCTCAGAAGAGGCCTTCCT GACTATAAACTGCACGTACACAGCCACAGGATACCCTTCCCTTTTCTGGTATGTCCA ATATCCTGGAGAAGGTCTACAGCTCCTCCTGAAAGCCACGAAGGCTGATGACAAGG GAAGCAACAAAGGTTTTGAAGCCACATACCGTAAAGAAACCACTTCTTTCCACTTGG AGAAAGGCTCAGTTCAAGTGTCAGACTCAGCGGTGTACTTCTGTGCTCTGCCCGCAG GAGGAGGTGCTGACGGACTCACCTTTGGCAAAGGGACTCATCTAATCATCCAGCCCT ATATCCAGAACCCTGACCCTGCCGTGTACCAGCTGAGAGACTCTAAATCCAGTGACA AGTCTGTCTGCCTATTCACCGATTTTGATTCTCAAACAAATGTGTCACAAAGTAAGG ATTCTGATGTGTATATCACAGACAAAACTGTGCTAGACATGAGGTCTATGGACTTCA AGAGCAACAGTGCTGTGGCCTGGAGCAACAAATCTGACTTTGCATGTGCAAACGCC TTCAACAACAGCATTATTCCAGAAGACACCTTCTTCCCCAGCCCAGAAAGTTCCTGT GATGTCAAGCTGGTCGAGAAAAGCTTTGAAACAGATACGAACCTAAACTTTCAAAA CCTGTCAGTGATTGGGTTCCGAATCCTCCTCCTGAAAGTGGCCGGGTTTAATCTGCT CATGACGCTGCGGCTGTGGTCCAGC |
| 171 | nwTCR-2563 complete TRA (TCR α chain) amino acid sequence | SQQGEEDPQALSIQEGENATMNCSYKTSINNLQWYRQNSGRGLVHLILIRSNEREKHSG RLRVTLDTSKKSSSLLITASRAADTASYFCATDRTGGYNKLIFGAGTRLAVHPYIQNPDP AVYQLRDSKSSDKSVCLFTDFDSQTNVSQSKDSDVYITDKTVLDMRSMDFKSNSAVAW SNKSDFACANAFNNSIIPEDTFFPSPESSCDVKLVEKSFETDTNLNFQNLSVIGFRILLLKV AGFNLLMTLRLWSS |
| 172 | nwTCR-2563 complete TRA (TCR α chain) nucleotide sequence | AGTCAACAGGGAGAAGAGGATCCTCAGGCCTTGAGCATCCAGGAGGGTGAAAATGC CACCATGAACTGCAGTTACAAAACTAGTATAAACAATTTACAGTGGTATAGACAAA ATTCAGGTAGAGGCCTTGTCCACCTAATTTTAATACGTTCAAATGAAAGAGAGAAAC ACAGTGGAAGATTAAGAGTCACGCTTGACACTTCCAAGAAAAGCAGTTCCTTGTTG ATCACGGCTTCCCGGGCAGCAGACACTGCTTCTTACTTCTGTGCTACGGACCGAACT GGTGGCTACAATAAGCTGATTTTTGGAGCAGGGACCAGGCTGGCTGTACACCCATAT ATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA GAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGA TGTCAAGCTGGTCGAGAAAAGCTTTGAAACAGATACGAACCTAAACTTTCAAAACC TGTCAGTGATTGGGTTCCGAATCCTCCTCCTGAAAGTGGCCGGGTTTAATCTGCTCA TGACGCTGCGGCTGTGGTCCAGC |
| 173 | nwTCR-2595 complete TRA (TCR α chain) amino acid sequence | ILNVEQSPQSLHVQEGDSTNFTCSFPSSNFYALHWYRWETAKSPEALFVMTLNGDEKKK GRISATLNTKEGYSYLYIKGSQPEDSATYLCAFNNQGGKLIFGQGTELSVKPNIQNPDPA VYQLRDSKSSDKSVCLFTDFDSQTNVSQSKDSDVYITDKTVLDMRSMDFKSNSAVAWS NKSDFACANAFNNSIIPEDTFFPSPESSCDVKLVEKSFETDTNLNFQNLSVIGFRILLLKVA GFNLLMTLRLWSS |
| 174 | nwTCR-2595 complete TRA (TCR α chain) nucleotide sequence | ATACTGAACGTGGAACAAAGTCCTCAGTCACTGCATGTTCAGGAGGGAGACAGCAC CAATTTCACCTGCAGCTTCCCTTCCAGCAATTTTTATGCCTTACACTGGTACAGATGG GAAACTGCAAAAAGCCCCGAGGCCTTGTTTGTAATGACTTTAAATGGGGATGAAAA GAAGAAAGGACGAATAAGTGCCACTCTTAATACCAAGGAGGGTTACAGCTATTTGT ACATCAAAGGATCCCAGCCTGAAGACTCAGCCACATACCTCTGTGCCTTTAATAACC AGGGAGGAAAGCTTATCTTCGGACAGGGAACGGAGTTATCTGTGAAACCCAATATC CAGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCT GATGTGTATATCACAGACAAAACTGTGCTAGACATGAGGTCTATGGACTTCAAGAG CAACAGTGCTGTGGCCTGGAGCAACAAATCTGACTTTGCATGTGCAAACGCCTTCAA CAACAGCATTATTCCAGAAGACACCTTCTTCCCCAGCCCAGAAAGTTCCTGTGATGT CAAGCTGGTCGAGAAAAGCTTTGAAACAGATACGAACCTAAACTTTCAAAACCTGT CAGTGATTGGGTTCCGAATCCTCCTCCTGAAAGTGGCCGGGTTTAATCTGCTCATGA CGCTGCGGCTGTGGTCCAGC |
| 175 | nwTCR-2629 complete TRA (TCR α chain) amino acid sequence | AQSVTQLDSHVSVSEGTPVLLRCNYSSSYSPSLFWYVQHPNKGLQLLLKYTSAATLVKG INGFEAEFKKSETSFHLTKPSAHMSDAAEYFCVVSVWTDKLIFGTGTRLQVFPNIQNPDP |
| | | AVYQLRDSKSSDKSVCLFTDFDSQTNVSQSKDSDVYITDKTVLDMRSMDFKSNSAVAW SNKSDFACANAFNNSIIPEDTFFPSPESSCDVKLVEKSFETDTNLNFQNLSVIGFRILLLKV AGFNLLMTLRLWSS |
| 176 | nwTCR-2629 complete TRA (TCR α chain) nucleotide sequence | GCCCAGTCGGTGACCCAGCTTGACAGCCACGTCTCTGTCTCTGAAGGAACCCCGGTG CTGCTGAGGTGCAACTACTCATCTTCTTATTCACCATCTCTCTTCTGGTATGTGCAAC ACCCCAACAAAGGACTCCAGCTTCTCCTGAAGTACACATCAGCGGCCACCCTGGTTA AAGGCATCAACGGTTTTGAGGCTGAATTTAAGAAGAGTGAAACCTCCTTCCACCTGA CGAAACCCTCAGCCCATATGAGCGACGCGGCTGAGTACTTCTGTGTTGTGAGTGTAT GGACCGACAAGCTCATCTTTGGGACTGGGACCAGATTACAAGTCTTTCCAAATATCC AGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCT GATGTGTATATCACAGACAAAACTGTGCTAGACATGAGGTCTATGGACTTCAAGAG CAACAGTGCTGTGGCCTGGAGCAACAAATCTGACTTTGCATGTGCAAACGCCTTCAA CAACAGCATTATTCCAGAAGACACCTTCTTCCCCAGCCCAGAAAGTTCCTGTGATGT CAAGCTGGTCGAGAAAAGCTTTGAAACAGATACGAACCTAAACTTTCAAAACCTGT CAGTGATTGGGTTCCGAATCCTCCTCCTGAAAGTGGCCGGGTTTAATCTGCTCATGA CGCTGCGGCTGTGGTCCAGC |
| 177 | nwTCR-0125 β chain CDR1 | MDHEN |
| 178 | nwTCR-0125 β chain CDR2 | SYDVKM |
| 179 | nwTCR-0125 β chain CDR3 | ASSPPHLASGASTDTQY |
| 180 | nwTCR-0126 β chain CDR1 | SGDLS |
| 181 | nwTCR-0126 β chain CDR2 | YYNGEE |
| 182 | nwTCR-0126 β chain CDR3 | ASSPRGGDSILDTQY |
| 183 | nwTCR-0127 β chain CDR1 | MNHEY |
| 194 | nwTCR-0127 β chain CDR2 | SMNVEV |
| 185 | nwTCR-0127 β chain CDR3 | ASSTRDRDNEQF |
| 186 | nwTCR-1708 β chain CDR1 | LGHNT |
| 187 | nwTCR-1708 β chain CDR2 | FRNRAP |
| 188 | nwTCR-1708 β chain CDR3 | ASGPEGLDNEQF |
| 189 | nwTCR-1862 β chain CDR1 | DFQATT |
| 190 | nwTCR-1862 β chain CDR2 | SNEGSKA |
| 191 | nwTCR-1862 β chain CDR3 | SARFANYYEQY |
| 192 | nwTCR-2162 β chain CDR1 | MGHDK |
| 193 | nwTCR-2162 β chain CDR2 | SYGVNS |
| 194 | nwTCR-2162 β chain CDR3 | ASSDPVHGDYGYT |
| 195 | nwTCR-2241 β chain CDR1 | KGHSY |
| 196 | nwTCR-2241 β chain CDR2 | FQNENV |
| 197 | nwTCR-2241 β chain CDR3 | ASSPHHRYEQY |
| 198 | nwTCR-2308 β chain CDR1 | MNHEY |
| 199 | nwTCR-2308 β chain CDR2 | SMNVEV |
| 200 | nwTCR-2308 β chain CDR3 | ASSTSPYNEQF |
| 201 | nwTCR-2310 β chain CDR1 | SGDLS |
| 202 | nwTCR-2310 β chain CDR2 | YYNGEE |
| 203 | nwTCR-2310 β chain CDR3 | ASSLHRDSDTEAF |
| 204 | nwTCR-2390 β chain CDR1 | SGDLS |
| 205 | nwTCR-2390 β chain CDR2 | YYNGEE |
| 206 | nwTCR-2390 β chain CDR3 | ASSHGRDSTDTQY |
| 207 | nwTCR-2392 β chain CDR1 | SGHTA |
| 208 | nwTCR-2392 β chain CDR2 | FQGNSA |
| 209 | nwTCR-2392 β chain CDR3 | ASSPGGNYGYT |
| 210 | nwTCR-2424 β chain CDR1 | SGDLS |
| 211 | nwTCR-2424 β chain CDR2 | YYNGEE |
| 212 | nwTCR-2424 β chain CDR3 | ASSLGDSEQF |
| 213 | nwTCR-2561 β chain CDR1 | SGHTA |
| 214 | nwTCR-2561 β chain CDR2 | FQGNSA |
| 215 | nwTCR-2561 β chain CDR3 | ASSFGTSGDQYEQY |
| 216 | nwTCR-2563 β chain CDR1 | SGHNT |
| 217 | nwTCR-2563 β chain CDR2 | YYREEE |
| 218 | nwTCR-2563 β chain CDR3 | ASSLQTGDEQY |
| 219 | nwTCR-2595 β chain CDR1 | MNHEY |
| 220 | nwTCR-2595 β chain CDR2 | SMNVEV |
| 221 | nwTCR-2595 β chain CDR3 | ASSSGTGGGAEAF |
| 222 | nwTCR-2629 β chain CDR1 | DFQATT |
| 223 | nwTCR-2629 β chain CDR2 | SNEGSKA |
| 224 | nwTCR-2629 β chain CDR3 | SANRDFGIYEQY |
| 225 | nwTCR-0125 amino acid sequence encoded by TRBV gene | DVKVTQSSRYLVKRTGEKVFLECVQDMDHENMFWYRQDPGLGLRLIYFSYDVKMKEK GDIPEGYSVSREKKERFSLILESASTNQTSMYLCAS |
| 226 | nwTCR-0125 nucleotide sequence of TRBV gene | GATGTGAAAGTAACCCAGAGCTCGAGATATCTAGTCAAAAGGACGGGAGAGAAAG TTTTTCTGGAATGTGTCCAGGATATGGACCATGAAAATATGTTCTGGTATCGACAAG ACCCAGGTCTGGGGCTACGGCTGATCTATTTCTCATATGATGTTAAAATGAAAGAAA AAGGAGATATTCCTGAGGGGTACAGTGTCTCTAGAGAGAAGAAGGAGCGCTTCTCC CTGATTCTGGAGTCCGCCAGCACCAACCAGACATCTATGTACCTCTGTGCCAGC |
| 227 | nwTCR-0125 amino acid sequence encoded by TRBD gene | SG |
| 228 | nwTCR-0125 nucleotide sequence of TRBD gene | AGCGGG |
| 229 | nwTCR-0125 amino acid sequence encoded by TRBJ gene | STDTQYFGPGTRLTVL |
| 230 | nwTCR-0125 nucleotide sequence of TRBJ gene | AGCACAGATACGCAGTATTTTGGCCCAGGCACCCGGCTGACAGTGCTC |
| 231 | nwTCR-0126 amino acid sequence encoded by TRBV gene | DSGVTQTPKHLITATGQRVTLRCSPRSGDLSVYWYQQSLDQGLQFLIQYYNGEERAKG NILERFSAQQFPDLHSELNLSSLELGDSALYFCASS |
| 232 | nwTCR-0126 nucleotide sequence of TRBV gene | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGC |
| 233 | nwTCR-0126 amino acid sequence encoded by TRBD gene | G |
| 234 | nwTCR-0126 nucleotide sequence of TRBD gene | GGG |
| 235 | nwTCR-0126 amino acid sequence encoded by TRBJ gene | DTQYFGPGTRLTVL |
| 236 | nwTCR-0126 nucleotide sequence of TRBJ gene | GATACGCAGTATTTTGGCCCAGGCACCCGGCTGACAGTGCTC |
| 237 | nwTCR-0127 amino acid sequence encoded by TRBV gene | EAQVTQNPRYLITVTGKKLTVTCSQNMNHEYMSWYRQDPGLGLRQIYYSMNVEVTDK GDVPEGYKVSRKEKRNFPLILESPSPNQTSLYFCAS |
| 238 | nwTCR-0127 nucleotide sequence of TRBV gene | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGC |
| 239 | nwTCR-0127 amino acid sequence encoded by TRBD gene | DR |
| 240 | nwTCR-0127 nucleotide sequence of TRBD gene | GACAGG |
| 241 | nwTCR-0127 amino acid sequence encoded by TRBJ gene | NEQFFGPGTRLTVL |
| 242 | nwTCR-0127 nucleotide sequence of TRBJ gene | AATGAGCAGTTCTTCGGGCCAGGGACACGGCTCACCGTGCTA |
| 243 | nwTCR-1708 amino acid sequence encoded by TRBV gene | DARVTQTPRDKVTEMGQEVTMRCQPILGHNTVFWYRQTMMQGLELLAYFRNRAPLDD SGMPKDRFSAEMPDATLATLKIQPSEPRDSAVYFCASG |
| 244 | nwTCR-1708 nucleotide sequence of TRBV gene | GATGCTAGAGTCACCCAGACACCAAGGGACAAGGTGACAGAGATGGGACAAGAAG TAACAATGAGATGTCAGCCAATTTTAGGCCACAATACTGTTTTCTGGTACAGACAGA CCATGATGCAAGGACTGGAGTTGCTGGCTTACTTCCGCAACCGGGCTCCTCTAGATG ATTCGGGGATGCCGAAGGATCGATTCTCAGCAGAGATGCCTGATGCAACTTTAGCC |
| | | ACTCTGAAGATCCAGCCCTCAGAACCCAGGGACTCAGCTGTGTATTTTTGTGCTAGT GGT |
| 245 | nwTCR-1708 amino acid sequence encoded by TRBD gene | G |
| 246 | nwTCR-1708 nucleotide sequence of TRBD gene | GGG |
| 247 | nwTCR-1708 amino acid sequence encoded by TRBJ gene | NEQFFGPGTRLTVL |
| 248 | nwTCR-1708 nucleotide sequence of TRBJ gene | AATGAGCAGTTCTTCGGGCCAGGGACACGGCTCACCGTGCTA |
| 249 | nwTCR-1862 amino acid sequence encoded by TRBV gene | GAVVSQHPSWVICKSGTSVKIECRSLDFQATTMFWYRQFPKQSLMLMATSNEGSKATY EQGVEKDKFLINHASLTLSTLTVTSAHPEDSSFYICSA |
| 250 | nwTCR-1862 nucleotide sequence of TRBV gene | GGTGCTGTCGTCTCTCAACATCCGAGCTGGGTTATCTGTAAGAGTGGAACCTCTGTG AAGATCGAGTGCCGTTCCCTGGACTTTCAGGCCACAACTATGTTTTGGTATCGTCAG TTCCCGAAACAGAGTCTCATGCTGATGGCAACTTCCAATGAGGGCTCCAAGGCCAC ATACGAGCAAGGCGTCGAGAAGGACAAGTTTCTCATCAACCATGCAAGCCTGACCT TGTCCACTCTGACAGTGACCAGTGCCCATCCTGAAGACAGCAGCTTCTACATCTGCA GTGCT |
| | nwTCR-1862 amino acid sequence encoded by TRBD gene | NA (abbreviation for "Not Applicable", referring to no sequence in the context herein) |
| | nwTCR-1862 nucleotide sequence of TRBD gene | NA |
| 251 | nwTCR-1862 amino acid sequence encoded by TRBJ gene | YEQYFGPGTRLTVT |
| 252 | nwTCR-1862 nucleotide sequence of TRBJ gene | TACGAGCAGTACTTCGGGCCGGGCACCAGGCTCACGGTCACA |
| 253 | nwTCR-2162 amino acid sequence encoded by TRBV gene | EADIYQTPRYLVIGTGKKITLECSQTMGHDKMYWYQQDPGMELHLIHYSYGVNSTEKG DLSSESTVSRIRTEHFPLTLESARPSHTSQYLCASS |
| 254 | nwTCR-2162 nucleotide sequence of TRBV gene | GAAGCTGACATCTACCAGACCCCAAGATACCTTGTTATAGGGACAGGAAAGAAGAT CACTCTGGAATGTTCTCAAACCATGGGCCATGACAAAATGTACTGGTATCAACAAG ATCCAGGAATGGAACTACACCTCATCCACTATTCCTATGGAGTTAATTCCACAGAGA AGGGAGATCTTTCCTCTGAGTCAACAGTCTCCAGAATAAGGACGGAGCATTTTCCCC TGACCCTGGAGTCTGCCAGGCCCTCACATACCTCTCAGTACCTCTGTGCCAGCAGT |
| | nwTCR-2162 amino acid sequence encoded by TRBD gene | NA |
| | nwTCR-2162 nucleotide sequence of TRBD gene | NA |
| 255 | nwTCR-2162 amino acid sequence encoded by TRBJ gene | YGYTFGSGTRLTVV |
| 256 | nwTCR-2162 nucleotide sequence of TRBJ gene | TATGGCTACACCTTCGGTTCGGGGACCAGGTTAACCGTTGTA |
| 257 | nwTCR-2241 amino acid sequence encoded by TRBV gene | GEEVAQTPKHLVRGEGQKAKLYCAPIKGHSYVFWYQQVLKNEFKFLISFQNENVFDET GMPKERFSAKCLPNSPCSLEIQATKLEDSAVYFCASS |
| 258 | nwTCR-2241 nucleotide sequence of TRBV gene | GGTGAAGAAGTCGCCCAGACTCCAAAACATCTTGTCAGAGGGGAAGGACAGAAAG CAAAATTATATTGTGCCCCAATAAAAGGACACAGTTATGTTTTTTGGTACCAACAGG TCCTGAAAAACGAGTTCAAGTTCTTGATTTCCTTCCAGAATGAAAATGTCTTTGATG AAACAGGTATGCCCAAGGAAAGATTTTCAGCTAAGTGCCTCCCAAATTCACCCTGTA GCCTTGAGATCCAGGCTACGAAGCTTGAGGATTCAGCAGTGTATTTTTGTGCCAGCA GC |
| 259 | nwTCR-2241 amino acid sequence encoded by TRBD gene | R |
| 260 | nwTCR-2241 nucleotide sequence of TRBD gene | AGG |
| 261 | nwTCR-2241 amino acid sequence encoded by TRBJ gene | YEQYFGPGTRLTVT |
| 262 | nwTCR-2241 nucleotide sequence of TRBJ gene | TACGAGCAGTACTTCGGGCCGGGCACCAGGCTCACGGTCACA |
| 263 | nwTCR-2308 amino acid sequence encoded by TRBV gene | EAQVTQNPRYLITVTGKKLTVTCSQNMNHEYMSWYRQDPGLGLRQIYYSMNVEVTDK GDVPEGYKVSRKEKRNFPLILESPSPNQTSLYFCAS |
| 264 | nwTCR-2308 nucleotide sequence of TRBV gene | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGC |
| 265 | nwTCR-2308 amino acid sequence encoded by TRBD gene | TS |
| 266 | nwTCR-2308 nucleotide sequence of TRBD gene | ACTAGC |
| 267 | nwTCR-2308 amino acid sequence encoded by TRBJ gene | YNEQFFGPGTRLTVL |
| 268 | nwTCR-2308 nucleotide sequence of TRBJ gene | TACAATGAGCAGTTCTTCGGGCCAGGGACACGGCTCACCGTGCTA |
| 269 | nwTCR-2310 amino acid sequence encoded by TRBV gene | DSGVTQTPKHLITATGQRVTLRCSPRSGDLSVYWYQQSLDQGLQFLIQYYNGEERAKG NILERFSAQQFPDLHSELNLSSLELGDSALYFCASS |
| 270 | nwTCR-2310 nucleotide sequence of TRBV gene | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGC |
| 271 | nwTCR-2310 amino acid sequence encoded by TRBD gene | D |
| 272 | nwTCR-2310 nucleotide sequence of TRBD gene | GAC |
| 273 | nwTCR-2310 amino acid sequence encoded by TRBJ gene | TEAFFGQGTRLTVV |
| 274 | nwTCR-2310 nucleotide sequence of TRBJ gene | ACTGAAGCTTTCTTTGGACAAGGCACCAGACTCACAGTTGTA |
| 275 | nwTCR-2390 amino acid sequence encoded by TRBV gene | DSGVTQTPKHLITATGQRVTLRCSPRSGDLSVYWYQQSLDQGLQFLIQYYNGEERAKG NILERFSAQQFPDLHSELNLSSLELGDSALYFCASS |
| 276 | nwTCR-2390 nucleotide sequence of TRBV gene | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGC |
| 277 | nwTCR-2390 amino acid sequence encoded by TRBD gene | GR |
| 278 | nwTCR-2390 nucleotide sequence of TRBD gene | GGGAGG |
| 279 | nwTCR-2390 amino acid sequence encoded by TRBJ gene | STDTQYFGPGTRLTVL |
| 280 | nwTCR-2390 nucleotide sequence of TRBJ gene | AGCACAGATACGCAGTATTTTGGCCCAGGCACCCGGCTGACAGTGCTC |
| 281 | nwTCR-2392 amino acid sequence encoded by TRBV gene | GAGVSQSPSNKVTEKGKDVELRCDPISGHTALYWYRQRLGQGLEFLIYFQGNSAPDKSG LPSDRFSAERTGESVSTLTIQRTQQEDSAVYLCAS |
| 282 | nwTCR-2392 nucleotide sequence of TRBV gene | GGAGCTGGAGTCTCCCAGTCCCCCAGTAACAAGGTCACAGAGAAGGGAAAGGATGT AGAGCTCAGGTGTGATCCAATTTCAGGTCATACTGCCCTTTACTGGTACCGACAGAG GCTGGGGCAGGGCCTGGAGTTTTTAATTTACTTCCAAGGCAACAGTGCACCAGACA AATCAGGGCTGCCCAGTGATCGCTTCTCTGCAGAGAGGACTGGGGAATCCGTCTCCA CTCTGACGATCCAGCGCACACAGCAGGAGGACTCGGCCGTGTATCTCTGTGCCAGC |
| 283 | nwTCR-2392 amino acid sequence encoded by TRBD gene | GG |
| 284 | nwTCR-2392 nucleotide sequence of TRBD gene | GGGGGG |
| 285 | nwTCR-2392 amino acid sequence encoded by TRBJ gene | NYGYTFGSGTRLTVV |
| 286 | nwTCR-2392 nucleotide sequence of TRBJ gene | AACTATGGCTACACCTTCGGTTCGGGGACCAGGTTAACCGTTGTA |
| 287 | nwTCR-2424 amino acid sequence encoded by TRBV gene | DSGVTQTPKHLITATGQRVTLRCSPRSGDLSVYWYQQSLDQGLQFLIQYYNGEERAKG NILERFSAQQFPDLHSELNLSSLELGDSALYFCASS |
| 288 | nwTCR-2424 nucleotide sequence of TRBV gene | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGC |
| 289 | nwTCR-2424 amino acid sequence encoded by TRBD gene | D |
| 290 | nwTCR-2424 nucleotide sequence of TRBD gene | GAC |
| 291 | nwTCR-2424 amino acid sequence encoded by TRBJ gene | EQFFGPGTRLTVL |
| 292 | nwTCR-2424 nucleotide sequence of TRBJ gene | GAGCAGTTCTTCGGGCCAGGGACACGGCTCACCGTGCTA |
| 293 | nwTCR-2561 amino acid sequence encoded by TRBV gene | GAGVSQSPSNKVTEKGKDVELRCDPISGHTALYWYRQSLGQGLEFLIYFQGNSAPDKSG LPSDRFSAERTGGSVSTLTIQRTQQEDSAVYLCASS |
| 294 | nwTCR-2561 nucleotide sequence of TRBV gene | GGAGCTGGAGTCTCCCAGTCCCCCAGTAACAAGGTCACAGAGAAGGGAAAGGATGT AGAGCTCAGGTGTGATCCAATTTCAGGTCATACTGCCCTTTACTGGTACCGACAGAG CCTGGGGCAGGGCCTGGAGTTTTTAATTTACTTCCAAGGCAACAGTGCACCAGACAA ATCAGGGCTGCCCAGTGATCGCTTCTCTGCAGAGAGGACTGGGGGATCCGTCTCCAC TCTGACGATCCAGCGCACACAGCAGGAGGACTCGGCCGTGTATCTCTGTGCCAGCA GC |
| 295 | nwTCR-2561 amino acid sequence encoded by TRBD gene | GTSG |
| 296 | nwTCR-2561 nucleotide sequence of TRBD gene | GGGACTAGCGGG |
| 297 | nwTCR-2561 amino acid sequence encoded by TRBJ gene | YEQYFGPGTRLTVT |
| 298 | nwTCR-2561 nucleotide sequence of TRBJ gene | TACGAGCAGTACTTCGGGCCGGGCACCAGGCTCACGGTCACA |
| 299 | nwTCR-2563 amino acid sequence encoded by TRBV gene | ETGVTQSPTHLIKTRGQQVTLRCSSQSGHNTVSWYQQALGQGPQFIFQYYREEENGRGN FPPRFSGLQFPNYSSELNVNALELDDSALYLCASS |
| 300 | nwTCR-2563 nucleotide sequence of TRBV gene | GAGACTGGAGTCACCCAAAGTCCCACACACCTGATCAAAACGAGAGGACAGCAAGT GACTCTGAGATGCTCTTCTCAGTCTGGGCACAACACTGTGTCCTGGTACCAACAGGC CCTGGGTCAGGGGCCCCAGTTTATCTTTCAGTATTATAGGGAGGAAGAGAATGGCA GAGGAAACTTCCCTCCTAGATTCTCAGGTCTCCAGTTCCCTAATTATAGCTCTGAGCT GAATGTGAACGCCTTGGAGCTGGACGACTCGGCCCTGTATCTCTGTGCCAGCAGC |
| 301 | nwTCR-2563 amino acid sequence encoded by TRBD gene | TG |
| 302 | nwTCR-2563 nucleotide sequence of TRBD gene | ACAGGG |
| 303 | nwTCR-2563 amino acid sequence encoded by TRBJ gene | EQYFGPGTRLTVT |
| 304 | nwTCR-2563 nucleotide sequence of TRBJ gene | GAGCAGTACTTCGGGCCGGGCACCAGGCTCACGGTCACA |
| 305 | nwTCR-2595 amino acid sequence encoded by TRBV gene | EAQVTQNPRYLITVTGKKLTVTCSQNMNHEYMSWYRQDPGLGLRQIYYSMNVEVTDK GDVPEGYKVSRKEKRNFPLILESPSPNQTSLYFCASS |
| 306 | nwTCR-2595 nucleotide sequence of TRBV gene | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGCAGT |
| 307 | nwTCR-2595 amino acid sequence encoded by TRBD gene | GTG |
| 308 | nwTCR-2595 nucleotide sequence of TRBD gene | GGGACAGGG |
| 309 | nwTCR-2595 amino acid sequence encoded by TRBJ gene | EAFFGQGTRLTVV |
| 310 | nwTCR-2595 nucleotide sequence of TRBJ gene | GAAGCTTTCTTTGGACAAGGCACCAGACTCACAGTTGTA |
| 311 | nwTCR-2629 amino acid sequence encoded by TRBV gene | GAVVSQHPSWVICKSGTSVKIECRSLDFQATTMFWYRQFPKQSLMLMATSNEGSKATY EQGVEKDKFLINHASLTLSTLTVTSAHPEDSSFYICS |
| 312 | nwTCR-2629 nucleotide sequence of TRBV gene | GGTGCTGTCGTCTCTCAACATCCGAGCTGGGTTATCTGTAAGAGTGGAACCTCTGTG AAGATCGAGTGCCGTTCCCTGGACTTTCAGGCCACAACTATGTTTTGGTATCGTCAG TTCCCGAAACAGAGTCTCATGCTGATGGCAACTTCCAATGAGGGCTCCAAGGCCAC ATACGAGCAAGGCGTCGAGAAGGACAAGTTTCTCATCAACCATGCAAGCCTGACCT TGTCCACTCTGACAGTGACCAGTGCCCATCCTGAAGACAGCAGCTTCTACATCTGCA GT |
| 313 | nwTCR-2629 amino acid sequence encoded by TRBD gene | D |
| 314 | nwTCR-2629 nucleotide sequence of TRBD gene | GAC |
| 315 | nwTCR-2629 amino acid sequence encoded by TRBJ gene | YEQYFGPGTRLTVT |
| 316 | nwTCR-2629 nucleotide sequence of TRBJ gene | TACGAGCAGTACTTCGGGCCGGGCACCAGGCTCACGGTCACA |
| 317 | nwTCR-0125 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 318 | nwTCR-0125 nucleotide sequence of TRBC gene | AGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCC GACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC |
| | | CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 319 | nwTCR-0126 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 320 | nwTCR-0126 nucleotide sequence of TRBC gene | AGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCC GACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 321 | nwTCR-0127 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 322 | nwTCR-0127 nucleotide sequence of TRBC gene | AGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCC GACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 323 | nwTCR-1708 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 324 | nwTCR-1708 nucleotide sequence of TRBC gene | AGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCC GACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 325 | nwTCR-1862 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 326 | nwTCR-1862 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 327 | nwTCR-2162 amino acid sequence encoded by TRBC gene | EDLNKVFPPEVAVFEPSEAEISHTQKATLVCLATGFFPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSVSYQQGVLSATILYEILLGKATLYAVLVSALVLMAMVKRK DF |
| 328 | nwTCR-2162 nucleotide sequence of TRBC gene | GAGGACCTGAACAAGGTGTTCCCACCCGAGGTCGCTGTGTTTGAGCCATCAGAAGC AGAGATCTCCCACACCCAAAAGGCCACACTGGTGTGCCTGGCCACAGGCTTCTTCCC TGACCACGTGGAGCTGAGCTGGTGGGTGAATGGGAAGGAGGTGCACAGTGGGGTCA GCACGGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCCGTCACCCAGATCGTCAGCGCCGAGGCCTGGGGTAGAGCAGACTGTG GCTTTACCTCGGTGTCCTACCAGCAAGGGGTCCTGTCTGCCACCATCCTCTATGAGA TCCTGCTAGGGAAGGCCACCCTGTATGCTGTGCTGGTCAGCGCCCTTGTGTTGATGG CCATGGTCAAGAGAAAGGATTTC |
| 329 | nwTCR-2241 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 330 | nwTCR-2241 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 331 | nwTCR-2308 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 332 | nwTCR-2308 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 333 | nwTCR-2310 amino acid sequence encoded by TRBC gene | EDLNKVFPPEVAVFEPSEAEISHTQKATLVCLATGFFPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSVSYQQGVLSATILYEILLGKATLYAVLVSALVLMAMVKRK DF |
| 334 | nwTCR-2310 nucleotide sequence of TRBC gene | GAGGACCTGAACAAGGTGTTCCCACCCGAGGTCGCTGTGTTTGAGCCATCAGAAGC AGAGATCTCCCACACCCAAAAGGCCACACTGGTGTGCCTGGCCACAGGCTTCTTCCC TGACCACGTGGAGCTGAGCTGGTGGGTGAATGGGAAGGAGGTGCACAGTGGGGTCA GCACGGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCCGTCACCCAGATCGTCAGCGCCGAGGCCTGGGGTAGAGCAGACTGTG GCTTTACCTCGGTGTCCTACCAGCAAGGGGTCCTGTCTGCCACCATCCTCTATGAGA TCCTGCTAGGGAAGGCCACCCTGTATGCTGTGCTGGTCAGCGCCCTTGTGTTGATGG CCATGGTCAAGAGAAAGGATTTC |
| 335 | nwTCR-2390 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 336 | nwTCR-2390 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 337 | nwTCR-2392 amino acid sequence encoded by TRBC gene | EDLNKVFPPEVAVFEPSEAEISHTQKATLVCLATGFFPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSVSYQQGVLSATILYEILLGKATLYAVLVSALVLMAMVKRK DF |
| 338 | nwTCR-2392 nucleotide sequence of TRBC gene | GAGGACCTGAACAAGGTGTTCCCACCCGAGGTCGCTGTGTTTGAGCCATCAGAAGC AGAGATCTCCCACACCCAAAAGGCCACACTGGTGTGCCTGGCCACAGGCTTCTTCCC TGACCACGTGGAGCTGAGCTGGTGGGTGAATGGGAAGGAGGTGCACAGTGGGGTCA GCACGGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCCGTCACCCAGATCGTCAGCGCCGAGGCCTGGGGTAGAGCAGACTGTG GCTTTACCTCGGTGTCCTACCAGCAAGGGGTCCTGTCTGCCACCATCCTCTATGAGA TCCTGCTAGGGAAGGCCACCCTGTATGCTGTGCTGGTCAGCGCCCTTGTGTTGATGG CCATGGTCAAGAGAAAGGATTTC |
| 339 | nwTCR-2424 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 340 | nwTCR-2424 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 341 | nwTCR-2561 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 342 | nwTCR-2561 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 343 | nwTCR-2563 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 344 | nwTCR-2563 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 345 | nwTCR-2595 amino acid sequence encoded by TRBC gene | EDLNKVFPPEVAVFEPSEAEISHTQKATLVCLATGFFPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSVSYQQGVLSATILYEILLGKATLYAVLVSALVLMAMVKRK DF |
| 346 | nwTCR-2595 nucleotide sequence of TRBC gene | GAGGACCTGAACAAGGTGTTCCCACCCGAGGTCGCTGTGTTTGAGCCATCAGAAGC AGAGATCTCCCACACCCAAAAGGCCACACTGGTGTGCCTGGCCACAGGCTTCTTCCC TGACCACGTGGAGCTGAGCTGGTGGGTGAATGGGAAGGAGGTGCACAGTGGGGTCA GCACGGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG |
| | | GGCCAAACCCGTCACCCAGATCGTCAGCGCCGAGGCCTGGGGTAGAGCAGACTGTG GCTTTACCTCGGTGTCCTACCAGCAAGGGGTCCTGTCTGCCACCATCCTCTATGAGA TCCTGCTAGGGAAGGCCACCCTGTATGCTGTGCTGGTCAGCGCCCTTGTGTTGATGG CCATGGTCAAGAGAAAGGATTTC |
| 347 | nwTCR-2629 amino acid sequence encoded by TRBC gene | EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 348 | nwTCR-2629 nucleotide sequence of TRBC gene | GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 349 | nwTCR-0125 complete TRB (TCR β chain) amino acid sequence | DVKVTQSSRYLVKRTGEKVFLECVQDMDHENMFWYRQDPGLGLRLIYFSYDVKMKEK GDIPEGYSVSREKKERFSLILESASTNQTSMYLCASSPPHLASGASTDTQYFGPGTRLTVL EDLKNVFPPEVAVFEPSEAEISHTQKATLVCLATGFYPDHVELSWWVNGKEVHSGVSTD PQPLKEQPALNDSRYCLSSRLRVSATFWQNPRNHFRCQVQFYGLSENDEWTQDRAKPV TQIVSAEAWGRADCGFTSESYQQGVLSATILYEILLGKATLYAVLVSALVLMAMVKRK DSRG |
| 350 | nwTCR-0125 complete TRB (TCR β chain) nucleotide sequence | GATGTGAAAGTAACCCAGAGCTCGAGATATCTAGTCAAAAGGACGGGAGAGAAAG TTTTTCTGGAATGTGTCCAGGATATGGACCATGAAAATATGTTCTGGTATCGACAAG ACCCAGGTCTGGGGCTACGGCTGATCTATTTCTCATATGATGTTAAAATGAAAGAAA AAGGAGATATTCCTGAGGGGTACAGTGTCTCTAGAGAGAAGAAGGAGCGCTTCTCC CTGATTCTGGAGTCCGCCAGCACCAACCAGACATCTATGTACCTCTGTGCCAGCAGC CCGCCGCATTTAGCTAGCGGGGCGAGCACAGATACGCAGTATTTTGGCCCAGGCAC CCGGCTGACAGTGCTCGAGGACCTGAAAAACGTGTTCCCACCCGAGGTCGCTGTGTT TGAGCCATCAGAAGCAGAGATCTCCCACACCCAAAAGGCCACACTGGTGTGCCTGG CCACAGGCTTCTACCCCGACCACGTGGAGCTGAGCTGGTGGGTGAATGGGAAGGAG GTGCACAGTGGGGTCAGCACAGACCCGCAGCCCCTCAAGGAGCAGCCCGCCCTCAA TGACTCCAGATACTGCCTGTCCTCCCGCCTGAGGGTCTCGGCCACCTTCTGGCAGAA CCCCCGCAACCACTTCCGCTGTCAAGTCCAGTTCTACGGGCTCTCGGAGAATGACGA GTGGACCCAGGATAGGGCCAAACCTGTCACCCAGATCGTCAGCGCCGAGGCCTGGG GTAGAGCAGACTGTGGCTTCACCTCCGAGTCTTACCAGCAAGGGGTCCTGTCTGCCA CCATCCTCTATGAGATCTTGCTAGGGAAGGCCACCTTGTATGCCGTGCTGGTCAGTG CCCTCGTGCTGATGGCCATGGTCAAGAGAAAGGATTCCAGAGGC |
| 351 | nwTCR-0126 complete TRB (TCR β chain) amino acid sequence | DSGVTQTPKHLITATGQRVTLRCSPRSGDLSVYWYQQSLDQGLQFLIQYYNGEERAKG NILERFSAQQFPDLHSELNLSSLELGDSALYFCASSPRGGDSILDTQYFGPGTRLTVLEDL KNVFPPEVAVFEPSEAEISHTQKATLVCLATGFYPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSESYQQGVLSATILYEILLGKATLYAVLVSALVLMAMVKRKDSR G |
| 352 | nwTCR-0126 complete TRB (TCR β chain) nucleotide sequence | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGCC CGAGGGGGGGGGACTCTATTTTGGATACGCAGTATTTTGGCCCAGGCACCCGGCTG ACAGTGCTCGAGGACCTGAAAAACGTGTTCCCACCCGAGGTCGCTGTGTTTGAGCCA TCAGAAGCAGAGATCTCCCACACCCAAAAGGCCACACTGGTGTGCCTGGCCACAGG CTTCTACCCCGACCACGTGGAGCTGAGCTGGTGGGTGAATGGGAAGGAGGTGCACA GTGGGGTCAGCACAGACCCGCAGCCCCTCAAGGAGCAGCCCGCCCTCAATGACTCC AGATACTGCCTGTCCTCCCGCCTGAGGGTCTCGGCCACCTTCTGGCAGAACCCCCGC AACCACTTCCGCTGTCAAGTCCAGTTCTACGGGCTCTCGGAGAATGACGAGTGGACC CAGGATAGGGCCAAACCTGTCACCCAGATCGTCAGCGCCGAGGCCTGGGGTAGAGC AGACTGTGGCTTCACCTCCGAGTCTTACCAGCAAGGGGTCCTGTCTGCCACCATCCT CTATGAGATCTTGCTAGGGAAGGCCACCTTGTATGCCGTGCTGGTCAGTGCCCTCGT GCTGATGGCCATGGTCAAGAGAAAGGATTCCAGAGGC |
| 353 | nwTCR-0127 complete TRB (TCR β chain) amino acid sequence | EAQVTQNPRYLITVTGKKLTVTCSQNMNHEYMSWYRQDPGLGLRQIYYSMNVEVTDK GDVPEGYKVSRKEKRNFPLILESPSPNQTSLYFCASSTRDRDNEQFFGPGTRLTVLEDLK NVFPPEVAVFEPSEAEISHTQKATLVCLATGFYPDHVELSWWVNGKEVHSGVSTDPQPL KEQPALNDSRYCLSSRLRVSATFWQNPRNHFRCQVQFYGLSENDEWTQDRAKPVTQIV |
| | | SAEAWGRADCGFTSESYQQGVLSATILYEILLGKATLYAVLVSALVLMAMVKRKDSRG |
| 354 | nwTCR-0127 complete TRB (TCR β chain) nucleotide sequence | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGCAGCA CCCGGGACAGGGACAATGAGCAGTTCTTCGGGCCAGGGACACGGCTCACCGTGCTA GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 355 | nwTCR-1708 complete TRB (TCR β chain) amino acid sequence | DARVTQTPRDKVTEMGQEVTMRCQPILGHNTVFWYRQTMMQGLELLAYFRNRAPLDD SGMPKDRFSAEMPDATLATLKIQPSEPRDSAVYFCASGPEGLDNEQFFGPGTRLTVLEDL KNVFPPEVAVFEPSEAEISHTQKATLVCLATGFYPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSESYQQGVLSATILYEILLGKATLYAVLVSALVLMAMVKRKDSR G |
| 356 | nwTCR-1708 complete TRB (TCR β chain) nucleotide sequence | GATGCTAGAGTCACCCAGACACCAAGGGACAAGGTGACAGAGATGGGACAAGAAG TAACAATGAGATGTCAGCCAATTTTAGGCCACAATACTGTTTTCTGGTACAGACAGA CCATGATGCAAGGACTGGAGTTGCTGGCTTACTTCCGCAACCGGGCTCCTCTAGATG ATTCGGGGATGCCGAAGGATCGATTCTCAGCAGAGATGCCTGATGCAACTTTAGCC ACTCTGAAGATCCAGCCCTCAGAACCCAGGGACTCAGCTGTGTATTTTTGTGCTAGT GGTCCCGAGGGGTTGGACAATGAGCAGTTCTTCGGGCCAGGGACACGGCTCACCGT GCTAGAGGACCTGAAAAACGTGTTCCCACCCGAGGTCGCTGTGTTTGAGCCATCAG AAGCAGAGATCTCCCACACCCAAAAGGCCACACTGGTGTGCCTGGCCACAGGCTTC TACCCCGACCACGTGGAGCTGAGCTGGTGGGTGAATGGGAAGGAGGTGCACAGTGG GGTCAGCACAGACCCGCAGCCCCTCAAGGAGCAGCCCGCCCTCAATGACTCCAGAT ACTGCCTGTCCTCCCGCCTGAGGGTCTCGGCCACCTTCTGGCAGAACCCCCGCAACC ACTTCCGCTGTCAAGTCCAGTTCTACGGGCTCTCGGAGAATGACGAGTGGACCCAGG ATAGGGCCAAACCTGTCACCCAGATCGTCAGCGCCGAGGCCTGGGGTAGAGCAGAC TGTGGCTTCACCTCCGAGTCTTACCAGCAAGGGGTCCTGTCTGCCACCATCCTCTAT GAGATCTTGCTAGGGAAGGCCACCTTGTATGCCGTGCTGGTCAGTGCCCTCGTGCTG ATGGCCATGGTCAAGAGAAAGGATTCCAGAGGC |
| 357 | nwTCR-1862 complete TRB (TCR β chain) amino acid sequence | GAVVSQHPSWVICKSGTSVKIECRSLDFQATTMFWYRQFPKQSLMLMATSNEGSKATY EQGVEKDKFLINHASLTLSTLTVTSAHPEDSSFYICSARFANYYEQYFGPGTRLTVTEDL KNVFPPEVAVFEPSEAEISHTQKATLVCLATGFYPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSESYQQGVLSATILYEILLGKATLYAVLVSALVLMAMVKRKDSR G |
| 358 | nwTCR-1862 complete TRB (TCR β chain) nucleotide sequence | GGTGCTGTCGTCTCTCAACATCCGAGCTGGGTTATCTGTAAGAGTGGAACCTCTGTG AAGATCGAGTGCCGTTCCCTGGACTTTCAGGCCACAACTATGTTTTGGTATCGTCAG TTCCCGAAACAGAGTCTCATGCTGATGGCAACTTCCAATGAGGGCTCCAAGGCCAC ATACGAGCAAGGCGTCGAGAAGGACAAGTTTCTCATCAACCATGCAAGCCTGACCT TGTCCACTCTGACAGTGACCAGTGCCCATCCTGAAGACAGCAGCTTCTACATCTGCA GTGCTAGGTTTGCCAATTACTACGAGCAGTACTTCGGGCCGGGCACCAGGCTCACGG TCACAGAGGACCTGAAAAACGTGTTCCCACCCGAGGTCGCTGTGTTTGAGCCATCAG AAGCAGAGATCTCCCACACCCAAAAGGCCACACTGGTGTGCCTGGCCACAGGCTTC TACCCCGACCACGTGGAGCTGAGCTGGTGGGTGAATGGGAAGGAGGTGCACAGTGG GGTCAGCACAGACCCGCAGCCCCTCAAGGAGCAGCCCGCCCTCAATGACTCCAGAT ACTGCCTGTCCTCCCGCCTGAGGGTCTCGGCCACCTTCTGGCAGAACCCCCGCAACC ACTTCCGCTGTCAAGTCCAGTTCTACGGGCTCTCGGAGAATGACGAGTGGACCCAGG ATAGGGCCAAACCTGTCACCCAGATCGTCAGCGCCGAGGCCTGGGGTAGAGCAGAC TGTGGCTTCACCTCCGAGTCTTACCAGCAAGGGGTCCTGTCTGCCACCATCCTCTAT GAGATCTTGCTAGGGAAGGCCACCTTGTATGCCGTGCTGGTCAGTGCCCTCGTGCTG ATGGCCATGGTCAAGAGAAAGGATTCCAGAGGC |
| 359 | nwTCR-2162 complete TRB (TCR β chain) amino acid sequence | EADIYQTPRYLVIGTGKKITLECSQTMGHDKMYWYQQDPGMELHLIHYSYGVNSTEKG DLSSESTVSRIRTEHFPLTLESARPSHTSQYLCASSDPVHGDYGYTFGSGTRLTVVEDLN KVFPPEVAVFEPSEAEISHTQKATLVCLATGFFPDHVELSWWVNGKEVHSGVSTDPQPL KEQPALNDSRYCLSSRLRVSATFWQNPRNHFRCQVQFYGLSENDEWTQDRAKPVTQIV SAEAWGRADCGFTSVSYQQGVLSATILYEILLGKATLYAVLVSALVLMAMVKRKDF |
| 360 | nwTCR-2162 complete TRB (TCR β chain) nucleotide sequence | GAAGCTGACATCTACCAGACCCCAAGATACCTTGTTATAGGGACAGGAAAGAAGAT CACTCTGGAATGTTCTCAAACCATGGGCCATGACAAAATGTACTGGTATCAACAAG ATCCAGGAATGGAACTACACCTCATCCACTATTCCTATGGAGTTAATTCCACAGAGA AGGGAGATCTTTCCTCTGAGTCAACAGTCTCCAGAATAAGGACGGAGCATTTTCCCC TGACCCTGGAGTCTGCCAGGCCCTCACATACCTCTCAGTACCTCTGTGCCAGCAGTG ACCCAGTACACGGCGACTATGGCTACACCTTCGGTTCGGGGACCAGGTTAACCGTTG TAGAGGACCTGAACAAGGTGTTCCCACCCGAGGTCGCTGTGTTTGAGCCATCAGAA GCAGAGATCTCCCACACCCAAAAGGCCACACTGGTGTGCCTGGCCACAGGCTTCTTC CCTGACCACGTGGAGCTGAGCTGGTGGGTGAATGGGAAGGAGGTGCACAGTGGGGT CAGCACGGACCCGCAGCCCCTCAAGGAGCAGCCCGCCCTCAATGACTCCAGATACT GCCTGTCCTCCCGCCTGAGGGTCTCGGCCACCTTCTGGCAGAACCCCCGCAACCACT TCCGCTGTCAAGTCCAGTTCTACGGGCTCTCGGAGAATGACGAGTGGACCCAGGAT AGGGCCAAACCCGTCACCCAGATCGTCAGCGCCGAGGCCTGGGGTAGAGCAGACTG TGGCTTTACCTCGGTGTCCTACCAGCAAGGGGTCCTGTCTGCCACCATCCTCTATGA GATCCTGCTAGGGAAGGCCACCCTGTATGCTGTGCTGGTCAGCGCCCTTGTGTTGAT GGCCATGGTCAAGAGAAAGGATTTC |
| 361 | nwTCR-2241 complete TRB (TCR β chain) amino acid sequence | GEEVAQTPKHLVRGEGQKAKLYCAPIKGHSYVFWYQQVLKNEFKFLISFQNENVFDET GMPKERFSAKCLPNSPCSLEIQATKLEDSAVYFCASSPHHRYEQYFGPGTRLTVTEDLKN VFPPEVAVFEPSEAEISHTQKATLVCLATGFYPDHVELSWWVNGKEVHSGVSTDPQPLK EQPALNDSRYCLSSRLRVSATFWQNPRNHFRCQVQFYGLSENDEWTQDRAKPVTQIVS AEAWGRADCGFTSESYQQGVLSATILYEILLGKATLYAVLVSALVLMAMVKRKDSRG |
| 362 | nwTCR-2241 complete TRB (TCR β chain) nucleotide sequence | GGTGAAGAAGTCGCCCAGACTCCAAAACATCTTGTCAGAGGGGAAGGACAGAAAG CAAAATTATATTGTGCCCCAATAAAAGGACACAGTTATGTTTTTTGGTACCAACAGG TCCTGAAAAACGAGTTCAAGTTCTTGATTTCCTTCCAGAATGAAAATGTCTTTGATG AAACAGGTATGCCCAAGGAAAGATTTTCAGCTAAGTGCCTCCCAAATTCACCCTGTA GCCTTGAGATCCAGGCTACGAAGCTTGAGGATTCAGCAGTGTATTTTTGTGCCAGCA GCCCACACCACAGGTACGAGCAGTACTTCGGGCCGGGCACCAGGCTCACGGTCACA GAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCC CGACCACGTGGAGCTGAGCTGGTGGGTGAATGGGAAGGAGGTGCACAGTGGGGTCA GCACAGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCTGTCACCCAGATCGTCAGCGCCGAGGCCTGGGGTAGAGCAGACTGTG GCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGG CCATGGTCAAGAGAAAGGATTCCAGAGGC |
| 363 | nwTCR-2308 complete TRB (TCR β chain) amino acid sequence | EAQVTQNPRYLITVTGKKLTVTCSQNMNHEYMSWYRQDPGLGLRQIYYSMNVEVTDK GDVPEGYKVSRKEKRNFPLILESPSPNQTSLYFCASSTSPYNEQFFGPGTRLTVLEDLKNV FPPEVAVFEPSEAEISHTQKATLVCLATGFYPDHVELSWWVNGKEVHSGVSTDPQPLKE QPALNDSRYCLSSRLRVSATFWQNPRNHFRCQVQFYGLSENDEWTQDRAKPVTQIVSA EAWGRADCGFTSESYQQGVLSATILYEILLGKATLYAVLVSALVLMAMVKRKDSRG |
| 364 | nwTCR-2308 complete TRB (TCR β chain) nucleotide sequence | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGCTCGA CTAGCCCGTACAATGAGCAGTTCTTCGGGCCAGGGACACGGCTCACCGTGCTAGAG GACCTGAAAAACGTGTTCCCACCCGAGGTCGCTGTGTTTGAGCCATCAGAAGCAGA GATCTCCCACACCCAAAAGGCCACACTGGTGTGCCTGGCCACAGGCTTCTACCCCGA CCACGTGGAGCTGAGCTGGTGGGTGAATGGGAAGGAGGTGCACAGTGGGGTCAGCA CAGACCCGCAGCCCCTCAAGGAGCAGCCCGCCCTCAATGACTCCAGATACTGCCTGT CCTCCCGCCTGAGGGTCTCGGCCACCTTCTGGCAGAACCCCCGCAACCACTTCCGCT GTCAAGTCCAGTTCTACGGGCTCTCGGAGAATGACGAGTGGACCCAGGATAGGGCC AAACCTGTCACCCAGATCGTCAGCGCCGAGGCCTGGGGTAGAGCAGACTGTGGCTT CACCTCCGAGTCTTACCAGCAAGGGGTCCTGTCTGCCACCATCCTCTATGAGATCTT GCTAGGGAAGGCCACCTTGTATGCCGTGCTGGTCAGTGCCCTCGTGCTGATGGCCAT GGTCAAGAGAAAGGATTCCAGAGGC |
| 365 | nwTCR-2310 complete TRB (TCR β chain) amino acid sequence | DSGVTQTPKHLITATGQRVTLRCSPRSGDLSVYWYQQSLDQGLQFLIQYYNGEERAKG NILERFSAQQFPDLHSELNLSSLELGDSALYFCASSLHRDSDTEAFFGQGTRLTVVEDLN KVFPPEVAVFEPSEAEISHTQKATLVCLATGFFPDHVELSWWVNGKEVHSGVSTDPQPL KEQPALNDSRYCLSSRLRVSATFWQNPRNHFRCQVQFYGLSENDEWTQDRAKPVTQIV SAEAWGRADCGFTSVSYQQGVLSATILYEILLGKATLYAVLVSALVLMAMVKRKDF |
| 366 | nwTCR-2310 complete TRB (TCR β chain) nucleotide sequence | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA |
| | | AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGCC TACACCGGGACAGTGACACTGAAGCTTTCTTTGGACAAGGCACCAGACTCACAGTT GTAGAGGACCTGAACAAGGTGTTCCCACCCGAGGTCGCTGTGTTTGAGCCATCAGA AGCAGAGATCTCCCACACCCAAAAGGCCACACTGGTGTGCCTGGCCACAGGCTTCTT CCCTGACCACGTGGAGCTGAGCTGGTGGGTGAATGGGAAGGAGGTGCACAGTGGGG TCAGCACGGACCCGCAGCCCCTCAAGGAGCAGCCCGCCCTCAATGACTCCAGATAC TGCCTGTCCTCCCGCCTGAGGGTCTCGGCCACCTTCTGGCAGAACCCCCGCAACCAC TTCCGCTGTCAAGTCCAGTTCTACGGGCTCTCGGAGAATGACGAGTGGACCCAGGAT AGGGCCAAACCCGTCACCCAGATCGTCAGCGCCGAGGCCTGGGGTAGAGCAGACTG TGGCTTTACCTCGGTGTCCTACCAGCAAGGGGTCCTGTCTGCCACCATCCTCTATGA GATCCTGCTAGGGAAGGCCACCCTGTATGCTGTGCTGGTCAGCGCCCTTGTGTTGAT GGCCATGGTCAAGAGAAAGGATTTC |
| 367 | nwTCR-2390 complete TRB (TCR β chain) amino acid sequence | DSGVTQTPKHLITATGQRVTLRCSPRSGDLSVYWYQQSLDQGLQFLIQYYNGEERAKG NILERFSAQQFPDLHSELNLSSLELGDSALYFCASSHGRDSTDTQYFGPGTRLTVLEDLK NVFPPEVAVFEPSEAEISHTQKATLVCLATGFYPDHVELSWWVNGKEVHSGVSTDPQPL KEQPALNDSRYCLSSRLRVSATFWQNPRNHFRCQVQFYGLSENDEWTQDRAKPVTQIV SAEAWGRADCGFTSESYQQGVLSATILYEILLGKATLYAVLVSALVLMAMVKRKDSRG |
| 368 | nwTCR-2390 complete TRB (TCR β chain) nucleotide sequence | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGCC ACGGGAGGGATAGCACAGATACGCAGTATTTTGGCCCAGGCACCCGGCTGACAGTG CTCGAGGACCTGAAAAACGTGTTCCCACCCGAGGTCGCTGTGTTTGAGCCATCAGAA GCAGAGATCTCCCACACCCAAAAGGCCACACTGGTGTGCCTGGCCACAGGCTTCTA CCCCGACCACGTGGAGCTGAGCTGGTGGGTGAATGGGAAGGAGGTGCACAGTGGGG TCAGCACAGACCCGCAGCCCCTCAAGGAGCAGCCCGCCCTCAATGACTCCAGATAC TGCCTGTCCTCCCGCCTGAGGGTCTCGGCCACCTTCTGGCAGAACCCCCGCAACCAC TTCCGCTGTCAAGTCCAGTTCTACGGGCTCTCGGAGAATGACGAGTGGACCCAGGAT AGGGCCAAACCTGTCACCCAGATCGTCAGCGCCGAGGCCTGGGGTAGAGCAGACTG TGGCTTCACCTCCGAGTCTTACCAGCAAGGGGTCCTGTCTGCCACCATCCTCTATGA GATCTTGCTAGGGAAGGCCACCTTGTATGCCGTGCTGGTCAGTGCCCTCGTGCTGAT GGCCATGGTCAAGAGAAAGGATTCCAGAGGC |
| 369 | nwTCR-2392 complete TRB (TCR β chain) amino acid sequence | GAGVSQSPSNKVTEKGKDVELRCDPISGHTALYWYRQRLGQGLEFLIYFQGNSAPDKSG LPSDRFSAERTGESVSTLTIQRTQQEDSAVYLCASSPGGNYGYTFGSGTRLTVVEDLNKV FPPEVAVFEPSEAEISHTQKATLVCLATGFFPDHVELSWWVNGKEVHSGVSTDPQPLKE QPALNDSRYCLSSRLRVSATFWQNPRNHFRCQVQFYGLSENDEWTQDRAKPVTQIVSA EAWGRADCGFTSVSYQQGVLSATILYEILLGKATLYAVLVSALVLMAMVKRKDF |
| 370 | nwTCR-2392 complete TRB (TCR β chain) nucleotide sequence | GGAGCTGGAGTCTCCCAGTCCCCCAGTAACAAGGTCACAGAGAAGGGAAAGGATGT AGAGCTCAGGTGTGATCCAATTTCAGGTCATACTGCCCTTTACTGGTACCGACAGAG GCTGGGGCAGGGCCTGGAGTTTTTAATTTACTTCCAAGGCAACAGTGCACCAGACA AATCAGGGCTGCCCAGTGATCGCTTCTCTGCAGAGAGGACTGGGGAATCCGTCTCCA CTCTGACGATCCAGCGCACACAGCAGGAGGACTCGGCCGTGTATCTCTGTGCCAGCT CCCCGGGGGGGAACTATGGCTACACCTTCGGTTCGGGGACCAGGTTAACCGTTGTA GAGGACCTGAACAAGGTGTTCCCACCCGAGGTCGCTGTGTTTGAGCCATCAGAAGC AGAGATCTCCCACACCCAAAAGGCCACACTGGTGTGCCTGGCCACAGGCTTCTTCCC TGACCACGTGGAGCTGAGCTGGTGGGTGAATGGGAAGGAGGTGCACAGTGGGGTCA GCACGGACCCGCAGCCCCTCAAGGAGCAGCCCGCCCTCAATGACTCCAGATACTGC CTGTCCTCCCGCCTGAGGGTCTCGGCCACCTTCTGGCAGAACCCCCGCAACCACTTC CGCTGTCAAGTCCAGTTCTACGGGCTCTCGGAGAATGACGAGTGGACCCAGGATAG GGCCAAACCCGTCACCCAGATCGTCAGCGCCGAGGCCTGGGGTAGAGCAGACTGTG GCTTTACCTCGGTGTCCTACCAGCAAGGGGTCCTGTCTGCCACCATCCTCTATGAGA TCCTGCTAGGGAAGGCCACCCTGTATGCTGTGCTGGTCAGCGCCCTTGTGTTGATGG CCATGGTCAAGAGAAAGGATTTC |
| 371 | nwTCR-2424 complete TRB (TCR β chain) amino acid sequence | DSGVTQTPKHLITATGQRVTLRCSPRSGDLSVYWYQQSLDQGLQFLIQYYNGEERAKG NILERFSAQQFPDLHSELNLSSLELGDSALYFCASSLGDSEQFFGPGTRLTVLEDLKNVFP PEVAVFEPSEAEISHTQKATLVCLATGFYPDHVELSWWVNGKEVHSGVSTDPQPLKEQP ALNDSRYCLSSRLRVSATFWQNPRNHFRCQVQFYGLSENDEWTQDRAKPVTQIVSAEA WGRADCGFTSESYQQGVLSATILYEILLGKATLYAVLVSALVLMAMVKRKDSRG |
| 372 | nwTCR-2424 complete TRB (TCR β chain) nucleotide sequence | GATTCTGGAGTCACACAAACCCCAAAGCACCTGATCACAGCAACTGGACAGCGAGT GACGCTGAGATGCTCCCCTAGGTCTGGAGACCTCTCTGTGTACTGGTACCAACAGAG CCTGGACCAGGGCCTCCAGTTCCTCATTCAGTATTATAATGGAGAAGAGAGAGCAA AAGGAAACATTCTTGAACGATTCTCCGCACAACAGTTCCCTGACTTGCACTCTGAAC TAAACCTGAGCTCTCTGGAGCTGGGGGACTCAGCTTTGTATTTCTGTGCCAGCAGCC TCGGGGACAGCGAGCAGTTCTTCGGGCCAGGGACACGGCTCACCGTGCTAGAGGAC |
| | | CTGAAAAACGTGTTCCCACCCGAGGTCGCTGTGTTTGAGCCATCAGAAGCAGAGAT CTCCCACACCCAAAAGGCCACACTGGTGTGCCTGGCCACAGGCTTCTACCCCGACCA CGTGGAGCTGAGCTGGTGGGTGAATGGGAAGGAGGTGCACAGTGGGGTCAGCACA GACCCGCAGCCCCTCAAGGAGCAGCCCGCCCTCAATGACTCCAGATACTGCCTGTCC TCCCGCCTGAGGGTCTCGGCCACCTTCTGGCAGAACCCCCGCAACCACTTCCGCTGT CAAGTCCAGTTCTACGGGCTCTCGGAGAATGACGAGTGGACCCAGGATAGGGCCAA ACCTGTCACCCAGATCGTCAGCGCCGAGGCCTGGGGTAGAGCAGACTGTGGCTTCA CCTCCGAGTCTTACCAGCAAGGGGTCCTGTCTGCCACCATCCTCTATGAGATCTTGC TAGGGAAGGCCACCTTGTATGCCGTGCTGGTCAGTGCCCTCGTGCTGATGGCCATGG TCAAGAGAAAGGATTCCAGAGGC |
| 373 | nwTCR-2561 complete TRB (TCR β chain) amino acid sequence | GAGVSQSPSNKVTEKGKDVELRCDPISGHTALYWYRQSLGQGLEFLIYFQGNSAPDKSG LPSDRFSAERTGGSVSTLTIQRTQQEDSAVYLCASSFGTSGDQYEQYFGPGTRLTVTEDL KNVFPPEVAVFEPSEAEISHTQKATLVCLATGFYPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSESYQQGVLSATILYEILLGKATLYAVLVSALVLMAMVKRKDSR G |
| 374 | nwTCR-2561 complete TRB (TCR β chain) nucleotide sequence | GGAGCTGGAGTCTCCCAGTCCCCCAGTAACAAGGTCACAGAGAAGGGAAAGGATGT AGAGCTCAGGTGTGATCCAATTTCAGGTCATACTGCCCTTTACTGGTACCGACAGAG CCTGGGGCAGGGCCTGGAGTTTTTAATTTACTTCCAAGGCAACAGTGCACCAGACAA ATCAGGGCTGCCCAGTGATCGCTTCTCTGCAGAGAGGACTGGGGGATCCGTCTCCAC TCTGACGATCCAGCGCACACAGCAGGAGGACTCGGCCGTGTATCTCTGTGCCAGCA GCTTTGGGACTAGCGGGGACCAATACGAGCAGTACTTCGGGCCGGGCACCAGGCTC ACGGTCACAGAGGACCTGAAAAACGTGTTCCCACCCGAGGTCGCTGTGTTTGAGCC ATCAGAAGCAGAGATCTCCCACACCCAAAAGGCCACACTGGTGTGCCTGGCCACAG GCTTCTACCCCGACCACGTGGAGCTGAGCTGGTGGGTGAATGGGAAGGAGGTGCAC AGTGGGGTCAGCACAGACCCGCAGCCCCTCAAGGAGCAGCCCGCCCTCAATGACTC CAGATACTGCCTGTCCTCCCGCCTGAGGGTCTCGGCCACCTTCTGGCAGAACCCCCG CAACCACTTCCGCTGTCAAGTCCAGTTCTACGGGCTCTCGGAGAATGACGAGTGGAC CCAGGATAGGGCCAAACCTGTCACCCAGATCGTCAGCGCCGAGGCCTGGGGTAGAG CAGACTGTGGCTTCACCTCCGAGTCTTACCAGCAAGGGGTCCTGTCTGCCACCATCC TCTATGAGATCTTGCTAGGGAAGGCCACCTTGTATGCCGTGCTGGTCAGTGCCCTCG TGCTGATGGCCATGGTCAAGAGAAAGGATTCCAGAGGC |
| 375 | nwTCR-2563 complete TRB (TCR β chain) amino acid sequence | ETGVTQSPTHLIKTRGQQVTLRCSSQSGHNTVSWYQQALGQGPQFIFQYYREEENGRGN FPPRFSGLQFPNYSSELNVNALELDDSALYLCASSLQTGDEQYFGPGTRLTVTEDLKNVF PPEVAVFEPSEAEISHTQKATLVCLATGFYPDHVELSWWVNGKEVHSGVSTDPQPLKEQ PALNDSRYCLSSRLRVSATFWQNPRNHFRCQVQFYGLSENDEWTQDRAKPVTQIVSAE AWGRADCGFTSESYQQGVLSATILYEILLGKATLYAVLVSALVLMAMVKRKDSRG |
| 376 | nwTCR-2563 complete TRB (TCR β chain) nucleotide sequence | GAGACTGGAGTCACCCAAAGTCCCACACACCTGATCAAAACGAGAGGACAGCAAGT GACTCTGAGATGCTCTTCTCAGTCTGGGCACAACACTGTGTCCTGGTACCAACAGGC CCTGGGTCAGGGGCCCCAGTTTATCTTTCAGTATTATAGGGAGGAAGAGAATGGCA GAGGAAACTTCCCTCCTAGATTCTCAGGTCTCCAGTTCCCTAATTATAGCTCTGAGCT GAATGTGAACGCCTTGGAGCTGGACGACTCGGCCCTGTATCTCTGTGCCAGCAGCCT GCAGACAGGGGACGAGCAGTACTTCGGGCCGGGCACCAGGCTCACGGTCACAGAG GACCTGAAAAACGTGTTCCCACCCGAGGTCGCTGTGTTTGAGCCATCAGAAGCAGA GATCTCCCACACCCAAAAGGCCACACTGGTGTGCCTGGCCACAGGCTTCTACCCCGA CCACGTGGAGCTGAGCTGGTGGGTGAATGGGAAGGAGGTGCACAGTGGGGTCAGCA CAGACCCGCAGCCCCTCAAGGAGCAGCCCGCCCTCAATGACTCCAGATACTGCCTGT CCTCCCGCCTGAGGGTCTCGGCCACCTTCTGGCAGAACCCCCGCAACCACTTCCGCT GTCAAGTCCAGTTCTACGGGCTCTCGGAGAATGACGAGTGGACCCAGGATAGGGCC AAACCTGTCACCCAGATCGTCAGCGCCGAGGCCTGGGGTAGAGCAGACTGTGGCTT CACCTCCGAGTCTTACCAGCAAGGGGTCCTGTCTGCCACCATCCTCTATGAGATCTT GCTAGGGAAGGCCACCTTGTATGCCGTGCTGGTCAGTGCCCTCGTGCTGATGGCCAT GGTCAAGAGAAAGGATTCCAGAGGC |
| 377 | nwTCR-2595 complete TRB (TCR β chain) amino acid sequence | EAQVTQNPRYLITVTGKKLTVTCSQNMNHEYMSWYRQDPGLGLRQIYYSMNVEVTDK GDVPEGYKVSRKEKRNFPLILESPSPNQTSLYFCASSSGTGGGAEAFFGQGTRLTVVEDL NKVFPPEVAVFEPSEAEISHTQKATLVCLATGFFPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSVSYQQGVLSATILYEILLGKATLYAVLVSALVLMAMVKRKDF |
| 378 | nwTCR-2595 complete TRB (TCR β chain) nucleotide sequence | GAAGCCCAAGTGACCCAGAACCCAAGATACCTCATCACAGTGACTGGAAAGAAGTT AACAGTGACTTGTTCTCAGAATATGAACCATGAGTATATGTCCTGGTATCGACAAGA CCCAGGGCTGGGCTTAAGGCAGATCTACTATTCAATGAATGTTGAGGTGACTGATAA GGGAGATGTTCCTGAAGGGTACAAAGTCTCTCGAAAAGAGAAGAGGAATTTCCCCC TGATCCTGGAGTCGCCCAGCCCCAACCAGACCTCTCTGTACTTCTGTGCCAGCAGTT CCGGGACAGGGGGTGGGGCTGAAGCTTTCTTTGGACAAGGCACCAGACTCACAGTT GTAGAGGACCTGAACAAGGTGTTCCCACCCGAGGTCGCTGTGTTTGAGCCATCAGA AGCAGAGATCTCCCACACCCAAAAGGCCACACTGGTGTGCCTGGCCACAGGCTTCTT |
| | | CCCTGACCACGTGGAGCTGAGCTGGTGGGTGAATGGGAAGGAGGTGCACAGTGGGG TCAGCACGGACCCGCAGCCCCTCAAGGAGCAGCCCGCCCTCAATGACTCCAGATAC TGCCTGTCCTCCCGCCTGAGGGTCTCGGCCACCTTCTGGCAGAACCCCCGCAACCAC TTCCGCTGTCAAGTCCAGTTCTACGGGCTCTCGGAGAATGACGAGTGGACCCAGGAT AGGGCCAAACCCGTCACCCAGATCGTCAGCGCCGAGGCCTGGGGTAGAGCAGACTG TGGCTTTACCTCGGTGTCCTACCAGCAAGGGGTCCTGTCTGCCACCATCCTCTATGA GATCCTGCTAGGGAAGGCCACCCTGTATGCTGTGCTGGTCAGCGCCCTTGTGTTGAT GGCCATGGTCAAGAGAAAGGATTTC |
| 379 | nwTCR-2629 complete TRB (TCR β chain) amino acid sequence | GAVVSQHPSWVICKSGTSVKIECRSLDFQATTMFWYRQFPKQSLMLMATSNEGSKATY EQGVEKDKFLINHASLTLSTLTVTSAHPEDSSFYICSANRDFGIYEQYFGPGTRLTVTEDL KNVFPPEVAVFEPSEAEISHTQKATLVCLATGFYPDHVELSWWVNGKEVHSGVSTDPQP LKEQPALNDSRYCLSSRLRVSATFWQNPRNHFRCQVQFYGLSENDEWTQDRAKPVTQI VSAEAWGRADCGFTSESYQQGVLSATILYEILLGKATLYAVLVSALVLMAMVKRKDSR G |
| 380 | nwTCR-2629 complete TRB (TCR β chain) nucleotide sequence | GGTGCTGTCGTCTCTCAACATCCGAGCTGGGTTATCTGTAAGAGTGGAACCTCTGTG AAGATCGAGTGCCGTTCCCTGGACTTTCAGGCCACAACTATGTTTTGGTATCGTCAG TTCCCGAAACAGAGTCTCATGCTGATGGCAACTTCCAATGAGGGCTCCAAGGCCAC ATACGAGCAAGGCGTCGAGAAGGACAAGTTTCTCATCAACCATGCAAGCCTGACCT TGTCCACTCTGACAGTGACCAGTGCCCATCCTGAAGACAGCAGCTTCTACATCTGCA GTGCGAACCGGGACTTCGGGATCTACGAGCAGTACTTCGGGCCGGGCACCAGGCTC ACGGTCACAGAGGACCTGAAAAACGTGTTCCCACCCGAGGTCGCTGTGTTTGAGCC ATCAGAAGCAGAGATCTCCCACACCCAAAAGGCCACACTGGTGTGCCTGGCCACAG GCTTCTACCCCGACCACGTGGAGCTGAGCTGGTGGGTGAATGGGAAGGAGGTGCAC AGTGGGGTCAGCACAGACCCGCAGCCCCTCAAGGAGCAGCCCGCCCTCAATGACTC CAGATACTGCCTGTCCTCCCGCCTGAGGGTCTCGGCCACCTTCTGGCAGAACCCCCG CAACCACTTCCGCTGTCAAGTCCAGTTCTACGGGCTCTCGGAGAATGACGAGTGGAC CCAGGATAGGGCCAAACCTGTCACCCAGATCGTCAGCGCCGAGGCCTGGGGTAGAG CAGACTGTGGCTTCACCTCCGAGTCTTACCAGCAAGGGGTCCTGTCTGCCACCATCC TCTATGAGATCTTGCTAGGGAAGGCCACCTTGTATGCCGTGCTGGTCAGTGCCCTCG TGCTGATGGCCATGGTCAAGAGAAAGGATTCCAGAGGC |
| 381 | Amino acid sequence from position 7 to 16 of KRAS, with the 12th amino acid mutated from G to V | VVVGAVGVGK |
| 382 | Amino acid sequence from position 8 to 16 of KRAS, with the 12th amino acid mutated from G to V | VVGAVGVGK |
| 383 | gRNA002 recognition sequence | TCAGGGTTCTGGATATCTGT |
| 384 | gRNA004 recognition sequence | CTGCCTGAGCAGCCGCCTGA |
| 385 | PAM sequence | GGG |
| 386 | Expression construct of nwTCR-0126 TCR sequence | CCATATTCTGGCAGGGTCAGTGGCTCCAACTAACATTTGTTTGGTACTTTACAGTTTA TTAAATAGATGTTTATATGGAGAAGCTCTCATTTCTTTCTCAGAAGAGCCTGGCTAG GAAGGTGGATGAGGCACCATATTCATTTTGCAGGTGAAATTCCTGAGATGTAAGGA GCTGCTGTGACTTGCTCAAGGCCTTATATCGAGTAAACGGTAGTGCTGGGGCTTAGA CGCAGGTGTTCTGATTTATAGTTCAAAACCTCTATCAATGAGAGAGCAATCTCCTGG TAATGTGATAGATTTCCCAACTTAATGCCAACATACCATAAACCTCCCATTCTGCTA ATGCCCAGCCTAAGTTGGGGAGACCACTCCAGATTCCAAGATGTACAGTTTGCTTTG CTGGGCCTTTTTCCCATGCCTGCCTTTACTCTGCCAGAGTTATATTGCTGGGGTTTTG AAGAAGATCCTATTAAATAAAAGAATAAGCAGTATTATTAAGTAGCCCTGCATTTCA GGTTTCCTTGAGTGGCAGGCCAGGCCTGGCCGTGAACGTTCACTGAAATCATGGCCT CTTGGCCAAGATTGATAGCTTGTGCCTGTCCCTGAGTCCCAGTCCATCACGAGCAGC TGGTTTCTAAGATGCTATTTCCCGTATAAAGCATGAGACCGTGACTTGCCAGCCCCA CAGAGCCCCGCCCTTGTCCATCACTGGCATCTGGACTCCAGCCTGGGTTGGGGCAAA GAGGGAAATGAGATCATGTCCTAACCCTGATCCTCTTGTCCCACAGATGGCAGCGGC GCCACCAACTTCAGCCTGCTGAAGCAGGCCGGCGACGTGGAGGAGAACCCCGGCCC |
| | | CATGGCCACCGGCAGCAGGACCAGCCTGCTGCTGGCCTTCGGCCTGCTGTGCCTGCC CTGGCTGCAGGAGGGCAGCGCCGATTCTGGAGTCACACAAACCCCAAAGCACCTGA TCACAGCAACTGGACAGCGAGTGACGCTGAGATGCTCCCCTAGGTCTGGAGACCTC TCTGTGTACTGGTACCAACAGAGCCTGGACCAGGGCCTCCAGTTCCTCATTCAGTAT TATAATGGAGAAGAGAGAGCAAAAGGAAACATTCTTGAACGATTCTCCGCACAACA GTTCCCTGACTTGCACTCTGAACTAAACCTGAGCTCTCTGGAGCTGGGGGACTCAGC TTTGTATTTCTGTGCCAGCAGCCCGAGGGGGGGGGACTCTATTTTGGATACGCAGTA TTTTGGCCCAGGCACCCGGCTGACAGTGCTCGAGGACCTGAAAAACGTGTTCCCACC CGAGGTCGCTGTGTTTGAGCCATCAGAAGCAGAGATCTCCCACACCCAAAAGGCCA CACTGGTGTGCCTGGCCACAGGCTTCTACCCCGACCACGTGGAGCTGAGCTGGTGGG TGAATGGGAAGGAGGTGCACAGTGGGGTCAGCACAGACCCGCAGCCCCTCAAGGA GCAGCCCGCCCTCAATGACTCCAGATACTGCCTGTCCTCCCGCCTGAGGGTCTCGGC CACCTTCTGGCAGAACCCCCGCAACCACTTCCGCTGTCAAGTCCAGTTCTACGGGCT CTCGGAGAATGACGAGTGGACCCAGGATAGGGCCAAACCTGTCACCCAGATCGTCA GCGCCGAGGCCTGGGGTAGAGCAGACTGTGGCTTCACCTCCGAGTCTTACCAGCAA GGGGTCCTGTCTGCCACCATCCTCTATGAGATCTTGCTAGGGAAGGCCACCTTGTAT GCCGTGCTGGTCAGTGCCCTCGTGCTGATGGCCATGGTCAAGAGAAAGGATTCCAG AGGCCGGGCCAAGCGGGGATCCGGAGCCACAAATTTCTCCCTGCTGAAACAGGCCG GCGATGTGGAGGAGAATCCTGGACCTATGGCCACCGGCAGCAGGACCAGCCTGCTG CTGGCCTTCGGCCTGCTGTGCCTGCCCTGGCTGCAGGAGGGCAGCGCCGCTCAGAAG GTAACTCAAGCGCAGACTGAAATTTCTGTGGTGGAGAAGGAGGATGTGACCTTGGA CTGTGTGTATGAAACCCGTGATACTACTTATTACTTATTCTGGTACAAGCAACCACC AAGTGGAGAATTGGTTTTCCTTATTCGTCGGAACTCTTTTGATGAGCAAAATGAAAT AAGTGGTCGGTATTCTTGGAACTTCCAGAAATCCACCAGTTCCTTCAACTTCACCAT CACAGCCTCACAAGTCGTGGACTCAGCAGTATACTTCTGTGCTCTGAGTGACCCCCG TTATTCAGGAGGAGGTGCTGACGGACTCACCTTTGGCAAAGGGACTCATCTAATCAT CCAGCCCTATATCCAGAACCCTGACCCTGCCGTGTACCAGCTGAGAGACTCTAAATC CAGTGACAAGTCTGTCTGCCTATTCACCGATTTTGATTCTCAAACAAATGTGTCACA AAGTAAGGATTCTGATGTGTATATCACAGACAAAACTGTGCTAGACATGAGGTCTAT GGACTTCAAGAGCAACAGTGCTGTGGCCTGGAGCAACAAATCTGACTTTGCATGTG CAAACGCCTTCAACAACAGCATTATTCCAGAAGACACCTTCTTCCCCAGCCCAGGTA AGGGCAGCTTTGGTGCCTTCGCAGGCTGTTTCCTTGCTTCAGGAATGGCCAGGTTCT GCCCAGAGCTCTGGTCAATGATGTCTAAAACTCCTCTGATTGGTGGTCTCGGCCTTA TCCATTGCCACCAAAACCCTCTTTTTACTAAGAAACAGTGAGCCTTGTTCTGGCAGT CCAGAGAATGACACGGGAAAAAAGCAGATGAAGAGAAGGTGGCAGGAGAGGGCAC GTGGCCCAGCCTCAGTCTCTCCAACTGAGTTCCTGCCTGCCTGCCTTTGCTCAGACTG TTTGCCCCTTACTGCTCTTCTAGGCCTCATTCTAAGCCCCTTCTCCAAGTTGCCTCTCC TTATTTCTCCCTGTCTGCCAAAAAATCTTTCCCAGCTCACTAAGTCAGTCTCACGCAG TCACTCATTAACCCACCAATCACTGATTGTGCCGGCACATGAATGCACCAGGTGTTG AAGTGGAGGAATTAAAAAGTCAGATGAGGGGTGTGCCCAGAGGAAGCACCATTCTA GTTGGGGGAGCC |
| 387 | Nucleotide sequence of 5'HA | CCATATTCTGGCAGGGTCAGTGGCTCCAACTAACATTTGTTTGGTACTTTACAGTTTA TTAAATAGATGTTTATATGGAGAAGCTCTCATTTCTTTCTCAGAAGAGCCTGGCTAG GAAGGTGGATGAGGCACCATATTCATTTTGCAGGTGAAATTCCTGAGATGTAAGGA GCTGCTGTGACTTGCTCAAGGCCTTATATCGAGTAAACGGTAGTGCTGGGGCTTAGA CGCAGGTGTTCTGATTTATAGTTCAAAACCTCTATCAATGAGAGAGCAATCTCCTGG TAATGTGATAGATTTCCCAACTTAATGCCAACATACCATAAACCTCCCATTCTGCTA ATGCCCAGCCTAAGTTGGGGAGACCACTCCAGATTCCAAGATGTACAGTTTGCTTTG CTGGGCCTTTTTCCCATGCCTGCCTTTACTCTGCCAGAGTTATATTGCTGGGGTTTTG AAGAAGATCCTATTAAATAAAAGAATAAGCAGTATTATTAAGTAGCCCTGCATTTCA GGTTTCCTTGAGTGGCAGGCCAGGCCTGGCCGTGAACGTTCACTGAAATCATGGCCT CTTGGCCAAGATTGATAGCTTGTGCCTGTCCCTGAGTCCCAGTCCATCACGAGCAGC TGGTTTCTAAGATGCTATTTCCCGTATAAAGCATGAGACCGTGACTTGCCAGCCCCA CAGAGCCCCGCCCTTGTCCATCACTGGCATCTGGACTCCAGCCTGGGTTGGGGCAAA GAGGGAAATGAGATCATGTCCTAACCCTGATCCTCTTGTCCCACAGAT |
| 388 | Nucleotide sequence of 2A | GGCAGCGGCGCCACCAACTTCAGCCTGCTGAAGCAGGCCGGCGACGTGGAGGAGAA CCCCGGCCCC |
| 389 | Nucleotide sequence of SP (signal peptide) | ATGGCCACCGGCAGCAGGACCAGCCTGCTGCTGGCCTTCGGCCTGCTGTGCCTGCCC TGGCTGCAGGAGGGCAGCGCC |
| 390 | Nucleotide sequence of 2A variant | CGGGCCAAGCGGGGATCCGGAGCCACAAATTTCTCCCTGCTGAAACAGGCCGGCGA TGTGGAGGAGAATCCTGGACCT |
| 391 | Nucleotide sequence of 3'HA | ATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGA TTCTGATGTGTATATCACAGACAAAACTGTGCTAGACATGAGGTCTATGGACTTCAA |
| | | GAGCAACAGTGCTGTGGCCTGGAGCAACAAATCTGACTTTGCATGTGCAAACGCCTT CAACAACAGCATTATTCCAGAAGACACCTTCTTCCCCAGCCCAGGTAAGGGCAGCTT TGGTGCCTTCGCAGGCTGTTTCCTTGCTTCAGGAATGGCCAGGTTCTGCCCAGAGCT CTGGTCAATGATGTCTAAAACTCCTCTGATTGGTGGTCTCGGCCTTATCCATTGCCAC CAAAACCCTCTTTTTACTAAGAAACAGTGAGCCTTGTTCTGGCAGTCCAGAGAATGA CACGGGAAAAAAGCAGATGAAGAGAAGGTGGCAGGAGAGGGCACGTGGCCCAGCC TCAGTCTCTCCAACTGAGTTCCTGCCTGCCTGCCTTTGCTCAGACTGTTTGCCCCTTA CTGCTCTTCTAGGCCTCATTCTAAGCCCCTTCTCCAAGTTGCCTCTCCTTATTTCTCCC TGTCTGCCAAAAAATCTTTCCCAGCTCACTAAGTCAGTCTCACGCAGTCACTCATTA ACCCACCAATCACTGATTGTGCCGGCACATGAATGCACCAGGTGTTGAAGTGGAGG AATTAAAAAGTCAGATGAGGGGTGTGCCCAGAGGAAGCACCATTCTAGTTGGGGGA GCC |
| 392 | Knock-in (KI) amino acid sequence of nwTCR-1708 | GSGATNFSLLKQAGDVEENPGPMATGSRTSLLLAFGLLCLPWLQEGSADARVTQTPRD KVTEMGQEVTMRCQPILGHNTVFWYRQTMMQGLELLAYFRNRAPLDDSGMPKDRFSA EMPDATLATLKIQPSEPRDSAVYFCASGPEGLDNEQFFGPGTRLTVLEDLKNVFPPEVAV FEPSEAEISHTQKATLVCLATGFYPDHVELSWWVNGKEVHSGVSTDPQPLKEQPALNDS RYCLSSRLRVSATFWQNPRNHFRCQVQFYGLSENDEWTQDRAKPVTQIVSAEAWGRA DCGFTSESYQQGVLSATILYEILLGKATLYAVLVSALVLMAMVKRKDSRGRAKRGSGA TNFSLLKQAGDVEENPGPMATGSRTSLLLAFGLLCLPWLQEGSAQKEVEQNSGPLSVPE GAIASLNCTYSDRGSQSFFWYRQYSGKSPELIMFIYSNGDKEDGRFTAQLNKASQYVSL LIRDSQPSDSATYLCAVNMPGNMLTFGGGTRLMVKPH |
| 393 | Knock-in (KI) nucleotide sequence of nwTCR-1708 | GGCAGCGGCGCCACCAACTTCAGCCTGCTGAAGCAGGCCGGCGACGTGGAGGAGAA CCCCGGCCCCATGGCCACCGGCAGCAGGACCAGCCTGCTGCTGGCCTTCGGCCTGCT GTGCCTGCCCTGGCTGCAGGAGGGCAGCGCCGATGCTAGAGTCACCCAGACACCAA GGGACAAGGTGACAGAGATGGGACAAGAAGTAACAATGAGATGTCAGCCAATTTTA GGCCACAATACTGTTTTCTGGTACAGACAGACCATGATGCAAGGACTGGAGTTGCTG GCTTACTTCCGCAACCGGGCTCCTCTAGATGATTCGGGGATGCCGAAGGATCGATTC TCAGCAGAGATGCCTGATGCAACTTTAGCCACTCTGAAGATCCAGCCCTCAGAACCC AGGGACTCAGCTGTGTATTTTTGTGCTAGTGGTCCCGAGGGGTTGGACAATGAGCAG TTCTTCGGGCCAGGGACACGGCTCACCGTGCTAGAGGACCTGAAAAACGTGTTCCC ACCCGAGGTCGCTGTGTTTGAGCCATCAGAAGCAGAGATCTCCCACACCCAAAAGG CCACACTGGTGTGCCTGGCCACAGGCTTCTACCCCGACCACGTGGAGCTGAGCTGGT GGGTGAATGGGAAGGAGGTGCACAGTGGGGTCAGCACAGACCCGCAGCCCCTCAA GGAGCAGCCCGCCCTCAATGACTCCAGATACTGCCTGTCCTCCCGCCTGAGGGTCTC GGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCG TCAGCGCCGAGGCCTGGGGTAGAGCAGACTGTGGCTTCACCTCCGAGTCTTACCAGC AAGGGGTCCTGTCTGCCACCATCCTCTATGAGATCTTGCTAGGGAAGGCCACCTTGT ATGCCGTGCTGGTCAGTGCCCTCGTGCTGATGGCCATGGTCAAGAGAAAGGATTCCA GAGGCCGGGCCAAGCGGGGATCCGGAGCCACAAATTTCTCCCTGCTGAAACAGGCC GGCGATGTGGAGGAGAATCCTGGACCTATGGCCACCGGCAGCAGGACCAGCCTGCT GCTGGCCTTCGGCCTGCTGTGCCTGCCCTGGCTGCAGGAGGGCAGCGCCCAGAAGG AGGTGGAGCAGAATTCTGGACCCCTCAGTGTTCCAGAGGGAGCCATTGCCTCTCTCA ACTGCACTTACAGTGACCGAGGTTCCCAGTCCTTCTTCTGGTACAGACAATATTCTG GGAAAAGCCCTGAGTTGATAATGTTCATATACTCCAATGGTGACAAAGAAGATGGA AGGTTTACAGCACAGCTCAATAAAGCCAGCCAGTATGTTTCTCTGCTCATCAGAGAC TCCCAGCCCAGTGATTCAGCCACCTACCTCTGTGCCGTGAACATGCCAGGCAACATG CTCACCTTTGGAGGGGGAACAAGGTTAATGGTCAAACCCCAT |
| 394 | KI amino acid sequence of nwTCR-1708-CD8a | GSGATNFSLLKQAGDVEENPGPMALPVTALLLPLALLLHAARPSQFRVSPLDRTWNLGE TVELKCQVLLSNPTSGCSWLFQPRGAAASPTFLLYLSQNKPKAAEGLDTQRFSGKRLGD TFVLTLSDFRRENEGYYFCSALSNSIMYFSHFVPVFLPAKPTTTPAPRPPTPAPTIASQPLS LRPEACRPAAGGAVHTRGLDFACDIYIWAPLAGTCGVLLLSLVITLYCNHRNRRRVCKC PRPVVKSGDKPSLSARYVRAKRGSGEGRGSLLTCGDVEENPGPMATGSRTSLLLAFGLL CLPWLQEGSADARVTQTPRDKVTEMGQEVTMRCQPILGHNTVFWYRQTMMQGLELLA YFRNRAPLDDSGMPKDRFSAEMPDATLATLKIQPSEPRDSAVYFCASGPEGLDNEQFFG PGTRLTVLEDLKNVFPPEVAVFEPSEAEISHTQKATLVCLATGFYPDHVELSWWVNGKE VHSGVSTDPQPLKEQPALNDSRYCLSSRLRVSATFWQNPRNHFRCQVQFYGLSENDEW TQDRAKPVTQIVSAEAWGRADCGFTSESYQQGVLSATILYEILLGKATLYAVLVSALVL MAMVKRKDSRGRAKRGSGATNFSLLKQAGDVEENPGPMATGSRTSLLLAFGLLCLPW LQEGSAQKEVEQNSGPLSVPEGAIASLNCTYSDRGSQSFFWYRQYSGKSPELIMFIYSNG DKEDGRFTAQLNKASQYVSLLIRDSQPSDSATYLCAVNMPGNMLTFGGGTRLMVKPH |
| 395 | KI nucleotide sequence of nwTCR-1708-CD8a | GGCAGCGGCGCCACCAACTTCAGCCTGCTGAAGCAGGCCGGCGACGTGGAGGAGAA CCCCGGCCCCATGGCCTTACCAGTGACCGCCTTGCTCCTGCCGCTGGCCTTGCTGCTC CACGCCGCCAGGCCGAGCCAGTTCCGGGTGTCGCCGCTGGATCGGACCTGGAACCT GGGCGAGACAGTGGAGCTGAAGTGCCAGGTGCTGCTGTCCAACCCGACGTCGGGCT GCTCGTGGCTCTTCCAGCCGCGCGGCGCCGCCGCCAGTCCCACCTTCCTCCTATACC |
| | | TCTCCCAAAACAAGCCCAAGGCGGCCGAGGGGCTGGACACCCAGCGGTTCTCGGGC AAGAGGTTGGGGGACACCTTCGTCCTCACCCTGAGCGACTTCCGCCGAGAGAACGA GGGCTACTATTTCTGCTCGGCCCTGAGCAACTCCATCATGTACTTCAGCCACTTCGTG CCGGTCTTCCTGCCAGCGAAGCCCACCACGACGCCAGCGCCGCGACCACCAACACC GGCGCCCACCATCGCGTCGCAGCCCCTGTCCCTGCGCCCAGAGGCGTGCCGGCCAG CGGCGGGGGGCGCAGTGCACACGAGGGGGCTGGACTTCGCCTGTGATATCTACATC TGGGCGCCCTTGGCCGGGACTTGTGGGGTCCTTCTCCTGTCACTGGTTATCACCCTTT ACTGCAACCACAGGAACCGAAGACGTGTTTGCAAATGTCCCCGGCCTGTGGTCAAA TCGGGAGACAAGCCCAGCCTTTCGGCGAGATACGTCAGAGCCAAAAGAGGAAGCG GAGAGGGCAGGGGAAGTCTTCTAACATGCGGGGACGTGGAGGAAAATCCCGGCCCC ATGGCCACCGGCAGCAGGACCAGCCTGCTGCTGGCCTTCGGCCTGCTGTGCCTGCCC TGGCTGCAGGAGGGCAGCGCCGATGCTAGAGTCACCCAGACACCAAGGGACAAGGT GACAGAGATGGGACAAGAAGTAACAATGAGATGTCAGCCAATTTTAGGCCACAATA CTGTTTTCTGGTACAGACAGACCATGATGCAAGGACTGGAGTTGCTGGCTTACTTCC GCAACCGGGCTCCTCTAGATGATTCGGGGATGCCGAAGGATCGATTCTCAGCAGAG ATGCCTGATGCAACTTTAGCCACTCTGAAGATCCAGCCCTCAGAACCCAGGGACTCA GCTGTGTATTTTTGTGCTAGTGGTCCCGAGGGGTTGGACAATGAGCAGTTCTTCGGG CCAGGGACACGGCTCACCGTGCTAGAGGACCTGAAAAACGTGTTCCCACCCGAGGT CGCTGTGTTTGAGCCATCAGAAGCAGAGATCTCCCACACCCAAAAGGCCACACTGG TGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCC CGCCCTCAATGACTCCAGATACTGCCTGTCCTCCCGCCTGAGGGTCTCGGCCACCTT CTGGCAGAACCCCCGCAACCACTTCCGCTGTCAAGTCCAGTTCTACGGGCTCTCGGA GAATGACGAGTGGACCCAGGATAGGGCCAAACCTGTCACCCAGATCGTCAGCGCCG AGGCCTGGGGTAGAGCAGACTGTGGCTTCACCTCCGAGTCTTACCAGCAAGGGGTC CTGTCTGCCACCATCCTCTATGAGATCTTGCTAGGGAAGGCCACCTTGTATGCCGTG CTGGTCAGTGCCCTCGTGCTGATGGCCATGGTCAAGAGAAAGGATTCCAGAGGCCG GGCCAAGCGGGGATCCGGAGCCACAAATTTCTCCCTGCTGAAACAGGCCGGCGATG TGGAGGAGAATCCTGGACCTATGGCCACCGGCAGCAGGACCAGCCTGCTGCTGGCC TTCGGCCTGCTGTGCCTGCCCTGGCTGCAGGAGGGCAGCGCCCAGAAGGAGGTGGA GCAGAATTCTGGACCCCTCAGTGTTCCAGAGGGAGCCATTGCCTCTCTCAACTGCAC TTACAGTGACCGAGGTTCCCAGTCCTTCTTCTGGTACAGACAATATTCTGGGAAAAG CCCTGAGTTGATAATGTTCATATACTCCAATGGTGACAAAGAAGATGGAAGGTTTAC AGCACAGCTCAATAAAGCCAGCCAGTATGTTTCTCTGCTCATCAGAGACTCCCAGCC CAGTGATTCAGCCACCTACCTCTGTGCCGTGAACATGCCAGGCAACATGCTCACCTT TGGAGGGGGAACAAGGTTAATGGTCAAACCCCAT |
| 396 | Amino acid sequence of CD8a | |
| 397 | Nucleotide sequence of CD8a | ATGGCCTTACCAGTGACCGCCTTGCTCCTGCCGCTGGCCTTGCTGCTCCACGCCGCC AGGCCGAGCCAGTTCCGGGTGTCGCCGCTGGATCGGACCTGGAACCTGGGCGAGAC AGTGGAGCTGAAGTGCCAGGTGCTGCTGTCCAACCCGACGTCGGGCTGCTCGTGGCT CTTCCAGCCGCGCGGCGCCGCCGCCAGTCCCACCTTCCTCCTATACCTCTCCCAAAA CAAGCCCAAGGCGGCCGAGGGGCTGGACACCCAGCGGTTCTCGGGCAAGAGGTTGG GGGACACCTTCGTCCTCACCCTGAGCGACTTCCGCCGAGAGAACGAGGGCTACTATT TCTGCTCGGCCCTGAGCAACTCCATCATGTACTTCAGCCACTTCGTGCCGGTCTTCCT GCCAGCGAAGCCCACCACGACGCCAGCGCCGCGACCACCAACACCGGCGCCCACCA TCGCGTCGCAGCCCCTGTCCCTGCGCCCAGAGGCGTGCCGGCCAGCGGCGGGGGGC GCAGTGCACACGAGGGGGCTGGACTTCGCCTGTGATATCTACATCTGGGCGCCCTTG GCCGGGACTTGTGGGGTCCTTCTCCTGTCACTGGTTATCACCCTTTACTGCAACCACA GGAACCGAAGACGTGTTTGCAAATGTCCCCGGCCTGTGGTCAAATCGGGAGACAAG CCCAGCCTTTCGGCGAGATACGTC |
| 398 | KI amino acid sequence of nwTCR-1708-CD8ab | GSGATNFSLLKQAGDVEENPGPMALPVTALLLPLALLLHAARPSQFRVSPLDRTW NLGETVELKCQVLLSNPTSGCSWLFQPRGAAASPTFLLYLSQNKPKAAEGLDTQRFSGK RLGDTFVLTLSDFRRENEGYYFCSALSNSIMYFSHFVPVFLPAKPTTTPAPRPPTPAPTIAS QPLSLRPEACRPAAGGAVHTRGLDFACDIYIWAPLAGTCGVLLLSLVITLYCNHRNRRR VCKCPRPVVKSGDKPSLSARYVRAKRGSGQCTNYALLKLAGDVESNPGPMRPRLWLLL AAQLTVLHGNSVLQQTPAYIKVQTNKMVMLSCEAKISLSNMRIYWLRQRQAPSSDSHH EFLALWDSAKGTIHGEEVEQEKIAVFRDASRFILNLTSVKPEDSGIYFCMIVGSPELTFGK GTQLSVVDFLPTTAQPTKKSTLKKRVCRLPRPETQKGPLCSPITLGLLVAGVLVLLVSLG VAIHLCCRRRRARLRFMKQFYKRAKRGSGEGRGSLLTCGDVEENPGPMATGSRTSLLL AFGLLCLPWLQEGSADARVTQTPRDKVTEMGQEVTMRCQPILGHNTVFWYRQTMMQ GLELLAYFRNRAPLDDSGMPKDRFSAEMPDATLATLKIQPSEPRDSAVYFCASGPEGLD NEQFFGPGTRLTVLEDLKNVFPPEVAVFEPSEAEISHTQKATLVCLATGFYPDHVELSW WVNGKEVHSGVSTDPQPLKEQPALNDSRYCLSSRLRVSATFWQNPRNHFRCQVQFYGL |
| | | SENDEWTQDRAKPVTQIVSAEAWGRADCGFTSESYQQGVLSATILYEILLGKATLYAVL VSALVLMAMVKRKDSRGRAKRGSGATNFSLLKQAGDVEENPGPMATGSRTSLLLAFG LLCLPWLQEGSAQKEVEQNSGPLSVPEGAIASLNCTYSDRGSQSFFWYRQYSGKSPELI MFIYSNGDKEDGRFTAQLNKASQYVSLLIRDSQPSDSATYLCAVNMPGNMLTFGGGTR LMVKPH |
| 399 | KI nucleotide sequence of nwTCR-1708-CD8ab | GGCAGCGGCGCCACCAACTTCAGCCTGCTGAAGCAGGCCGGCGACGTGGAGG AGAACCCCGGCCCCATGGCCTTACCAGTGACCGCCTTGCTCCTGCCGCTGGCCTTGC TGCTCCACGCCGCCAGGCCGAGCCAGTTCCGGGTGTCGCCGCTGGATCGGACCTGG AACCTGGGCGAGACAGTGGAGCTGAAGTGCCAGGTGCTGCTGTCCAACCCGACGTC GGGCTGCTCGTGGCTCTTCCAGCCGCGCGGCGCCGCCGCCAGTCCCACCTTCCTCCT ATACCTCTCCCAAAACAAGCCCAAGGCGGCCGAGGGGCTGGACACCCAGCGGTTCT CGGGCAAGAGGTTGGGGGACACCTTCGTCCTCACCCTGAGCGACTTCCGCCGAGAG AACGAGGGCTACTATTTCTGCTCGGCCCTGAGCAACTCCATCATGTACTTCAGCCAC TTCGTGCCGGTCTTCCTGCCAGCGAAGCCCACCACGACGCCAGCGCCGCGACCACCA ACACCGGCGCCCACCATCGCGTCGCAGCCCCTGTCCCTGCGCCCAGAGGCGTGCCG GCCAGCGGCGGGGGGCGCAGTGCACACGAGGGGGCTGGACTTCGCCTGTGATATCT ACATCTGGGCGCCCTTGGCCGGGACTTGTGGGGTCCTTCTCCTGTCACTGGTTATCA CCCTTTACTGCAACCACAGGAACCGAAGACGTGTTTGCAAATGTCCCCGGCCTGTGG TCAAATCGGGAGACAAGCCCAGCCTTTCGGCGAGATACGTCAGAGCCAAAAGAGGA AGCGGACAGTGTACTAATTATGCTCTCTTGAAATTGGCTGGAGATGTTGAGAGCAAC CCAGGTCCCATGCGGCCGCGGCTGTGGCTCCTCTTGGCCGCGCAGCTGACAGTTCTC CATGGCAACTCAGTCCTCCAGCAGACCCCTGCATACATAAAGGTGCAAACCAACAA GATGGTGATGCTGTCCTGCGAGGCTAAAATCTCCCTCAGTAACATGCGCATCTACTG GCTGAGACAGCGCCAGGCACCGAGCAGTGACAGTCACCACGAGTTCCTGGCCCTCT GGGATTCCGCAAAAGGGACTATCCACGGTGAAGAGGTGGAACAGGAGAAGATAGC TGTGTTTCGGGATGCAAGCCGGTTCATTCTCAATCTCACAAGCGTGAAGCCGGAAGA CAGTGGCATCTACTTCTGCATGATCGTCGGGAGCCCCGAGCTGACCTTCGGGAAGGG AACTCAGCTGAGTGTGGTTGATTTCCTTCCCACCACTGCCCAGCCCACCAAGAAGTC CACCCTCAAGAAGAGAGTGTGCCGGTTACCCAGGCCAGAGACCCAGAAGGGCCCAC TTTGTAGCCCCATCACCCTTGGCCTGCTGGTGGCTGGCGTCCTGGTTCTGCTGGTTTC CCTGGGAGTGGCCATCCACCTGTGCTGCCGGCGGAGGAGAGCCCGGCTTCGTTTCAT GAAACAATTTTACAAAAGAGCCAAAAGAGGAAGCGGAGAGGGCAGGGGAAGTCTT CTAACATGCGGGGACGTGGAGGAAAATCCCGGCCCCATGGCCACCGGCAGCAGGAC CAGCCTGCTGCTGGCCTTCGGCCTGCTGTGCCTGCCCTGGCTGCAGGAGGGCAGCGC CGATGCTAGAGTCACCCAGACACCAAGGGACAAGGTGACAGAGATGGGACAAGAA GTAACAATGAGATGTCAGCCAATTTTAGGCCACAATACTGTTTTCTGGTACAGACAG ACCATGATGCAAGGACTGGAGTTGCTGGCTTACTTCCGCAACCGGGCTCCTCTAGAT GATTCGGGGATGCCGAAGGATCGATTCTCAGCAGAGATGCCTGATGCAACTTTAGC CACTCTGAAGATCCAGCCCTCAGAACCCAGGGACTCAGCTGTGTATTTTTGTGCTAG TGGTCCCGAGGGGTTGGACAATGAGCAGTTCTTCGGGCCAGGGACACGGCTCACCG TGCTAGAGGACCTGAAAAACGTGTTCCCACCCGAGGTCGCTGTGTTTGAGCCATCAG AAGCAGAGATCTCCCACACCCAAAAGGCCACACTGGTGTGCCTGGCCACAGGCTTC TACCCCGACCACGTGGAGCTGAGCTGGTGGGTGAATGGGAAGGAGGTGCACAGTGG GGTCAGCACAGACCCGCAGCCCCTCAAGGAGCAGCCCGCCCTCAATGACTCCAGAT ACTGCCTGTCCTCCCGCCTGAGGGTCTCGGCCACCTTCTGGCAGAACCCCCGCAACC ACTTCCGCTGTCAAGTCCAGTTCTACGGGCTCTCGGAGAATGACGAGTGGACCCAGG ATAGGGCCAAACCTGTCACCCAGATCGTCAGCGCCGAGGCCTGGGGTAGAGCAGAC TGTGGCTTCACCTCCGAGTCTTACCAGCAAGGGGTCCTGTCTGCCACCATCCTCTAT GAGATCTTGCTAGGGAAGGCCACCTTGTATGCCGTGCTGGTCAGTGCCCTCGTGCTG ATGGCCATGGTCAAGAGAAAGGATTCCAGAGGCCGGGCCAAGCGGGGATCCGGAG CCACAAATTTCTCCCTGCTGAAACAGGCCGGCGATGTGGAGGAGAATCCTGGACCT ATGGCCACCGGCAGCAGGACCAGCCTGCTGCTGGCCTTCGGCCTGCTGTGCCTGCCC TGGCTGCAGGAGGGCAGCGCCCAGAAGGAGGTGGAGCAGAATTCTGGACCCCTCAG TGTTCCAGAGGGAGCCATTGCCTCTCTCAACTGCACTTACAGTGACCGAGGTTCCCA GTCCTTCTTCTGGTACAGACAATATTCTGGGAAAAGCCCTGAGTTGATAATGTTCAT ATACTCCAATGGTGACAAAGAAGATGGAAGGTTTACAGCACAGCTCAATAAAGCCA GCCAGTATGTTTCTCTGCTCATCAGAGACTCCCAGCCCAGTGATTCAGCCACCTACC TCTGTGCCGTGAACATGCCAGGCAACATGCTCACCTTTGGAGGGGGAACAAGGTTA ATGGTCAAACCCCAT |
| 400 | Amino acid sequence of CD8b | MRPRLWLLLAAQLTVLHGNSVLQQTPAYIKVQTNKMVMLSCEAKISLSNMRIYWLRQR QAPSSDSHHEFLALWDSAKGTIHGEEVEQEKIAVFRDASRFILNLTSVKPEDSGIYFCMIV GSPELTFGKGTQLSVVDFLPTTAQPTKKSTLKKRVCRLPRPETQKGPLCSPITLGLLVAG VLVLLVSLGVAIHLCCRRRRARLRFMKQFYK |
| 401 | Nucleotide sequence of CD8b | ATGCGGCCGCGGCTGTGGCTCCTCTTGGCCGCGCAGCTGACAGTTCTCCATGGCAAC TCAGTCCTCCAGCAGACCCCTGCATACATAAAGGTGCAAACCAACAAGATGGTGAT GCTGTCCTGCGAGGCTAAAATCTCCCTCAGTAACATGCGCATCTACTGGCTGAGACA |
| | | GCGCCAGGCACCGAGCAGTGACAGTCACCACGAGTTCCTGGCCCTCTGGGATTCCG CAAAAGGGACTATCCACGGTGAAGAGGTGGAACAGGAGAAGATAGCTGTGTTTCGG GATGCAAGCCGGTTCATTCTCAATCTCACAAGCGTGAAGCCGGAAGACAGTGGCAT CTACTTCTGCATGATCGTCGGGAGCCCCGAGCTGACCTTCGGGAAGGGAACTCAGCT GAGTGTGGTTGATTTCCTTCCCACCACTGCCCAGCCCACCAAGAAGTCCACCCTCAA GAAGAGAGTGTGCCGGTTACCCAGGCCAGAGACCCAGAAGGGCCCACTTTGTAGCC CCATCACCCTTGGCCTGCTGGTGGCTGGCGTCCTGGTTCTGCTGGTTTCCCTGGGAGT GGCCATCCACCTGTGCTGCCGGCGGAGGAGAGCCCGGCTTCGTTTCATGAAACAATT TTACAAA |
| 402 | Amino acid sequence of 2A | GSGATNFSLLKQAGDVEENPGP |
| 403 | Amino acid sequence of SP (signal peptide) | MATGSRTSLLLAFGLLCLPWLQEGSA |
| 404 | Amino acid sequence of 2A variant | RAKRGSGATNFSLLKQAGDVEENPGP |
| 405 | Amino acid sequence of Cas9 enzyme | |

## Claims

1. An isolated or purified T-cell receptor (also abbreviated as TCR), **characterized in that** the TCR binds specifically to KRAS_G12V mutant antigen, and comprises an α chain and a β chain, wherein each of the α chain and β chain comprises three complementarity determining regions (also abbreviated as CDRs), and wherein the amino acid sequence of CDR3 of the α chain is selected from SEQ ID NOs: 3, 6, 9, 12, 15, 18, 21, 24, 27, 30, 33, 36, 39, 42, 45, 48, and a variant having one or two amino acid residue changes from the sequence, and the amino acid sequence of CDR3 of the β chain is selected from SEQ ID NOs: 179, 182, 185, 188, 191, 194, 197, 200, 203, 206, 209, 212, 215, 218, 221, 224, and a variant having one or two amino acid residue changes from the sequence.

2. The TCR according to claim 1, wherein the amino acid sequence of CDR3 of the α chain and the amino acid sequence of CDR3 of the β chain are:
(i) the α chain CDR3 amino acid sequence shown in SEQ ID NO: 3 or a variant having 1 or 2 amino acid residue changes from the sequence; and the β chain CDR3 amino acid sequence shown in SEQ ID NO: 179 or a variant having 1 or 2 amino acid residue changes from the sequence;
(ii) the α chain CDR3 amino acid sequence shown in SEQ ID NO: 6 or a variant having 1 or 2 amino acid residue changes from the sequence; and the β chain CDR3 amino acid sequence shown in SEQ ID NO: 182 or a variant having 1 or 2 amino acid residue changes from the sequence;
(iii) the α chain CDR3 amino acid sequence shown in SEQ ID NO: 9 or a variant having 1 or 2 amino acid residue changes from the sequence; and the β chain CDR3 amino acid sequence shown in SEQ ID NO: 185 or a variant having 1 or 2 amino acid residue changes from the sequence;
(iv) the α chain CDR3 amino acid sequence shown in SEQ ID NO: 12 or a variant having 1 or 2 amino acid residue changes from the sequence; and the β chain CDR3 amino acid sequence shown in SEQ ID NO: 188 or a variant having 1 or 2 amino acid residue changes from the sequence;
(v) the α chain CDR3 amino acid sequence shown in SEQ ID NO: 15 or a variant having 1 or 2 amino acid residue changes from the sequence; and the β chain CDR3 amino acid sequence shown in SEQ ID NO: 191 or a variant having 1 or 2 amino acid residue changes from the sequence;
(vi) the α chain CDR3 amino acid sequence shown in SEQ ID NO: 18 or a variant having 1 or 2 amino acid residue changes from the sequence; and the β chain CDR3 amino acid sequence shown in SEQ ID NO: 194 or a variant having 1 or 2 amino acid residue changes from the sequence;
(vii) the α chain CDR3 amino acid sequence shown in SEQ ID NO: 21 or a variant having 1 or 2 amino acid residue changes from the sequence; and the β chain CDR3 amino acid sequence shown in SEQ ID NO: 197 or a variant having 1 or 2 amino acid residue changes from the sequence;
(viii) the α chain CDR3 amino acid sequence shown in SEQ ID NO: 24 or a variant having 1 or 2 amino acid residue changes from the sequence; and the β chain CDR3 amino acid sequence shown in SEQ ID NO: 200 or a variant having 1 or 2 amino acid residue changes from the sequence;
(ix) the α chain CDR3 amino acid sequence shown in SEQ ID NO: 27 or a variant having 1 or 2 amino acid residue changes from the sequence; and the β chain CDR3 amino acid sequence shown in SEQ ID NO: 203 or a variant having 1 or 2 amino acid residue changes from the sequence;
(x) the α chain CDR3 amino acid sequence shown in SEQ ID NO: 30 or a variant having 1 or 2 amino acid residue changes from the sequence; and the β chain CDR3 amino acid sequence shown in SEQ ID NO: 206 or a variant having 1 or 2 amino acid residue changes from the sequence;
(xi) the α chain CDR3 amino acid sequence shown in SEQ ID NO: 33 or a variant having 1 or 2 amino acid residue changes from the sequence; and the β chain CDR3 amino acid sequence shown in SEQ ID NO: 209 or a variant having 1 or 2 amino acid residue changes from the sequence;
(xii) the α chain CDR3 amino acid sequence shown in SEQ ID NO: 36 or a variant having 1 or 2 amino acid residue changes from the sequence; and the β chain CDR3 amino acid sequence shown in SEQ ID NO: 212 or a variant having 1 or 2 amino acid residue changes from the sequence;
(xiii) the α chain CDR3 amino acid sequence shown in SEQ ID NO: 39 or a variant having 1 or 2 amino acid residue changes from the sequence; and the β chain CDR3 amino acid sequence shown in SEQ ID NO: 215 or a variant having 1 or 2 amino acid residue changes from the sequence;
(xiv) the α chain CDR3 amino acid sequence shown in SEQ ID NO: 42 or a variant having 1 or 2 amino acid residue changes from the sequence; and the β chain CDR3 amino acid sequence shown in SEQ ID NO: 218 or a variant having 1 or 2 amino acid residue changes from the sequence;
(xv) the α chain CDR3 amino acid sequence shown in SEQ ID NO: 45 or a variant having 1 or 2 amino acid residue changes from the sequence; and the β chain CDR3 amino acid sequence shown in SEQ ID NO: 221 or a variant having 1 or 2 amino acid residue changes from the sequence; or
(xvi) the α chain CDR3 amino acid sequence shown in SEQ ID NO: 48 or a variant having 1 or 2 amino acid residue changes from the sequence; and the β chain CDR3 amino acid sequence shown in SEQ ID NO: 224 or a variant having 1 or 2 amino acid residue changes from the sequence;
Preferably, the amino acid sequence of CDR3 of the α chain and the amino acid sequence of CDR3 of the β chain are:
(i) the α chain CDR3 amino acid sequence shown in SEQ ID NO: 3; and the β chain CDR3 amino acid sequence shown in SEQ ID NO: 179;
(ii) the α chain CDR3 amino acid sequence shown in SEQ ID NO: 6; and the β chain CDR3 amino acid sequence shown in SEQ ID NO: 182;
(iii) the α chain CDR3 amino acid sequence shown in SEQ ID NO: 9; and the β chain CDR3 amino acid sequence shown in SEQ ID NO: 185;
(iv) the α chain CDR3 amino acid sequence shown in SEQ ID NO: 12; and the β chain CDR3 amino acid sequence shown in SEQ ID NO: 188;
(v) the α chain CDR3 amino acid sequence shown in SEQ ID NO: 15; and the β chain CDR3 amino acid sequence shown in SEQ ID NO: 191;
(vi) the α chain CDR3 amino acid sequence shown in SEQ ID NO: 18; and the β chain CDR3 amino acid sequence shown in SEQ ID NO: 194;
(vii) the α chain CDR3 amino acid sequence shown in SEQ ID NO: 21; and the β chain CDR3 amino acid sequence shown in SEQ ID NO: 197;
(viii) the α chain CDR3 amino acid sequence shown in SEQ ID NO: 24; and the β chain CDR3 amino acid sequence shown in SEQ ID NO: 200;
(ix) the α chain CDR3 amino acid sequence shown in SEQ ID NO: 27; and the β chain CDR3 amino acid sequence shown in SEQ ID NO: 203;
(x) the α chain CDR3 amino acid sequence shown in SEQ ID NO: 30; and the β chain CDR3 amino acid sequence shown in SEQ ID NO: 206;
(xi) the α chain CDR3 amino acid sequence shown in SEQ ID NO: 33; and the β chain CDR3 amino acid sequence shown in SEQ ID NO: 209;
(xii) the α chain CDR3 amino acid sequence shown in SEQ ID NO: 36; and the β chain CDR3 amino acid sequence shown in SEQ ID NO: 212;
(xiii) the α chain CDR3 amino acid sequence shown in SEQ ID NO: 39; and the β chain CDR3 amino acid sequence shown in SEQ ID NO: 215;
(xiv) the α chain CDR3 amino acid sequence shown in SEQ ID NO: 42; and the β chain CDR3 amino acid sequence shown in SEQ ID NO: 218;
(xv) the α chain CDR3 amino acid sequence shown in SEQ ID NO: 45; and the β chain CDR3 amino acid sequence shown in SEQ ID NO: 221; or
(xvi) the α chain CDR3 amino acid sequence shown in SEQ ID NO: 48; and the β chain CDR3 amino acid sequence shown in SEQ ID NO: 224.

3. The TCR according to claim 2, wherein the amino acid sequences of the three CDRs comprised in the α chain and the amino acid sequences of the three CDRs comprised in the β chain are:
(i) the α chain CDR1, CDR2, CDR3 amino acid sequences shown in SEQ ID NOs: 1, 2, 3 or variants having 1 or 2 amino acid residue changes from the sequences respectively; and the β chain CDR1, CDR2, CDR3 amino acid sequences shown in SEQ ID NOs: 177, 178, 179 or variants having 1 or 2 amino acid residue changes from the sequences respectively;
(ii) the α chain CDR1, CDR2, CDR3 amino acid sequences shown in SEQ ID NOs: 4, 5, 6 or variants having 1 or 2 amino acid residue changes from the sequences respectively; and the β chain CDR1, CDR2, CDR3 amino acid sequences shown in SEQ ID NOs: 180, 181, 182 or variants having 1 or 2 amino acid residue changes from the sequences respectively;
(iii) the α chain CDR1, CDR2, CDR3 amino acid sequences shown in SEQ ID NOs: 7, 8, 9 or variants having 1 or 2 amino acid residue changes from the sequences respectively; and the β chain CDR1, CDR2, CDR3 amino acid sequences shown in SEQ ID NOs: 183, 184, 185 or variants having 1 or 2 amino acid residue changes from the sequences respectively;
(iv) the α chain CDR1, CDR2, CDR3 amino acid sequences shown in SEQ ID NOs: 10, 11, 12 or variants having 1 or 2 amino acid residue changes from the sequences respectively; and the β chain CDR1, CDR2, CDR3 amino acid sequences shown in SEQ ID NOs: 186, 187, 188 or variants having 1 or 2 amino acid residue changes from the sequences respectively;
(v) the α chain CDR1, CDR2, CDR3 amino acid sequence shown in SEQ ID NOs: 13, 14, 15 or variants having 1 or 2 amino acid residue changes from the sequences respectively; and the β chain CDR1, CDR2, CDR3 amino acid sequence shown in SEQ ID NOs: 189, 190, 191 or variants having 1 or 2 amino acid residue changes from the sequences respectively;
(vi) the α chain CDR1, CDR2, CDR3 amino acid sequences shown in SEQ ID NOs: 16, 17, 18 or variants having 1 or 2 amino acid residue changes from the sequences respectively; and the β chain CDR1, CDR2, CDR3 amino acid sequences shown in SEQ ID NOs: 192, 193, 194 or variants having 1 or 2 amino acid residue changes from the sequences respectively;
(vii) the α chain CDR1, CDR2, CDR3 amino acid sequences shown in SEQ ID NOs: 19, 20, 21 or variants having 1 or 2 amino acid residue changes from the sequences respectively; and the β chain CDR1, CDR2, CDR3 amino acid sequences shown in SEQ ID NOs: 195, 196, 197 or variants having 1 or 2 amino acid residue changes from the sequences respectively;
(viii) the α chain CDR1, CDR2, CDR3 amino acid sequences shown in SEQ ID NOs: 22, 23, 24 or variants having 1 or 2 amino acid residue changes from the sequences respectively; and the β chain CDR1, CDR2, CDR3 amino acid sequences shown in SEQ ID NOs: 198, 199, 200 or variants having 1 or 2 amino acid residue changes from the sequences respectively;
(ix) the α chain CDR1, CDR2, CDR3 amino acid sequences shown in SEQ ID NOs: 25, 26, 27 or variants having 1 or 2 amino acid residue changes from the sequences respectively; and the β chain CDR1, CDR2, CDR3 amino acid sequences shown in SEQ ID NOs: 201, 202, 203 or variants having 1 or 2 amino acid residue changes from the sequences respectively;
(x) the α chain CDR1, CDR2, CDR3 amino acid sequences shown in SEQ ID NOs: 28, 29, 30 or variants having 1 or 2 amino acid residue changes from the sequences respectively; and the β chain CDR1, CDR2, CDR3 amino acid sequences shown in SEQ ID NOs: 204, 205, 206 or variants having 1 or 2 amino acid residue changes from the sequences respectively;
(xi) the α chain CDR1, CDR2, CDR3 amino acid sequences shown in SEQ ID NOs: 31, 32, 33 or variants having 1 or 2 amino acid residue changes from the sequences respectively; and the β chain CDR1, CDR2, CDR3 amino acid sequences shown in SEQ ID NOs: 207, 208, 209 or variants having 1 or 2 amino acid residue changes from the sequences respectively;
(xii) the α chain CDR1, CDR2, CDR3 amino acid sequences shown in SEQ ID NOs: 34, 35, 36 or variants having 1 or 2 amino acid residue changes from the sequences respectively; and the β chain CDR1, CDR2, CDR3 amino acid sequences shown in SEQ ID NOs: 210, 211, 212 or variants having 1 or 2 amino acid residue changes from the sequences respectively;
(xiii) the α chain CDR1, CDR2, CDR3 amino acid sequences shown in SEQ ID NOs: 37, 38, 39 or variants having 1 or 2 amino acid residue changes from the sequences respectively; and the β chain CDR1, CDR2, CDR3 amino acid sequences shown in SEQ ID NOs: 213, 214, 215 or variants having 1 or 2 amino acid residue changes from the sequences respectively;
(xiv) the α chain CDR1, CDR2, CDR3 amino acid sequences shown in SEQ ID NOs: 40, 41, 42 or variants having 1 or 2 amino acid residue changes from the sequences respectively; and the β chain CDR1, CDR2, CDR3 amino acid sequences shown in SEQ ID NOs: 216, 217, 218 or variants having 1 or 2 amino acid residue changes from the sequences respectively;
(xv) the α chain CDR1, CDR2, CDR3 amino acid sequence shown in SEQ ID NOs: 43, 44, 45 or variants having 1 or 2 amino acid residue changes from the sequences respectively; and the β chain CDR1, CDR2, CDR3 amino acid sequence shown in SEQ ID NOs: 219, 220, 221 or variants having 1 or 2 amino acid residue changes from the sequences respectively; or
(xvi) the α chain CDR1, CDR2, CDR3 amino acid sequences shown in SEQ ID NOs: 46, 47, 48 or variants having 1 or 2 amino acid residue changes from the sequences respectively; and the β chain CDR1, CDR2, CDR3 amino acid sequences shown in SEQ ID NOs: 222, 223, 224 or variants having 1 or 2 amino acid residue changes from the sequences respectively;
Preferably, the amino acid sequences of the three CDRs comprised in the α chain and the amino acid sequences of the three CDRs comprised in the β chain are:
(i) the α chain CDR1, CDR2, CDR3 amino acid sequences shown in SEQ ID NOs: 1, 2, 3; and the β chain CDR1, CDR2, CDR3 amino acid sequences shown in SEQ ID NOs: 177, 178, 179;
(ii) the α chain CDR1, CDR2, CDR3 amino acid sequences shown in SEQ ID NOs: 4, 5, 6; and the β chain CDR1, CDR2, CDR3 amino acid sequences shown in SEQ ID NOs: 180, 181, 182;
(iii) the α chain CDR1, CDR2, CDR3 amino acid sequences shown in SEQ ID NOs: 7, 8, 9; and the β chain CDR1, CDR2, CDR3 amino acid sequences shown in SEQ ID NOs: 183, 184, 185;
(iv) the α chain CDR1, CDR2, CDR3 amino acid sequences shown in SEQ ID NOs: 10, 11, 12; and the β chain CDR1, CDR2, CDR3 amino acid sequences shown in SEQ ID NOs: 186, 187, 188;
(v) the α chain CDR1, CDR2, CDR3 amino acid sequence shown in SEQ ID NOs: 13, 14, 15; and the β chain CDR1, CDR2, CDR3 amino acid sequence shown in SEQ ID NOs: 189, 190, 191;
(vi) the α chain CDR1, CDR2, CDR3 amino acid sequences shown in SEQ ID NOs: 16, 17, 18; and the β chain CDR1, CDR2, CDR3 amino acid sequences shown in SEQ ID NOs: 192, 193, 194;
(vii) the α chain CDR1, CDR2, CDR3 amino acid sequences shown in SEQ ID NOs: 19, 20, 21; and the β chain CDR1, CDR2, CDR3 amino acid sequences shown in SEQ ID NOs: 195, 196, 197;
(viii) the α chain CDR1, CDR2, CDR3 amino acid sequences shown in SEQ ID NOs: 22, 23, 24; and the β chain CDR1, CDR2, CDR3 amino acid sequences shown in SEQ ID NOs: 198, 199, 200;
(ix) the α chain CDR1, CDR2, CDR3 amino acid sequences shown in SEQ ID NOs: 25, 26, 27; and the β chain CDR1, CDR2, CDR3 amino acid sequences shown in SEQ ID NOs: 201, 202, 203;
(x) the α chain CDR1, CDR2, CDR3 amino acid sequences shown in SEQ ID NOs: 28, 29, 30; and the β chain CDR1, CDR2, CDR3 amino acid sequences shown in SEQ ID NOs: 204, 205, 206;
(xi) the α chain CDR1, CDR2, CDR3 amino acid sequences shown in SEQ ID NOs: 31, 32, 33; and the β chain CDR1, CDR2, CDR3 amino acid sequences shown in SEQ ID NOs: 207, 208, 209;
(xii) the α chain CDR1, CDR2, CDR3 amino acid sequences shown in SEQ ID NOs: 34, 35, 36; and the β chain CDR1, CDR2, CDR3 amino acid sequences shown in SEQ ID NOs: 210, 211, 212;
(xiii) the α chain CDR1, CDR2, CDR3 amino acid sequences shown in SEQ ID NOs: 37, 38, 39; and the β chain CDR1, CDR2, CDR3 amino acid sequences shown in SEQ ID NOs: 213, 214, 215;
(xiv) the α chain CDR1, CDR2, CDR3 amino acid sequences shown in SEQ ID NOs: 40, 41, 42; and the β chain CDR1, CDR2, CDR3 amino acid sequences shown in SEQ ID NOs: 216, 217, 218;
(xv) the α chain CDR1, CDR2, CDR3 amino acid sequence shown in SEQ ID NOs: 43, 44, 45, respectively; and the β chain CDR1, CDR2, CDR3 amino acid sequence shown in SEQ ID NOs: 219, 220, 221; or
(xvi) the α chain CDR1, CDR2, CDR3 amino acid sequences shown in SEQ ID NOs: 46, 47, 48; and the β chain CDR1, CDR2, CDR3 amino acid sequences shown in SEQ ID NOs: 222, 223, 224.

4. The TCR according to any one of claims 1 to 3, wherein the TCR further comprises a constant region, e.g., a mouse constant region;
Preferably, the TCR comprises an α chain sequence shown in SEQ ID NO: 145, 147, 149, 151, 153, 155, 157, 159, 161, 163, 165, 167, 169, 171, 173, or 175, or a sequence having at least 90%, 91%, 92%, 93%, 94%, 95%, 96%, 97%, 98%, or 99% identity thereto; and a β chain sequence shown in SEQ ID NO: 349, 351, 353, 355, 357, 359, 361, 363, 365, 367, 369, 371, 373, 375, 377, or 379, or a sequence having at least 90%, 91%, 92%, 93%, 94%, 95%, 96%, 97%, 98%, or 99% identity thereto;
More preferably, the TCR comprises an α chain sequence shown in SEQ ID NO: 145 and a β chain sequence shown in SEQ ID NO: 349;
the TCR comprises an α chain sequence shown in SEQ ID NO: 147 and a β chain sequence shown in SEQ ID NO: 351;
the TCR comprises an α chain sequence shown in SEQ ID NO: 149 and a β chain sequence shown in SEQ ID NO: 353;
the TCR comprises an α chain sequence shown in SEQ ID NO: 151 and a β chain sequence shown in SEQ ID NO: 355;
the TCR comprises an α chain sequence shown in SEQ ID NO: 153 and a β chain sequence shown in SEQ ID NO: 357;
the TCR comprises an α chain sequence shown in SEQ ID NO: 155 and a β chain sequence shown in SEQ ID NO: 359;
the TCR comprises an α chain sequence shown in SEQ ID NO: 157 and a β chain sequence shown in SEQ ID NO: 361;
the TCR comprises an α chain sequence shown in SEQ ID NO: 159 and a β chain sequence shown in SEQ ID NO: 363;
the TCR comprises an α chain sequence shown in SEQ ID NO: 161 and a β chain sequence shown in SEQ ID NO: 365;
the TCR comprises an α chain sequence shown in SEQ ID NO: 163 and a β chain sequence shown in SEQ ID NO: 367;
the TCR comprises an α chain sequence shown in SEQ ID NO: 165 and a β chain sequence shown in SEQ ID NO: 369;
the TCR comprises an α chain sequence shown in SEQ ID NO: 167 and a β chain sequence shown in SEQ ID NO: 371;
the TCR comprises an α chain sequence shown in SEQ ID NO: 169 and a β chain sequence shown in SEQ ID NO: 373;
the TCR comprises an α chain sequence shown in SEQ ID NO: 171 and a β chain sequence shown in SEQ ID NO: 375;
the TCR comprises an α chain sequence shown in SEQ ID NO: 173 and a β chain sequence shown in SEQ ID NO: 377;
the TCR comprises an α chain sequence shown in SEQ ID NO: 175 and a β chain sequence shown in SEQ ID NO: 379.

5. A nucleic acid molecule, **characterized in that** the nucleic acid molecule encodes the TCR according to any one of claims 1-4, preferably the nucleic acid molecule is a codon-optimized nucleotide sequence encoding the TCR according to any one of claims 1-4.

6. A vector, **characterized in that** the vector comprises the nucleic acid molecule according to claim 5, preferably the vector is a plasmid, a shuttle plasmid, a phagemid, a cosmid, an expression vector; more preferably the vector is a vector for homology directed repair (HDR) or a viral vector, e.g. a lentiviral vector, an adenoviral vector, an adeno-associated viral (AAV) vector, a herpesvirus vector, a retroviral vector, a baculoviral vector.

7. A T-cell receptor fusion protein or T-cell receptor conjugate, comprising the TCR according to any one of claims 1-4 and other biologically active molecules, wherein the other biologically active molecules are e.g., antibodies, cytokines, cytotoxic agents, enzymes, radioactive substances, detectable labels, wherein the TCR and the other biologically active molecules are linked to each other with or without a linker.

8. An engineered cell, **characterized in that** the engineered cell expresses the TCR according to any one of claims 1-4, preferably, the engineered cell is an engineered T cell, an engineered NK cell; or the engineered cell is an engineered stem cell, e.g., the engineered cell is an engineered human CD4+ T cell or an engineered human CD8+ T cell, or a mixed cell population of the engineered human CD4+ T cells and the engineered human CD8+ T cells; or, the engineered cell is an engineered hematopoietic stem cell.

9. An engineered human CD4+ T cell and/or an engineered human CD8+ T cell, **characterized in that** the engineered human CD4+ T cell and/or the engineered human CD8+ T cell express the TCR according to any one of claims 1-4 and an exogenous CD8a or CD8ab.

10. A method for generating the T cell according to claim 9, comprising the step of transfecting a CD4+ T cell and/or a CD8+ T cell with an exogenous CD8a or CD8ab and the TCR according to any one of claims 1-4, for example, wherein the exogenous CD8a or CD8ab and the TCR according to any one of claims 1-4 are expressed in the CD4+ T cell and/or the CD8+ T cell from the same vector, e.g., wherein a construct expressing the exogenous CD8a or CD8ab and a construct expressing the TCR according to any one of claims 1-4 in the same vector are separated by a 2A element or an IRES element.

11. A pharmaceutical composition, **characterized in that** the pharmaceutical composition comprises the engineered cell according to claim 8 and/or the engineered human CD4+ T cell and/or the engineered human CD8+ T cell according to claim 9.

12. Use of the pharmaceutical composition according to claim 11, for the manufacture of a medicament for treating a disease (e.g., a tumor) having a KRAS_G12V mutation.
